# EUROPEAN PATENT SPECIFICATION

(11) **EP 3 137 645 B1**
(45) Date of publication and mention of the grant of the patent: **29.05.2024**
(21) Application number: 15785298.9
(22) Date of filing: 29.04.2015
(51) Int. Cl.: C22F 1/10, A61B 17/064, A61B 17/72, A61B 17/80, A61B 17/86

(54) **CONTROLLING THE UNLOADING STRESS OF NITINOL DEVICES**
STEUERUNG DES ENTLASTUNGSSTRESSES VON NITINOLVORRICHTUNGEN
RÉGULATION DE LA CONTRAINTE DE DÉCHARGE DE DISPOSITIFS DE NITINOL

(30) Priority: 29.04.2014 US 201461985910 P; 20.05.2014 US 201462000621 P; 28.05.2014 US 201462004033 P; 04.06.2014 US 201462007711 P; 07.08.2014 US 201462034208 P; 07.08.2014 US 201462034204 P; 07.08.2014 US 201462034213 P; 15.09.2014 US 201462050430 P; 12.11.2014 US 201414539650; 13.11.2014 US 201414541017; 13.11.2014 US 201414540351; 28.01.2015 US 201562108843 P; 13.03.2015 US 201514658009; 24.03.2015 US 201562137570 P; 24.03.2015 US 201562137496 P; 24.03.2015 US 201562137645 P; 24.03.2015 US 201562137614 P; 20.04.2015 US 201562149714 P; 20.04.2015 US 201562149706 P
(43) Date of publication of application: 08.03.2017
(73) Proprietor: Arthrex, Inc., Naples, FL 34108-1945 (US)
(72) Inventor: PALMER, Matthew, Cambridge, MA 02139 (US); FONTE, Matthew, Concord, MA 01742 (US); DEVANEY, Robert, Auburndale, MA 02466 (US)
(74) Representative: Lohr, Jöstingmeier & Partner
(86) International application number: PCT/US2015/028328
(87) International publication number: WO 2015/168311

(56) References cited:
- US-A- 5 190 546
- US-A- 5 766 218
- US-A- 5 876 434
- US-A- 5 876 434
- US-A1- 2004 260 377
- US-A1- 2005 043 757
- US-A1- 2005 240 190
- US-A1- 2010 063 506
- US-A1- 2011 008 643
- US-A1- 2011 008 643
- US-A1- 2011 247 731
- US-A1- 2011 247 731

## Description

### Field Of The Invention

The present invention relates to approaches for controlling the unloading stress of Nitinol devices, and/or other shape memory material devices, in order to facilitate healing of diseased or damaged tissue. The present invention finds particular utility in the field of orthopedics, and specifically as an approach for creating and using devices for reducing fractures and generating and maintaining compression between bone fragments. While the present invention has application throughout the body, its utility will sometimes hereinafter be illustrated in the context of providing and using screws, staples, plates, and/or intramedullary devices for the repair of fractured or displaced bone tissue.

### Background Of The Invention

In the field of orthopedic surgery, it is common to rejoin broken bones. The success of the surgical procedure often depends on the ability to reapproximate the fractured bone, the amount of compression achieved between the bone fragments, and the ability to sustain that compression over a period of time. If the surgeon is unable to bring the bone fragments into close contact, a gap will exist between the bone fragments and the bone tissue will need to first fill that gap before complete healing can take place. Furthermore, gaps between bone fragments that are too large can allow motion to occur between the bone fragments, disrupting the healing tissue and thus slowing the healing process. Thus, non-unions, mal-unions, and delayed-unions of fractures can occur when the gap between bone fragments is too large. Optimal healing requires that the bone fragments be in close contact with each other, and for a compressive load to be applied and maintained between the bone fragments. Compressive strain between bone fragments has been found to accelerate the healing process in accordance with Wolf's Law.

Broken bones can be rejoined using screws, staples, plates, intramedullary devices, and other devices known in the art. These devices are designed to assist the surgeon with reducing the fracture and creating a compressive load between the bone fragments.

Screws are typically manufactured from either titanium or stainless steel alloys and may be lag screws or headless screws. Lag screws have a distal threaded region and an enlarged head. The head contacts the cortical bone surface and the action of the threaded region reduces the fracture and generates a compressive load. Headless screws typically have a threaded proximal region and a threaded distal region. A differential in the thread pitch of the two regions generates compression across the fracture site. There also exist fully-threaded headless compression screws that have a thread pitch which differs over the length of the single continuous thread.

Staples are formed from a plurality of legs (typically two legs, although sometimes more than two legs may be provided) connected together by a bridge. Staples are typically manufactured from stainless steel alloys, titanium alloys or Nitinol, a shape memory alloy. The staples are inserted into pre-drilled holes on either side of the fracture site.

Plates are also used to rejoin broken bones. These plates are generally formed from a sheet or ribbon of material having a plurality of holes formed therein. The plates are typically manufactured from either stainless steel alloys or titanium alloys. The plates are placed adjacent to a fracture so that the plate spans the fracture line, and then screws are inserted through the holes in the plate and into the bone fragments on either side of the fracture site so as to stabilize the bone fragments relative to one another.

Intramedullary devices are often used for fractures of the long bones; however, they are also frequently used in the phalanges, and specifically for the treatment of "hammer toe", which is a deformity of the proximal interphalangeal joint of the second, third, or fourth toe causing the toe to be permanently bent, e.g., bent upwards. Typical intramedullary devices used in the phalanges have opposing ends that are adapted to grip the interior wall of the intramedullary canal. These intramedullary devices are typically made of titanium alloys, stainless steel alloys, Nitinol and/or other materials, e.g., PEEK. The titanium alloy devices and stainless steel alloy devices often have barbs or threaded regions at their opposing ends to grip the interior wall of the intramedullary canal. The Nitinol devices may have a pair of radially-extending "legs" at their opposing ends that expand outward when warmed to body temperature, with the pair of legs at each end of the device being disposed in a common plane.

While the foregoing devices (e.g., screws, staples, plates, and intramedullary devices) are designed to bring the bone fragments into close contact and to generate a compressive load between the bone fragments, the devices do not always succeed in accomplishing this objective. It is widely reported that the compressive load generated by these devices between the bone fragments dissipates rapidly as the bone relaxes and remodels around the device.

Nitinol can be used to improve the functional performance of these devices by utilizing either the shape memory effect of Nitinol or the superelastic properties of Nitinol to pull together the opposing bone fragments; however, the recovery forces generated by the Nitinol versions of these devices are often too great, and result in the devices "tearing through" the bone tissue and thus not providing a means to generate and maintain compression between the bone fragments. Thus there exists a clinical need for controlling the unloading stress of Nitinol devices, and/or other shape memory material devices, so as to allow for devices that are able to bring bone fragments into close proximity with each other, generate a compressive load, and maintain that compressive load for a prolonged period of time while healing occurs - without the negative effect of having the device "tear through" the bone tissue.

Furthermore, current Nitinol devices are single-action devices, i.e., they rely on either (i) the superelastic effect of Nitinol (i.e., the superelasticity of Nitinol) to create compression, or (ii) the shape memory effect of Nitinol (i.e., the temperature-induced shape change of Nitinol) to create compression. There exists a clinical need for devices (e.g., made of Nitinol and/or other shape memory materials) that can have a staged unloading of the device without the need for complex delivery devices (i.e., a first stage can be radially expanding to grip bone, and a second stage can be shortening to pull fragments together).

Finally, while Nitinol's superelastic and shape memory properties allow for many design possibilities, it can be clinically beneficial to provide Nitinol devices (and/or other shape memory material devices) that have regions which do not unload (i.e., regions which may be permanently deformed). By way of example but not limitation, it would be beneficial to provide a monolithic (i.e., one-piece) Nitinol plate (or a plate made of other shape memory materials) that in one region is able to generate compression through superelasticity or shape memory effect, but in another region is able to be contoured to the shape of the bone.

US 2011/0247731 A1 discloses a method for producing strain induced austenite and US 2010/063506 A1 discloses a method and apparatus for a multiple transition temperature implant.

### Summary Of The Invention

The invention is set out in the appended set of claims.

The present invention provides a novel approach for controlling the unloading stress of a Nitinol device. The device may be used to bring bone fragments into close proximity with each other, generate a compressive load between the bone fragments, and maintain that compressive load between the bone fragments while healing occurs.

Among other things, the present invention comprises approaches for controlling the unloading stress of screws, staples, plates, and intramedullary devices, etc. made out of Nitinol.

In accordance with the present invention, it is possible to provide devices that utilize Nitinol's superelastic properties and/or Nitinol's shape memory effect to generate a compressive load between the bone fragments and maintain that compressive load between the bone fragments while healing occurs; however, it is critical that the Nitinol device does not generate too great of an unloading stress, since excessive forces may cause damage to the bone or cause the device to "tear through" the bone.

In one form of the present invention, the invention comprises eliminating or reducing the Load Transition and Deflection Range from the Nitinol device's mechanical hysteresis curve when the device is deformed using superelasticity.

This is accomplished by allowing the device to partially recover the elastic strain of the deformed device prior to use. This allows the device to recover strain during use at a nearly linear level (i.e., on the lower plateau of the mechanical hysteresis curve) without subjecting the bone tissue to stress loads that are above the lower plateau of the Nitinol device's mechanical hysteresis curve.

In another form of the present invention, the invention comprises eliminating or reducing the Load Transition and Deflection Range from the Nitinol device's mechanical hysteresis curve when the device is deformed using the shape memory effect. This is accomplished by straining the device in its martensitic state and, when thereafter warmed to above its austenite start temperature, allowing the device to partially recover the elastic strain prior to use. This allows the deformed device to recover strain during use at a nearly linear level (i.e., on the lower plateau of the mechanical hysteresis curve) without subjecting the bone tissue to stress loads that are above the lower plateau of the Nitinol device's mechanical hysteresis curve.

In another form of the present invention, the unloading of the Nitinol device is separated into (i) unloading caused by the superelasticity of the Nitinol, and (ii) unloading caused by the shape memory effect of the Nitinol. This is accomplished by providing a monolithic (i.e., one-piece) device having multiple temperature transition zones. Regions which are to unload via superlasticity may have very low transition temperatures, whereas regions which are to unload via the shape memory effect may have transition temperatures closer to that of the human body. Further, the unloading of the Nitinol device may be separated into (i) unloading caused by the shape memory effect of the Nitinol at one Aₛ temperature, and (ii) unloading caused by the shape memory effect of the Nitinol at a second, greater Aₛ temperature. This is accomplished by providing a monolithic (i.e., one-piece) device having multiple temperature transition zones. It should be appreciated that the unloading of the device can be controlled by allowing for partial strain recovery as detailed above (i.e., where partial unloading is allowed to occur prior to use).

In another form of the present invention, the Nitinol device can be configured so that some regions of the device unload their stress via superelasticity or shape memory effect, and other regions of the device do not unload their stress at all (i.e., they may be permanently deformed). This can be achieved by providing a device with different transition temperatures. The regions that do not unload their stress (i.e., that may be permanently deform) can have transition temperatures greater than body temperature, or can be fully annealed so as to not have a transition temperature. It should be appreciated that the regions of the device that do unload their stress via superelasticity or shape memory effect can be controlled by allowing for partial strain recovery as detailed above (i.e., where partial unloading is allowed to occur prior to use) and that the unloading of the device may occur in multiple stages.

Furthermore, it should be appreciated that it is possible to further control the unloading stress of the Nitinol device by varying percentages of cold work, and different transition temperatures, can be combined with each other, and with the aforementioned partial recovery approach (i.e., where partial unloading is allowed to occur prior to use) to further control the unloading stress of the Nitinol device).

In one preferred form, not part of the present invention, there is provided a method for applying compression to bone, said method comprising:
providing a shape memory material device;
deforming said shape memory material device using the superelastic properties of said shape memory material device so that said shape memory material device generates a stress level which is at or above the upper plateau of the mechanical hysteresis curve of said shape memory material device;
allowing said shape memory material device to recover a portion of its deformation so that said shape memory material device generates a stress level which is substantially at the lower plateau of said mechanical hysteresis curve of said shape memory material device;
mechanically constraining said shape memory material device;
deploying said shape memory material device so that said shape memory material device is in association with bone; and
releasing the mechanical constraint so that said shape memory material device applies compression to bone.

In another preferred form not part of the present invention, there is provided apparatus comprising:
a shape memory material device deformed using its superelastic properties and constrained so that said shape memory material device generates a stress level which is substantially at the lower plateau of the mechanical hysteresis curve of said shape memory material device.

In another preferred form not part of the present invention, there is provided a method for applying compression to bone, said method comprising:
providing a shape memory material device at a temperature below Mₛ;
deforming said shape memory material device while at a temperature below Mₛ using the shape memory effect properties of said shape memory material device so that said shape memory material device forms detwinned martensite;
applying a mechanical constraint to said shape memory material device that allows said shape memory material device to partially recover the deformation when said shape memory material device is heated to a temperature above Aₛ;
heating said shape memory material device to a temperature above Aₛ so that said shape memory material device recovers some strain via free recovery, whereupon said mechanical constraint limits further strain recovery and the stress in said mechanical device increases;
deploying said shape memory material device so that said shape memory material device is in association with bone; and
releasing said mechanical constraint so that said shape memory material device applies compression to bone.

In another preferred form not being part of the present invention, there is provided
apparatus comprising:
a shape memory material device which has been deformed while at a temperature below Mₛ, and which has then been heated to a temperature above Aₛ so that some of the strain is recovered via free recovery, and which has then been constrained so as to increase the stress within said shape memory material device without allowing additional strain recovery, such that when the constraint is thereafter removed, said shape memory material device generates a stress level which is substantially at the lower plateau of the mechanical hysteresis curve of said shape memory material device.

In another preferred form not being part of the present invention, there is provided a method for applying compression to bone, said method comprising:
providing a shape memory material device having a first region having a first Af temperature and a second region having a second Af temperature, wherein the second Af temperature is higher than the first Af temperature;
straining said shape memory material device using at least one of the superelastic property of said shape memory material device and the shape memory effect property of said shape memory material device;
deploying said shape memory material device so that said shape memory material device is in association with bone; and
releasing the strain of said shape memory material device so that said shape memory material device applies compression to bone.

In another preferred form not being part of the present invention, there is provided apparatus comprising:
a shape memory material device comprising a first region having a first A_{f} temperature and a second region having a second Af temperature, wherein the second Af temperature is higher than the first Af temperature.

In another preferred form not being part of the present invention, there is provided a method for applying compression to bone, said method comprising:
providing a shape memory material device having a first region having a first Af temperature and a second region having a second Af temperature, wherein the first Af temperature is below 37°C and hence austenitic at 37°C and the second A_{f} temperature is above 37°C and hence martensitic at 37°C;
straining said first region of said shape memory material device at a temperature below 37°C and maintaining the strain so that said first region exhibits superelasticity, and straining said second region of said shape memory material device at a temperature of below 37°C so that said second region is deformed;
deploying said shape memory material device so that said shape memory material device is in association with bone; and
releasing the strain of said first region of said shape memory material device so that said shape memory material device applies compression to bone.

In another preferred form not being part of the present invention, there is provided
apparatus comprising:
a shape memory material device having a first region having a first Af temperature and a second region having a second Af temperature, wherein the first A_{f} temperature is below 37°C and hence austenitic at 37°C, and the second A_{f} temperature is above 37°C and hence martensitic at 37°C.

### Brief Description Of The Drawings

These and other objects and features of the present invention will be more fully disclosed or rendered obvious by the following detailed description of the preferred embodiments of the invention, which is to be considered together with the accompanying drawings wherein like numbers refer to like parts and wherein:
Fig. 1 is a schematic view showing the stress-strain diagrams for different biomaterials - Nitinol, unlike steel (and other structural alloys), shows the ability to recover large amounts of strain, similar to human hair, bone, and tendon, i.e., Nitinol can elastically recover approximately 8% strain;
Fig. 1A is a schematic view showing the mechanical hysteresis curve for Nitinol when the Nitinol is subjected to different levels of stretch (i.e., strain), e.g., at a 3% stretch, a 4% stretch, a 5% stretch and a 6% stretch;
Figs. 2 and 3 are schematic views showing how the shape memory effect and superelasticity of Nitinol depends on the solid-solid, diffusionless phase transformation process known as martensitic transformation - at different temperatures, different structures are preferred, and when the austenitic structure is cooled through the transition temperature, the martensitic structure forms from the austenitic phase;
Figs. 4 and 5 are schematic views showing the process known as superelasticity or pseudoelasticity -this stress strain loop (curve) is referred to as a hysteresis loop (curve);
Fig. 6 is a schematic view showing how partial recovery of strain can reduce the unloading stress of Nitinol and allow the device to recover only along the lower plateau (of the mechanical hysteresis curve) when Nitinol's superelastic properties are utilized;

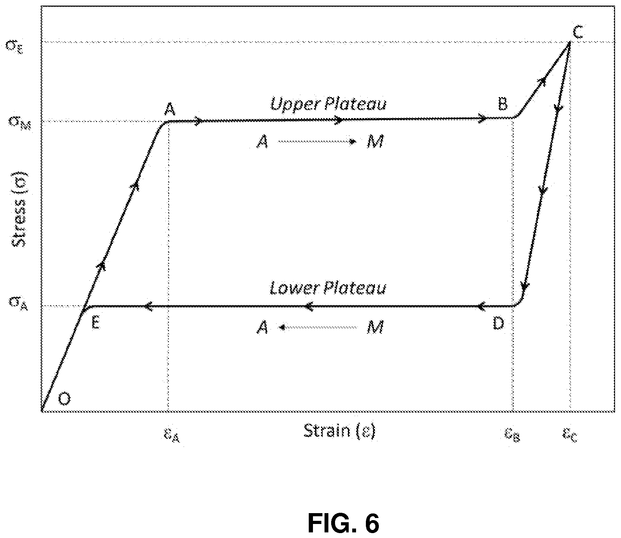

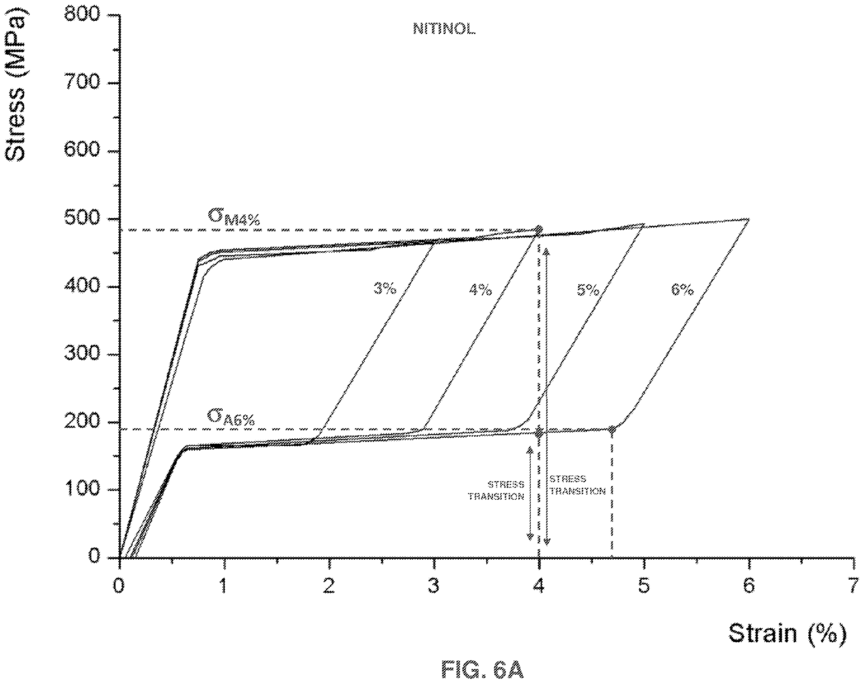

Fig. 6A is a schematic view showing that the amount of stress imposed on the bone by an unloading Nitinol device differs significantly between (i) a first situation where the Nitinol device is stressed, then "backed off' to the lower plateau of the mechanical hysteresis curve, then further "backed off' along the lower plateau to a given level of strain, then constrained at that given level of strain, and then released into bone at that given level of strain (so that the Nitinol device unloads from that given level of strain along the lower plateau of the mechanical hysteresis curve), and (ii) a second situation where the Nitinol device is stressed to a given level of strain, then constrained at that given level of strain, and then released into the bone at that given level of strain (so that the Nitinol device unloads from the upper plateau of the mechanical hysteresis curve);
Fig. 7 is a schematic view showing the shape memory effect exhibited by Nitinol;
Figs. 8 and 9 are schematic views showing how partial recovery of strain can reduce the unloading stress of Nitinol and allow the device to further recover only along the lower plateau (of the hysteresis curve) when the shape memory effect is utilized to create "constrained martensite";
Fig. 10 is a schematic view showing how different heat treatment cycles can be used to raise the austenite finish temperature of Nitinol;
Figs. 11A and 11B are schematic views showing examples of heat treatment fixtures which can apply selective heat treatment to a Nitinol device;
Figs. 12A and 12B are schematic views showing a Nitinol device that has regions that are malleable and other regions that are superelastic;
Fig. 13 is a schematic view showing how the difference between the Nitinol's austenite finish temperature and the test temperature (e.g., 37°C) affects the mechanical hysteresis curve of a Nitinol device;
Fig. 14 is a schematic view showing the effect of cold work on the mechanical hysteresis curve of a Nitinol device;
Fig. 15 is a schematic view showing a novel compression screw system formed in accordance with the present invention;
Figs. 16 and 17 are schematic views showing the compression screw of the compression screw system shown in Fig. 15;
Figs. 18 and 19 are schematic views showing the internal retaining pin of the compression screw system shown in Fig. 15;
Figs. 20-22 are schematic views showing the internal retaining pin disposed within the interior of a strained (i.e., stretched) compression screw;
Fig. 23 is a schematic view showing how the compression screw may be strained (i.e., stretched), retained in that strained (i.e., stretched) condition by inserting the internal retaining pin within the interior of the strained (i.e., stretched) compression screw, and then allowed to foreshorten by removal of the internal retaining pin;
Fig. 24 is a schematic view showing how the compression screw system of Fig. 15 may be used to treat a fracture;
Figs. 24A-24E are schematic views showing a two-part driver which may be used to deploy the compression screw system shown in Fig. 15;
Fig. 25 is a schematic view showing another novel compression screw system formed in accordance with the present invention;
Fig. 26 is a schematic view showing the compression screw of the compression screw system shown in Fig. 25;
Figs. 27-29 are schematic views showing the cannulated inner driver of the compression screw system shown in Fig. 25;
Figs. 30 and 31 are schematic views showing the cannulated outer driver of the compression screw system shown in Fig. 25;
Figs. 32-38 are schematic views showing how the already-strained compression screw (i.e., the already-stretched compression screw) may be retained in that strained (i.e., stretched) condition by mounting the strained (i.e., stretched) compression screw on the cannulated inner driver and then securing the strained (i.e., stretched) compression screw on the cannulated inner driver by means of the cannulated outer driver;
Figs. 39-42 are schematic views showing how the strained (i.e., stretched) compression screw may be deployed from the cannulated outer driver and the cannulated inner driver causing the screw to release from the driver system and foreshorten;
Figs. 43 and 44 are schematic views showing how the compression screw system of Fig. 25 may be used to treat a fracture;
Fig. 45 is a schematic view of a novel staple formed in accordance with the present invention, wherein the staple comprises a bridge which is capable of being elastically strained and legs which are capable of being elastically strained, and further wherein the staple is shown in its unstrained condition;
Fig. 46 is a schematic view of the novel staple shown in Fig. 45, wherein the bridge of the staple has been elastically strained (i.e., longitudinally stretched) and the legs of the staple have been elastically bent outwards;
Fig. 47 is a schematic view showing how the elastically strained staple of Fig. 46 will foreshorten along its bridge, and have its legs "kick inward", when the strain on the staple is removed;
Figs. 48 and 49 are schematic views showing an exemplary delivery device which may be used with the novel staple shown in Fig. 45 to elastically strain (i.e., stretch) the bridge of the staple and elastically bend the legs of the staple;
Figs. 50 and 51 are schematic views showing the delivery device of Figs. 48 and 49 being used with the novel staple shown in Fig. 45 to elastically strain (i.e., stretch) the bridge of the staple and elastically bend the legs of the staple;
Fig. 52 is a schematic view showing how the novel staple of Fig. 45 may be used to generate and maintain a greater, and more uniform, compression between bone fragments so as to aid in fracture healing;
Figs. 52A, 52B and 52C are schematic views showing another form of delivery device which may be used with the novel staple shown in Fig. 45 to elastically strain (i.e., stretch) the bridge of the staple and elastically bend the legs of the staple;
Figs. 53 and 54 are schematic views of another novel staple formed in accordance with the present invention, wherein the staple comprises a malleable bridge which is capable of being inelastically deformed and legs which are capable of being elastically strained, and further wherein Fig. 53 shows the staple in its unstrained condition and Fig. 54 shows the staple with its bridge bent but its legs in an unstrained condition;
Fig. 54A is a schematic view of another novel staple formed in accordance with the present invention, wherein the staple has a bridge that is convex;
Figs. 55 and 56 are schematic views showing an exemplary bending device which may be used with the novel staple shown in Fig. 53 and 54 to inelastically bend the bridge of the staple to more appropriately conform to the surface profile of the cortical bone;
Fig. 57 is a schematic view which shows the staple of Figs. 53 and 54 after the bridge of the staple has been inelastically bent and after the legs of the staple have been elastically strained into a parallel condition;
Figs. 58-60 are schematic views showing a plier assembly which may be used with the novel staple shown in Fig. 53 and 54 to elastically strain (i.e., stretch) the legs of the staple after the bridge of the staple has already been inelastically bent;
Figs. 61 and 62 are schematic views showing how the novel staple shown in Figs. 53 and 54 may have the bridge of the staple inelastically bent to conform to the surface profile of a bone, the legs of the staple elastically bent into a parallel condition, and the staple thereafter deployed in bone so as to provide compression across a fracture.
Fig. 63 is a schematic view of another novel staple formed in accordance with the present invention, wherein the staple comprises a malleable bridge which is capable of being inelastically deformed and legs which are capable of being elastically strained, and further wherein the bridge of the staple has been deformed to have a convex configuration after bending;
Figs. 64 and 65 are schematic views of another novel device which may be used to bend the bridge of the staple shown in Fig. 54A;
Figs. 66-69 are schematic views showing an intramedullary fusion device formed in accordance with the present invention;
Fig. 69A is a schematic view showing removable retaining tabs which may be used to hold the first and second barbed end regions of the intramedullary fusion device of Figs. 66-69 in their radially constrained condition;
Figs. 70-80 are schematic views showing the novel intramedullary fusion device of Figs. 66-69 being used to treat a hammer toe condition;
Fig. 80A is a schematic view showing another intramedullary fusion device formed in accordance with the present invention;
Fig. 80B is a schematic view showing another intramedullary fusion device formed in accordance with the present invention;
Figs. 81-83 are schematic views showing another intramedullary fusion device formed in accordance with the present invention;
Figs. 84-90 are schematic views showing an intramedullary fusion system formed in accordance with the present invention;
Figs. 91-101 are schematic views showing the novel intramedullary fusion system of Figs. 84-90 being used to treat a hammer toe condition;
Fig. 102 is a schematic view showing another intramedullary fusion device formed in accordance with the present invention and which may be used with the intramedullary fusion system shown in Figs. 84-90;
Fig. 103 is a schematic view of a novel compression plate formed in accordance with the present invention, wherein the compression plate comprises a pair of bridge members which are capable of being elastically strained, two openings for receiving screws, and further wherein the compression plate is shown in its unstrained condition;
Fig. 104 is a schematic view of the novel compression plate shown in Fig. 103, wherein the pair of elastic bridge members of the compression plate have been elastically strained to a near-parallel condition;
Fig. 105 is a schematic view showing how the elastically strained compression plate of Fig. 104 will foreshorten when the strain on the compression plate is removed;
Fig. 106 is a schematic view of another novel compression plate formed in accordance with the present invention, wherein the compression plate comprises a pair of bridge members which are capable of being elastically strained, wherein each of the opposing regions of the compression plate comprises two openings for receiving screws, and further wherein the compression plate is shown in its unstrained condition;
Fig. 107 is a schematic view of the novel compression plate shown in Fig. 106, wherein the two elastic bridge members of the compression plate have been elastically strained to a near-parallel condition;
Fig. 108 is a schematic view showing how the elastically strained compression plate of Fig. 107 will foreshorten when the strain on the compression plate is removed;
Fig. 109 is a schematic view of another novel compression plate formed in accordance with the present invention, wherein the compression plate comprises a single "S-shaped" bridge member which is capable of being elastically strained, two openings for receiving screws, and further wherein the compression plate is shown in its unstrained (i.e., unstretched) condition;
Fig. 110 is a schematic view of the novel compression plate shown in Fig. 109, wherein the single "S-shaped" elastic bridge member of the compression plate has been elastically strained (i.e., longitudinally stretched);
Fig. 111 is a schematic view showing how the elastically strained compression plate of Fig. 109 will foreshorten when the strain on the compression plate is removed;
Figs. 112A-112F are schematic views showing other novel compression plates formed in accordance with the present invention - these compression plates may be used when a more tailored anatomical fixation is required (e.g., for Cranio-Maxillofacial fracture fixation);
Fig. 113 is a schematic view of a screw system which can be used to attach the novel compression plate of the present invention to bone - in this embodiment, the screw is a snap-off screw that detaches from the driver when the screw has been fully seated in the bone;
Fig. 114 is a schematic view showing snap-off screws being used to attach the novel compression plate to the bone;
Fig. 115 is a schematic view showing a delivery device which may be used with the novel compression plate shown in Fig. 106 to elastically strain (i.e., stretch) the two elastic bridge members of the compression plate so as to make them parallel (or at least more parallel than their unconstrained state);
Fig. 116 is a schematic view showing how the delivery device shown in Fig. 115 engages the novel compression plate shown in Fig. 106;
Fig. 117 is a schematic cross-sectional view of the delivery device showing in Fig. 115;
Fig. 118 is a schematic view of an alternative delivery device which may be used with the novel compression plate shown in Fig. 104;
Figs. 119 and 120 are schematic views showing how the novel compression plate shown in Figs. 106 and 107 may be used to provide compression across a bone fracture;
Fig. 121 is a schematic view of another novel compression plate formed in accordance with the present invention, wherein the compression plate is contoured so as to allow for multiple compression plates to be assembled together into a custom plate configuration, and further wherein the two elastic bridge members of the compression plate can be elastically strained to a near-parallel condition;
Fig. 122 is a schematic view of a multi-member compression plate configuration made up of a plurality of the compression plates shown in Fig. 121;
Fig. 123 is a schematic view of the lap joint which is created when two of the compression plates of Fig. 121 are assembled together;
Fig. 124 is a schematic view of another novel compression plate formed in accordance with the present invention, wherein areas of the compression plate (e.g., the two opposing regions of the compression plate) have been selectively treated so as to allow for plastic deformation to occur in those areas, and elastic strain to occur in other areas of the compression plate (e.g., the two elastic bridge members of the compression plate);
Figs. 125-127 are schematic views showing plastic threaded inserts disposed between the openings of the compression plate and the fixation screws which are used to hold the compression plate to bone; and
Fig. 128 is a schematic view of a novel distraction plate formed in accordance with the present invention.

### Detailed Description Of The Preferred Embodiments

### Nitinol In General

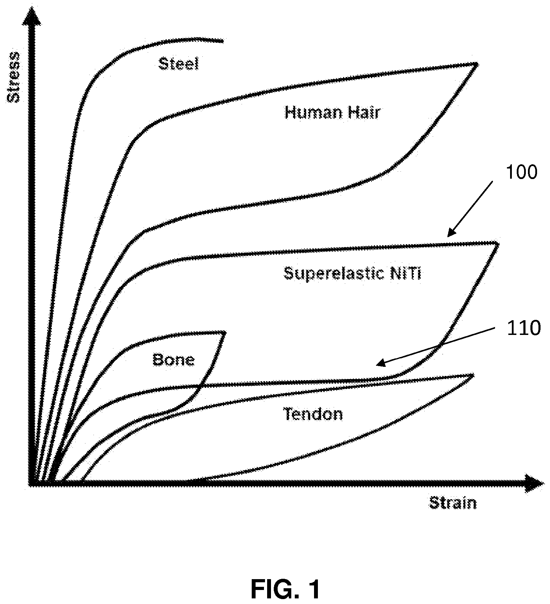

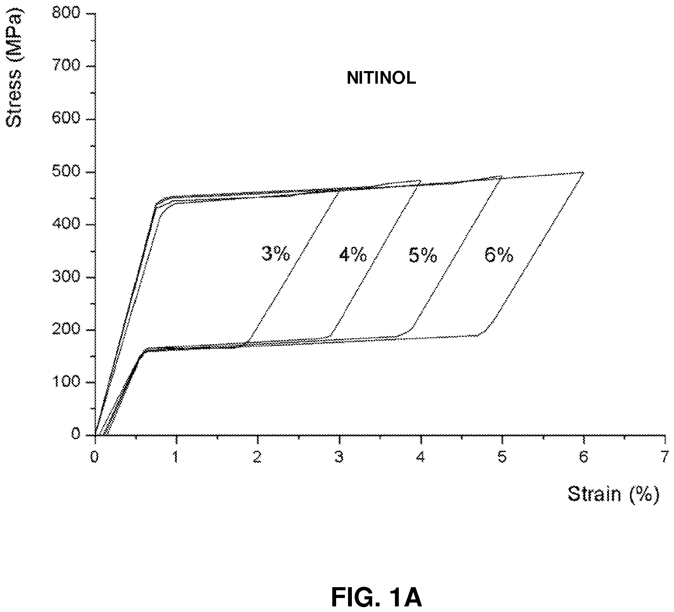

Looking first at Fig. 1, the stress-strain diagrams for different biomaterials is shown. Nitinol, unlike steel (and other structural alloys), shows the ability to recover large amounts of strain, similar to human hair, bone, and tendon. Nitinol can elastically recover approximately 8% strain while steel can only elastically recover approximately 1% strain. When Nitinol is strained, it deforms at a stress shown by the upper plateau 100 of the Nitinol's mechanical hysteresis curve. When the strain is released, Nitinol returns to its original shape with a stress shown by the lower plateau 110 of the Nitinol's mechanical hysteresis curve.

As seen in Fig. 1A, when Nitinol is strained to different levels, it will have different mechanical hysteresis curves according to the extent of the strain, e.g., the mechanical hysteresis curve for a Nitinol device subjected to a 3% stretch is different from the mechanical hysteresis curve for a Nitinol device subjected to a 4% stretch, etc.

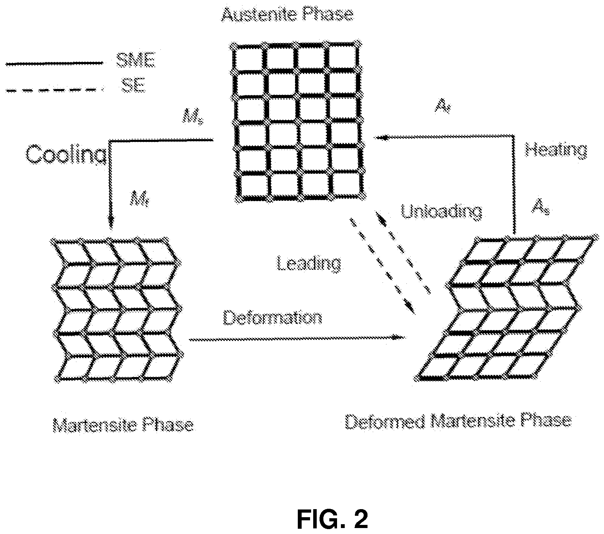

Nitinol exhibits two unique properties: shape memory effect (SME) and superelasticity (SE). These properties depend on the solid-solid, diffusionless phase transformation process known as martensitic transformation (Fig. 2) in which the more-ordered parent phase austenite is transformed to the less-ordered phase martensite. The phase transformation from austenite to martensite (or vice versa) is marked by four transition temperatures: the Martensite finish (M_{f}) temperature, the Martensite start (Mₛ) temperature, the Austenite finish (Af) temperature, and the Austenite start (Aₛ) temperature, where M_{f} < Mₛ < Aₛ < Af. A change in the temperature T within the range Mₛ < T < Aₛ induces no phase change. Both martensite and austenite may coexist at the temperature T within the range M_{f} < T < Af.

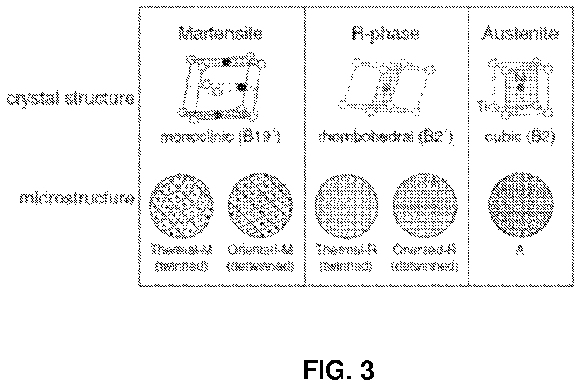

At different temperatures, different phase structures are preferred, and when the structure is cooled through the transition temperature, the martensitic structure forms from the austenitic phase. Austenite is the high-temperature, stress-free phase which has a high-symmetry crystal structure usually based on a cubic lattice (Fig. 3). Austenite has an ordered B2 crystal structure, which can be viewed as two interpenetrating simple cubic lattices of nickel (Ni) and titanium (Ti), respectively. Martensite, the low temperature phase, is monoclinic and heavily twinned. Martensite can be deformed up to 8% strain by detwinning. Martensite has an ordered B19' crystal structure, where "B19" denotes an orthorhombic structure resulting from unequal normal strains relative to the "[110]" vector directions of the austenite structure, and the prime "'" indicates that the Nitinol has additionally been distorted by a shear strain, resulting in monoclinic symmetry.

### Method For Controlling The Unloading Of Stress In Nitinol When Utilizing Superelasticity

Nitinol screws, staples, plates and intermedullary devices, as well as other devices, can be configured to utilize Nitinol's superelastic properties to generate and maintain compression across a fracture. The present invention discloses how to control the stress at which the Nitinol recovers its elastic deformation, i.e., the present invention discloses how to control the unloading of stress of Nitinol when utilizing superelasticity.

In superelasticity, at T > Aε, the Nitinol is entirely in the parent austenitic phase. When the Nitinol is then mechanically loaded (i.e., strained), the stress in the Nitinol increases. There is a critical stress at which the crystal transforms from austenite to martensite. Stressing the material above this critical value results in device deformation, with the austenitic Nitinol transforming to martensitic Nitinol. Deformation can be as large as about 8% with elastic recovery. At T > A_{f}, the martensitic Nitinol of the deformed device is unstable and, when the load is removed, the reverse phase transformation occurs (i.e., the martensite transforms to austenite), and the material fully regains its original shape. In practice, it is often advantageous to deform the Nitinol device, and then mechanically constrain the Nitinol device in its deformed shape, before providing the device to the surgeon. This allows the surgeon to release the mechanical constraint on the deformed device and, at T > Aε, the device will fully regain its original shape. Thus, with superelasticity, the austenite-to-martensite-to-austenite transformation allows for a large amount of elastic deformation and shape recovery.

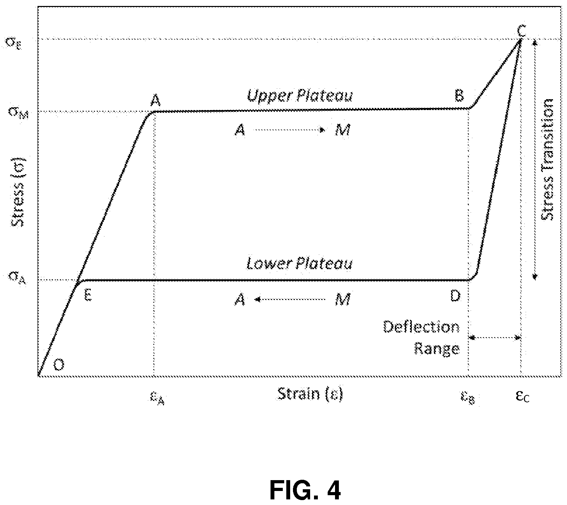

Looking now at Fig. 4, when T > Aₛ, and when strain is applied to the Nitinol device, the Nitinol device deforms elastically along line OA until a critical stress (σ_{M}) is reached. σ_{M} is the level of stress at which stress-induced martensite is created from austenite. Increasing deformation (strain) results in the creation of increasing amounts of stress-induced martensite, as is shown along the line AB. At point B, the alloy has become fully martensitic. Once fully martensitic, elastic deformation of the martensite occurs along the line BC.

Since the alloy is at a temperature greater than Aₛ, the martensite is unstable (i.e., the Nitinol wants to return to the austenitic phase). When the strain is removed, the alloy recovers elastically from point C to D (the Deflection Range, i.e.,ε_{C}-ε_{B}). The stress at point D (σ_{A}) represents the stress level at which martensite reverts back to austenite. Reversion occurs at essentially a constant stress (line DE). Finally, at point E, the material has become fully austenitic. Further stress recovery (from point E to O) occurs through the elastic recovery of the fully austenitic alloy.

Conventionally, once a Nitinol device is deformed to point C, the device is constrained so as to prevent the device from recovering the elastic stress. At this point the device may be packaged and sterilized. It is then provided to the surgeon ready for use. When the surgeon removes the constraint, the device is allowed to recover its deformation, recovering from point C to D (the Deflection Range, i.e., ε_{C}-ε_{B}), then to point E, and then to point O. As this occurs, the device transforms from stress-induced martensite to austenite.

It should be appreciated that when the Nitinol device is recovering strain in the Deflection Range (i.e., from point C to point D), a large Stress Transition (σ_{E} to σ_{A}) exists where the Nitinol device will unload stress at a value greater than the lower plateau (defined as line DE, stress level σ_{A}) of the Nitinol's mechanical hysteresis curve. In other words, when the deformed Nitinol is released at point C and allowed to recover its elastic deformation, the Nitinol device does not immediately unload at σ_{A} (i.e., the lower plateau stress level, as shown in Fig. 4). Instead, the deformed Nitinol unloads from either σ_{M} (i.e., the upper plateau stress level) to σ_{A}, or from σ_{E} (i.e., the martensite elastic stress level) to σ_{A}, depending on the extent of elastic strain. σ_{M} may be comparatively high (e.g., around 345 MPa (50 ksi)), while σ_{A} may be substantially lower (e.g., around 69 MPa (10 ksi)). σ_{E} will be even higher than σ_{M}, i.e., above 345 MPa (50 ksi). Thus, the Deflection Range (i.e., ε_{C}-ε_{B}) may have a Stress Transition (i.e., σ_{E}-σ_{A}, or σ_{M}-σ_{A}) of greater than about 276 MPa (40 ksi). At this level of stress, the device may cause damage to bone or cause the device to "tear through" the bone.

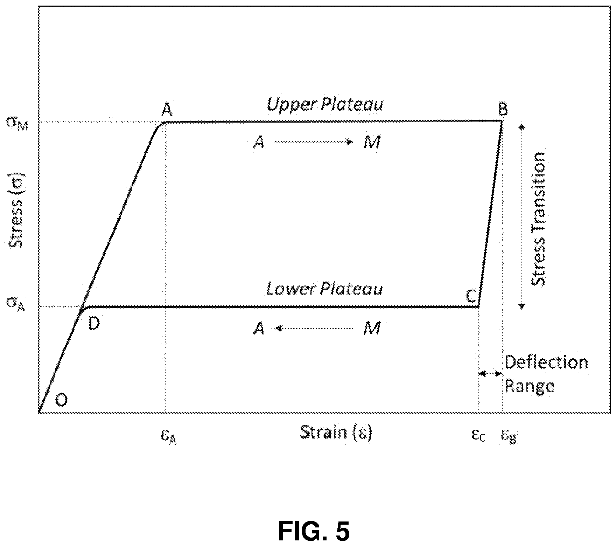

Even when the Nitinol device is not strained enough so as to induce elastic strain of the martensite (i.e., when the Nitinol device is not strained enough to transition along the line BC in Fig. 4), there still exists a Deflection Range (i.e., ε_{B}-ε_{C}) and Stress Transition (σ_{M}-σ_{A}) (Fig. 5). It should further be appreciated that the loads generated by the Nitinol device while operating in the Stress Transition (i.e., σ_{M}-σ_{A}) of Fig. 5 may still be too high for bone tissue. This may result in the Nitinol device pulling through ("tearing through") bone.

For this reason, the conventional approach of deforming a Nitinol device so as to create stress-induced martensite, restraining the Nitinol device in its deformed state, and then releasing the deformed device at the time of use, suffers from the disadvantage that the Stress Transition (σ_{E}-σ_{A} or σ_{M}-σ_{A}, Fig. 4; or σ_{M}-σ_{A}, Fig. 5) may be too great for the bone to handle, and may result in bone damage and/or the device tearing through the bone.

In accordance with the present invention, in order to eliminate or reduce the Stress Transition (i.e., σ_{E}-σ_{A}, or σ_{M}-σ_{A}, Fig. 6) and the Deflection Range (i.e., ε_{C}-ε_{B}, Fig. 6), the Nitinol device is strained up to ε_{C} (generating a stress level of σ_{E}) and, prior to being mechanically constrained, is allowed to recover its elastic deformation to ε_{B} (recovering to a stress of σ_{A}). At ε_{B}, the device is mechanically constrained. The device may then be packaged and sterilized. When the device is thereafter used, the constraint is released (i.e., at the surgical site) and the deformed device is allowed to recover the remaining elastic deformation, doing so at a stress level equal to the lower plateau (i.e., σ_{A}). In a clinical application, this allows for a constrained, elastically deformed Nitinol device to be unconstrained, and recover its elastic deformation, at a stress level equal to σ_{A} (i.e., the stress level of the lower plateau), without having to transition through a higher stress region. In other words, with the present invention, by unloading the stress of the deformed device (i.e., from σ_{E} to σ_{A}, or from σ_{M} to σ_{A}), the stress transition of the device can be reduced to the lower plateau, so that the bone never sees as stress of greater than σ_{A}.

Note that the amount of stress imposed on the bone by the unloading Nitinol device differs significantly between: (i) a first situation where the Nitinol device is stressed (e.g., to a 6% strain), then "backed off' to the lower plateau of the mechanical hysteresis curve, then further "backed off' along the lower plateau to a given level of strain (e.g., to a 4% strain), then constrained at that given level of strain (e.g., a 4% strain), and then released into the bone at that given level of strain (e.g., a 4% strain) - whereupon the maximum stress generated by the Nitinol device never rises above the lower plateau of the mechanical hysteresis curve (i.e., the maximum stress generated by the Nitinol device never rises above σ_{A6%} (see Fig. 6A); and (ii) a second situation where the Nitinol device is stressed to that same given level of strain (e.g., 4% strain), then constrained at that same given level of strain (e.g., 4% strain), and then released into the bone at that same given level of strain (e.g., 4% strain) - whereupon the maximum stress generated by the Nitinol device starts at the upper plateau of the mechanical hysteresis curve (i.e., the maximum stress generated by the Nitinol device reaches σ_{M4%} (see Fig. 6A). In other words, the stress transition produced in the aforementioned first situation (i.e., where the Nitinol device is unloaded to the lower plateau of the mechanical hysteresis curve prior to implantation) is significantly lower than the stress transition produced in the aforementioned second situation (i.e., where the Nitinol device is fully unloaded after implantation). Thus, the same amount of strain recovery (e.g., 4%) can exhibit different stress levels depending on whether the device begins unloading at the upper or lower plateau.

### Method For Controlling The Unloading Of Stress In Nitinol When Utilizing Shape Memory Effect

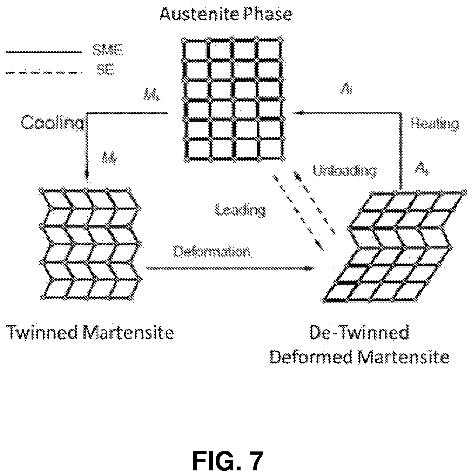

In an alternative embodiment of the present invention, the unloading of the stress of Nitinol devices may be controlled using the shape memory effect (Fig. 7).

In the shape memory effect, when T > Aε, the Nitinol is in the more-ordered parent austenite phase. Under the stress-free condition, if the Nitinol is cooled to any temperature T < M_{f}, the martensitic transformation (MT) occurs and the material converts to the less-ordered martensitic phase. This is referred to as self-accommodating twinned martensite. If a mechanical load is then applied to the Nitinol and the stress level of the deformed Nitinol reaches a certain critical value, the pairs of martensitic twins begin "detwinning". "Detwinning" is marked by an increasing level of stress with additional applied strain. Detwinned martensite is thermodynamically stable at T < Aₛ. Upon heating above Aₛ, the detwinned martensite transforms to its parent (austenite) phase, and the residual strain is fully recovered. However, if a mechanical constraint is used to prevent this free recovery to the Nitinol's original shape, the Nitinol device generates large recovery stresses.

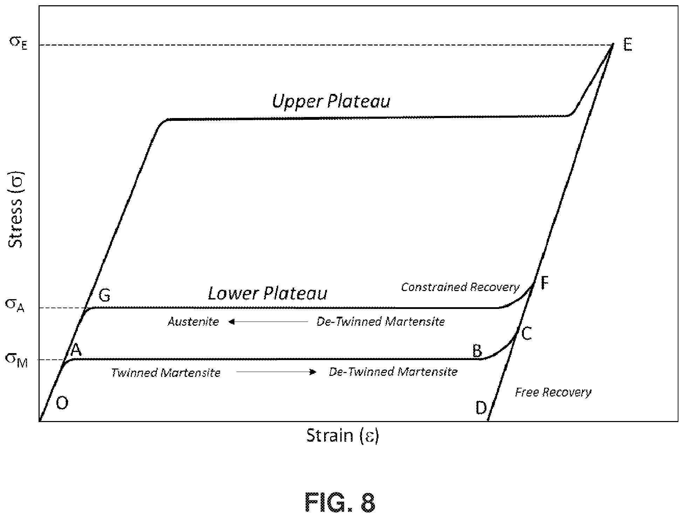

Specifically, when Nitinol is deformed at a temperature below Mₛ, it deforms elastically along the line OA (Fig. 8) until a critical stress (σ_{M}) is reached. At σ_{M}, the material can be strained at a nearly constant stress level until point B. At point B, the material has been fully detwinned, and the material can be further elastically strained to point C. At point C, if the material is not constrained (i.e., it is allowed to freely recover) and is heated to a temperature above Aₛ, the material will recover its deformation from point C to point D to point O.

While the aforementioned shape memory effect of Nitinol can be used in devices to allow controlled deformation of the device, it is generally not preferred since the device needs to be kept at a temperature below Mₛ after the Nitinol device has been deformed and until it will be used. This can be difficult in practice, since cleaning, sterilization, and transportation often expose the Nitinol device to elevated temperatures. As such, it has become popular to constrain the deformed Nitinol device (as Constrained Martensite) immediately following deformation of the Nitinol device, while the Nitinol device is at a temperature below Mₛ. This allows the Nitinol device to remain deformed even if the Nitinol device is thereafter exposed to temperatures above Aₛ.

If the aforementioned Nitinol device is strained to point C, and then constrained, when thereafter warmed to a temperature above Mₛ, the Nitinol wants to flip from its unstable detwinned martensite state to its stable austenite state, and a large amount of force can be generated by the device as it transforms from the detwinned martensite state to the austenite state. If the deformed Nitinol is constrained at point C, and then heated to a temperature above Aₛ, the stress will increase to σ_{E} (Fig. 8). This is referred to as Constrained Recovery. If the constraint is then removed, the Nitinol device will recover its deformation along the line EF, the line FG and the line GO (Fig. 8). Note the large Stress Transition from σ_{E} to σ_{A}.

Again, the Deflection Range (i.e., the change in ε) and the Stress Transition (i.e., the change in σ) should be noted as the material transitions along the mechanical hysteresis curve of Nitinol. It should be appreciated that the stresses generated by the Nitinol device while operating in the Deflection Range (i.e., between σ_{E} and (σ_{A}) may be too high for bone tissue. This may result in the Nitinol device damaging the bone and/or "tearing through" the bone. The use of Constrained Martensite can be preferable over superelasticity for the following reasons:
(1) When cooled to martensite (i.e., T < M_{f}), the Nitinol device can be strained at this softer, more formable phase, thus requiring a lower load to achieve the deformation, and thereby imposing less stress on the Nitinol device and reducing wear on the surface of the Nitinol device which is crucial to maintaining biocompatibility;
(2) the deformed device will stay deformed as long as it is kept at a temperature less than Mₛ, thereby limiting the force imposed on the constraint;
(3) the device can be mechanically constrained so as to prevent shape change if the device is warmed prematurely/accidentally to a temperature above Mₛ; and
(4) the device can be inserted into the body in the Constrained Martensitic condition and staged for shape recovery when the surgeon is ready.

In accordance with the present invention, there is also provided an approach for combining device deformation (by forming detwinned martensite) with controlling the stress level at which the Nitinol device recovers its elastic strain.

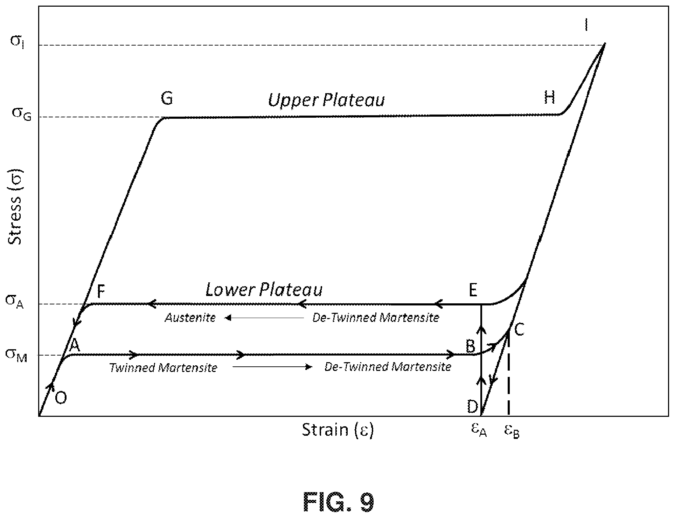

To achieve this objective, in one example, and looking now at Fig. 9, the device can be cooled to a temperature below Mₛ and then deformed along lines OA, AB, and BC of its mechanical hysteresis curve. A mechanical constraint can then be installed on the Nitinol device that allows the Nitinol device to partially recover the deformation (ε_{B}-ε_{A}) when the Nitinol device is heated to a temperature above Aₛ. The Nitinol device can then be warmed to above its Aₛ temperature. Warming results in the recovery of strain from ε_{B} to ε_{A} along the line CD via Free Recovery. A mechanical restraint can then be installed at point D, the Nitinol device has recovered enough elastic strain so that the mechanical constraint is now limiting further strain recovery. This results in the stress increasing to σ_{A} (point E) with no additional strain recovery (Constrained Recovery). Subsequently, when the mechanical constraint is removed, the Nitinol device will recover its elastic strain along the lines EF and FO of its mechanical hysteresis curve. As is the case with the aforementioned stress-induced martensite, allowing partial strain recovery prior to mechanically constraining the Nitinol device allows the Nitinol device to unload directly at the lower plateau of the mechanical hysteresis curve of the Nitinol device.

In a second example, the device can be cooled to a temperature below Mₛ and then deformed along lines OA, AB, and BC of its mechanical hysteresis curve. The Nitinol device can then be warmed to above its Aₛ temperature and strain recovery can be controlled, permitting recovery from ε_{B} to ε_{A} along the line CD via Free Recovery. When the strain level is at ε_{A}, a mechanical restraint can then be installed. At point D, the Nitinol device has recovered enough elastic strain so that the mechanical constraint is now limiting further strain recovery. This results in the stress increasing to σ_{A} (point E) with no additional strain recovery (Constrained Recovery). Subsequently, when the mechanical constraint is removed, the Nitinol device will recover its elastic strain along the lines EF and FO of its mechanical hysteresis curve. As is the case with the aforementioned stress-induced martensite, allowing partial strain recovery via the Nitinol's shape memory effect prior to mechanically constraining the Nitinol device allows the Nitinol device to unload directly at the lower plateau of the mechanical hysteresis curve of the Nitinol device.

### Method For Controlling The Unloading Of Stress In Nitinol By Combining Superelasticity And Shape Memory Effect

It should be appreciated that it is possible to combine the controlled recovery of stress in Nitinol by combining superelastically straining the device with partial recovery via shape memory effect. In this example, and looking at Fig. 9, it is possible to strain a Nitinol device from point O to G to H to I. At point I, the device can be cooled to below the material's Mₛ temperature, reducing the stress to point C. At point C, a constraint can be installed that allows for partial recovery (from ε_{B}-ε_{A}). The device can then be warmed to above Aₛ. The device will undergo free recovery from point C to point D. At point D, the constraint will not permit further recovery and the load will increase to point E. Subsequent removal of the constraint will allow the device to recover from point E to point F to point O, while never imparting a load greater than σ_{A}.

### Method For Controlling The Unloading Of Stress In Nitinol By Utilizing Multiple Transition Temperatures

There are instances when a delayed, or staged, unloading of the stress in Nitinol is preferred for a Nitinol device. By way of example but not limitation, it may be beneficial for a Nitinol device to first radially expand to grip bone fragments and then, in a second stage, longitudinally shorten to pull the bone fragments into compression.

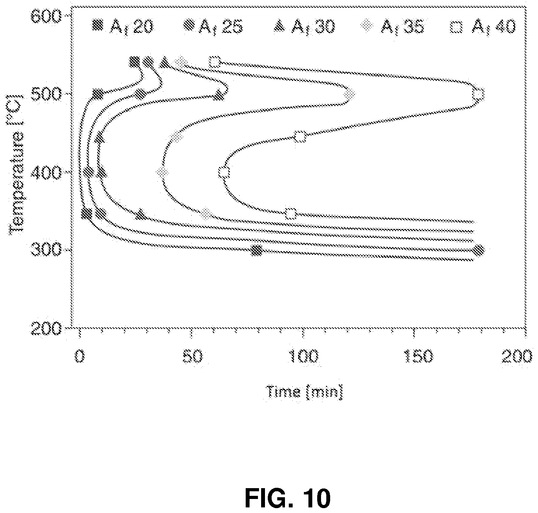

One way of accomplishing such a delayed, or staged, or "staggered", unloading action is by forming the Nitinol device with regions having two (or more) different Af temperatures (Fig. 10). By way of example but not limitation, a first region of the Nitinol device could be heat treated (aged) so as to provide an Aε temperature of 10°C for that first region. Then, isolating that first region, a second region of the device can be heat treated (aged) so as to provide an Af temperature of 37°C for that second region. As a result, at room temperature (i.e., 22°C), the first region is superelastic (since the Nitinol device will be at a temperature above the Aε temperature of the first region) but the second region is not superelastic (since the Nitinol device will be at a temperature below the A_{f} temperature of the second region) and will not "activate" until reaching a temperature above body temperature (i.e., 37°C). Thus, a Nitinol device can be made having a first region that deforms via superelasticity, and then, when the Nitinol device is warmed to body temperature, the second region changes shape according to the shape memory effect. By way of example but not limitation, a Nitinol device can thus be made having a first region that radially expands via superelasticity to grip bone, and then, when the Nitinol device is warmed to body temperature, shortens to pull the fragments together via the shape memory effect.

By way of further example but not limitation, heat treating (aging) the Nitinol material between about 300°C and 550°C causes Nickel-rich precipitation reactions to occur, changing the alloy's exact matrix composition. For example, heat treating (aging) Nitinol at about 400°C for about 70 minutes will result in the Nitinol having an Aε temperature of about 40°C.

It should be appreciated that the Nitinol device can be heat treated so as to allow the Nitinol device to have two or more Af temperatures greater than room temperature. Therefore, the Nitinol device will exhibit shape memory effect at the two (or more) A_{f} temperatures. By way of example but not limitation, a Nitinol device can be made that radially expands at an A_{f} temperature of 37°C and shortens at an Aε temperature of 50°C. When implanted into the body, the Nitinol device will radially expand via shape memory effect, and pouring warm saline over the device will trigger it to shorten via shape memory effect.

### Method For Controlling The Unloading Of Stress In Nitinol By Utilizing A Combination Of (i) Superelasticity Or Shape Memory Effect, And (ii) Malleable Regions

There may be instances where it is preferable to have the entire Nitinol device, or selected regions of the Nitinol device, not unload stress when deformed (i.e., be permanently deformed). This would allow a Nitinol device (or at least a portion of the Nitinol device) to take a set so that the Nitinol device can be contoured to a patient's anatomy. In practice, it may be desirable to have only specific regions of the Nitinol device not unload (i.e., be permanently deformed) at body temperature, while allowing other regions of the Nitinol device to unload via superelasticity or shape memory effect at body temperature. By way of example but not limitation, it may be clinically beneficial to create a fracture plate that has malleable end regions to allow those malleable end regions to be contoured longitudinally to the curvature of the bone, while having a superelastic bridge that can pull the bone fragments together in compression. In addition, it may be beneficial to form malleable end regions of a Nitinol device out of martensitic Nitinol, since martensitic Nitinol has a lower modulus which more closely matches the modulus of bone.

One way to accomplish multiple transition zones within a monolithic device (i.e., a one-piece device) is, for example, by selectively heat-treating regions of the Nitinol device to 400°C for 70 minutes, while insulating adjacent regions of the Nitinol device to maintain temperatures below 300°C, thus preventing those insultated regions from being heat treated (aged) (Fig. 10). Regions that are heat treated (aged) at 400°C for 70 minutes will have Af temperatures of about 40°C, which is greater than room temperature (22°C) and which is greater than body temperature (37°C), and thus those regions will be martensitic at room temperature (and body temperature) and hence be plastic. When deformed, these plastic regions will remain deformed. Regions that were not exposed to that heat treatment (i.e., the insulated regions) will have an Af temperature of less then room temperature and less than body temperature, and thus will be austenitic at room temperature (and at body temperature) and hence will be capable of exhibiting superelastic properties at those temperatures.

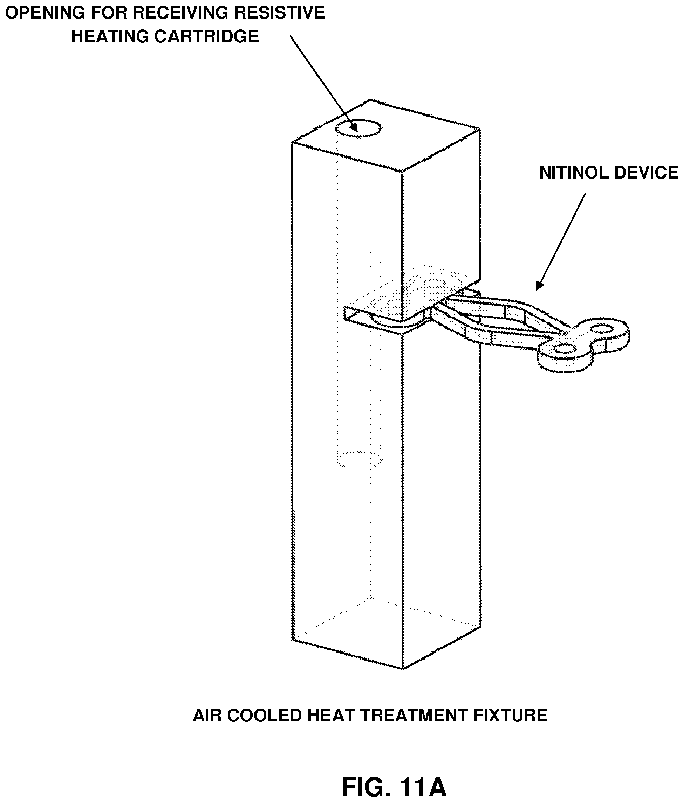

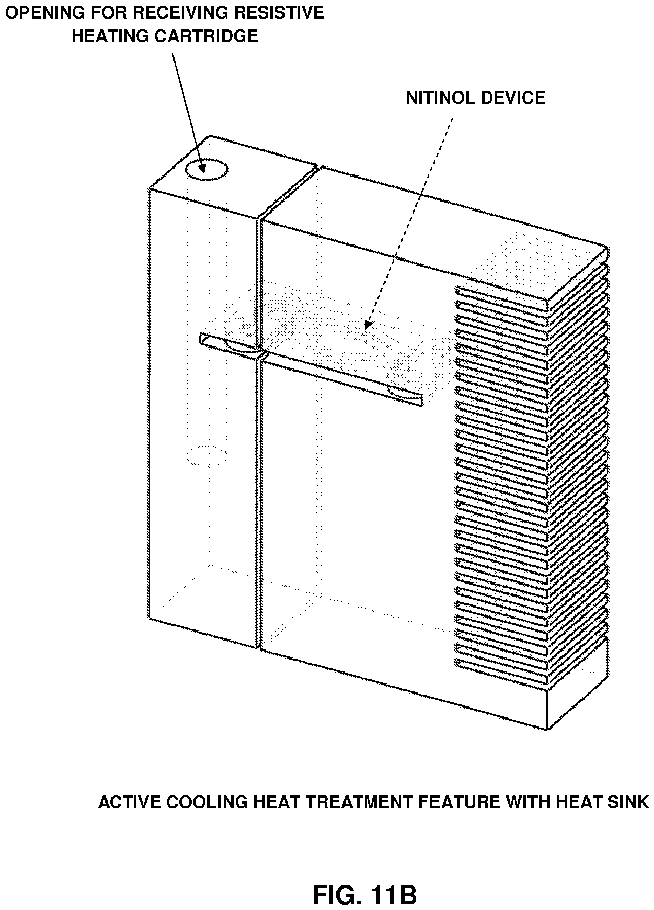

Special heat treatment fixtures can be provided to assist in the selective heat treatment of the Nitinol devices. Heat treatment fixtures can be provided that utilize resistive heating or inductive heating to selectively heat-treat regions of the Nitinol device. The Nitinol device can be heated with a fluidized sand/salt bath, or by conduction with a metallic fixture, preferably made out of aluminum or other materials with high heat-transfer coefficients. The regions where heat treating is not desired can be passively cooled by air (see Fig. 11A), or can be actively cooled with the aid of a heatsink or chiller (see Fig. 11B).

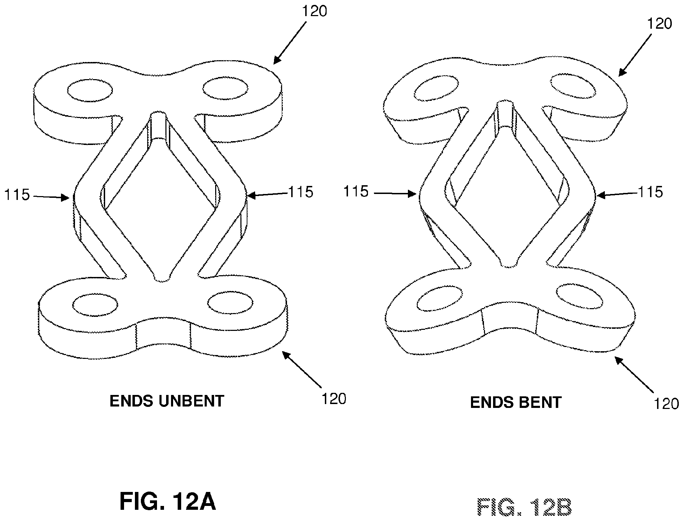

Using this heat treatment approach, it is possible to create monolithic devices (i.e., one-piece devices) that simultaneously provide both superelastic and malleable regions at body temperature. It is also possible to provide shape memory effect and malleable regions at body temperature. By way of example but not limitation, at body temperature, the Nitinol device shown in Figs. 12A and 12B may have a superelastic bridge region 115, and martensitic end regions 120 that allow the Nitinol device to be bent so as to conform to the anatomy.

### Additional Method For Controlling The Unloading Properties Of A Nitinol Device

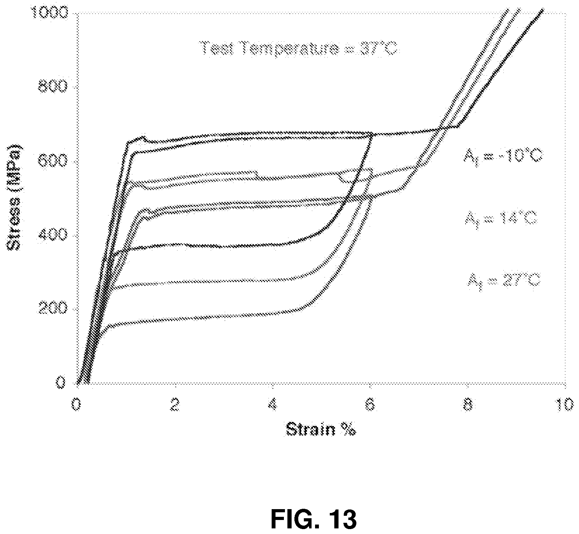

The temperature differential between (i) the body that the Nitinol device will be installed into (assumed to be 37°C, which is the temperature of a human body), and (ii) the Aε temperature of the shape memory material forming the Nitinol device affects the unloading characteristics (Fig. 13) of the Nitinol device. A smaller temperature differential between the two will result in the device generating a smaller compressive load; conversely, a larger temperature differential between the two will result in the device generating a larger compressive load.

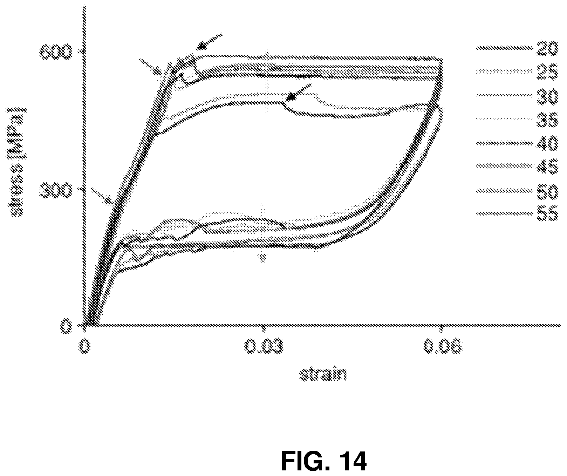

In addition to the Aε temperature of the Nitinol device, the stress levels of the upper and lower plateaus of the Nitinol's mechanical hysteresis curve are affected by the amount of "cold work" in the device (Fig. 14). Cold work is measured as the reduction in the cross-sectional area that the material experiences as part of the raw material's manufacturing. Rolling or drawing operations are typically used to produce Nitinol bar, tube and sheet material. Final cold work is typically about 30%-50%. During processing it is possible to control the amount of cold work in the material through high temperature (e.g., greater than about 600°C) annealing. This high temperature annealing erases all thermomechanical processing and returns the alloy to its ingot properties.

Reduced amounts of cold work result in reduced strength. As the percent of cold work is increased, the stress level of the upper plateau rises, and the stress level of the lower plateau falls. The percentage of cold work in the Nitinol device affects its unloading characteristics. As the percentage of cold work increases, the unloading stress decreases.

### Example 1

### Screws For Generating And Applying Compression Within A Body

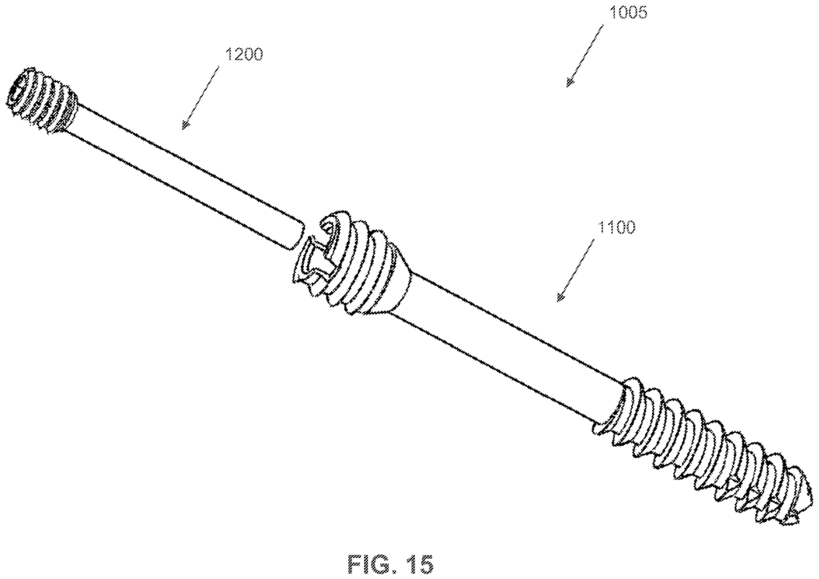

Looking next at Fig. 15, there is shown a novel compression screw system 1005 for bringing bone fragments into close proximity with each other, generating a compressive load, and maintaining that compressive load for a prolonged period of time while the bone tissue heals. Novel compression screw system 1005 generally comprises a compression screw 1100 and an internal retaining pin 1200, as will hereinafter be discussed.

In one preferred form of the invention, compression screw 1100 and internal retaining pin 1200 are provided in the form of a sterilized kit. The kit may include additional instruments to aid in the implantation of the screw (e.g., k-wire, drill bit, screw size guide, etc.).

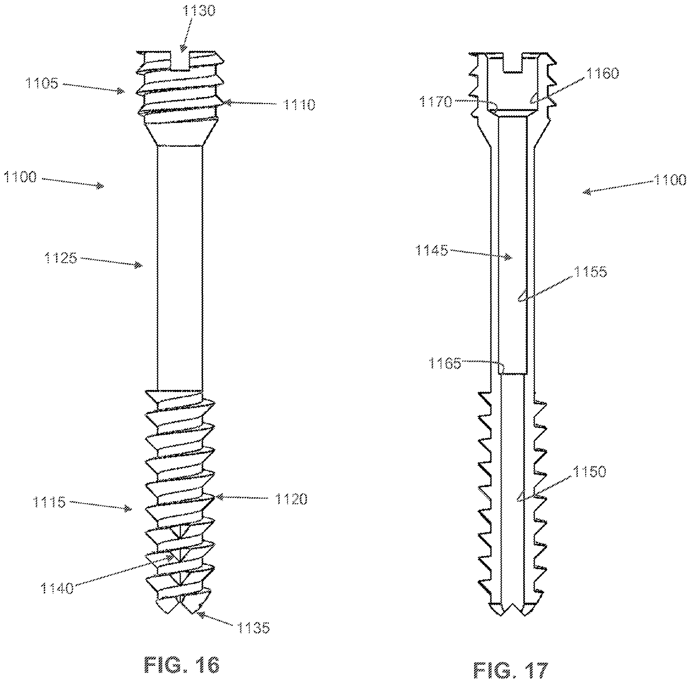

Compression screw 1100 is shown in greater detail in Figs. 16 and 17. Compression screw 1100 is manufactured from a shape memory material (e.g., a material capable of exhibiting superelasticity and/or a temperature-induced shape change). The shape memory material may be a metal alloy (e.g., Nitinol) or a polymer (e.g., appropriately processed PEEK). Compression screw 1100 is designed to engage bone fragments and generate compression between the bone fragments. Compression screw 1100 comprises a proximal threaded region 1105 comprising proximal screw thread 1110 and a distal threaded region 1115 comprising distal screw thread 1120. The thread pitch of proximal screw thread 1110 is finer than the thread pitch of distal screw thread 1120 (i.e., the thread pitch on proximal threaded region 1105 has more threads per inch than the thread pitch on distal threaded region 1115). This pitch differential aids in reducing fractures and generating compression. The respective thread geometry on proximal threaded region 1105 and distal threaded region 1115 are mirrored, creating a "book-end" effect that increases the compression holding capabilities of compression screw 1100 when compression screw 1100 extends across a fracture line in bone (e.g., the thread geometry on proximal threaded region 1105 has an incline in the proximal direction and a flat surface in the distal direction that is substantially perpendicular to the longitudinal axis of the screw; the thread geometry on distal threaded region 1105 is mirrored, having an incline in the distal direction and a flat surface in the proximal direction that is substantially perpendicular to the longitudinal axis of the screw).

Proximal threaded region 1105 and distal threaded region 1115 are connected by a hollow central bridge region 1125. Hollow central bridge region 1125 can be strained and reversibly elongated by virtue of the fact that compression screw 1100 is manufactured from a shape memory material (e.g., a material capable of exhibiting superelasticity and/or a temperature-induced shape change), which may be a metal alloy (e.g., Nitinol) or a polymer (e.g., appropriately processed PEEK). By way of example but not limitation, where compression screw 1100 is formed out of Nitinol, hollow central bridge region 1125 can be strained and reversibly elongated by up to 8% without taking a set. By straining and reversibly elongating hollow central bridge region 1125 prior to implantation across a fracture line in bone, and by thereafter releasing that strain after implantation across the fracture line, contracting hollow central bridge region 1125 can provide additional compression to the bone fracture.

Compression screw 1100 comprises a drive feature 1130 (e.g., a slot) in proximal threaded region 1105 for engagement by an appropriate driver (not shown) of the sort well known in the art, whereby to turn compression screw 1100 (e.g., into bone). Additionally, the distal threaded region 1115 of compression screw 1100 preferably comprises self-drilling features 1135 (e.g., cutting edges) of the sort well known in the screw art, and self-tapping features 1140 (e.g., flutes) of the sort well known in the screw art.

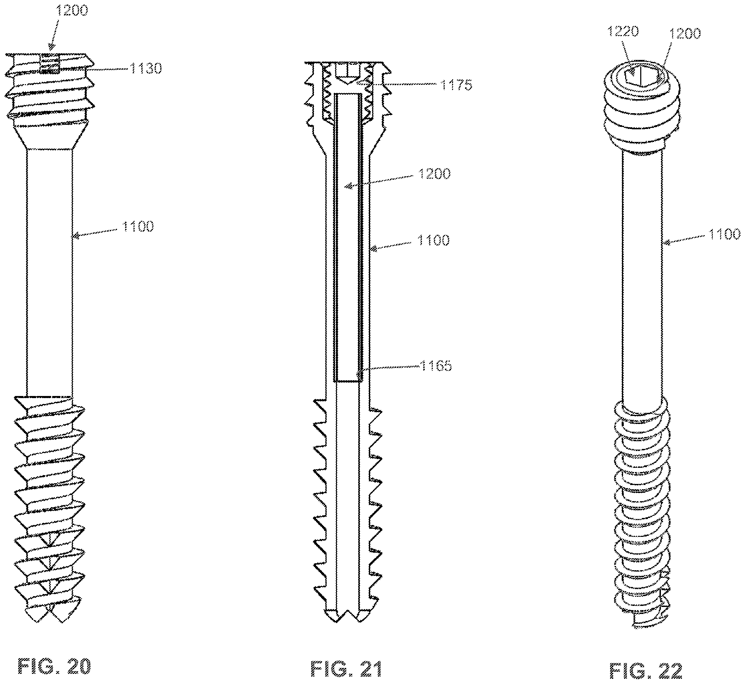

Compression screw 1100 comprises a central lumen 1145 which extends the length of the compression screw. Central lumen 1145 generally comprises a distal bore 1150, an intermediate counterbore 1155 and a proximal counterbore 1160. Intermediate counterbore 1155 has a diameter which is wider than the diameter of distal bore 1150, and an annular shoulder 1165 is formed at the intersection of distal bore 1150 and intermediate counterbore 1155. Annular shoulder 1165 preferably lies in a plane perpendicular to the longitudinal axis of the compression screw. Proximal counterbore 1160 has a diameter which is wider than the diameter of intermediate counterbore 1155, and an annular shoulder 1170 is formed at the intersection of intermediate counterbore 1155 and proximal counterbore 1160. Proximal counterbore 1160 is threaded, e.g., with an internal thread 1175 (for purposes of clarity, not shown in Fig. 17, but shown in Fig. 21).

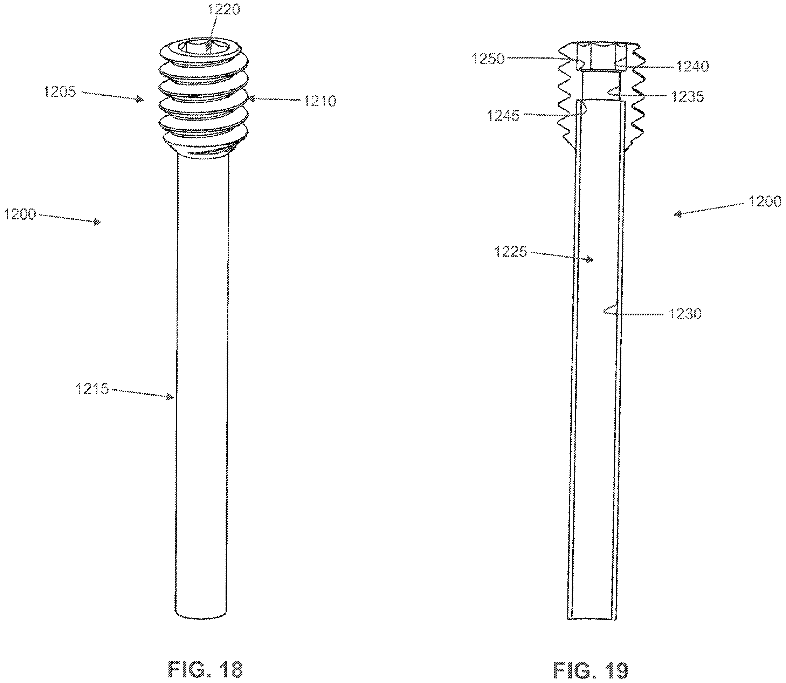

Internal retaining pin 1200 is shown in greater detail in Figs. 18 and 19. Internal retaining pin 1200 comprises a proximal threaded region 1205 comprising proximal screw thread 1210 that engages internal thread 1175 of proximal counterbore 1160 of central lumen 1145 of compression screw 1100, as will hereinafter be discussed. Internal retaining pin 1200 comprises a hollow distal cylindrical region 1215 disposed distal to proximal threaded region 1205. Hollow cylindrical region 1215 is sized to be received in central lumen 1145 of compression screw 1100 and engage annular shoulder 1165 of an already-strained (i.e., an already-stretched) compression screw 1100, as will hereinafter be discussed in further detail.

More particularly, internal retaining pin 1200 comprises proximal threaded region 1205 and distal cylindrical region 1215. Internal retaining pin 1200 is cannulated so as to allow a k-wire to be passed through internal retaining pin 1200 as will hereinafter be discussed, and has a drive feature 1220 in its threaded proximal region 1205 to facilitate turning internal retaining pin 1200, whereby to insert internal retaining pin 1200 into compression screw 1100 or to remove internal retaining pin 1200 from compression screw 1100, as will hereinafter be discussed. To this end, internal retaining pin 1200 comprises a central lumen 1225 which extends the length of internal retaining pin 1200. Central lumen 1225 preferably comprises a distal section 1230, an intermediate section 1235 and a proximal section 1240. Distal section 1230 has a diameter which is wider than the diameter of intermediate section 1235, and a shoulder 1245 is formed at the intersection of distal section 1230 and intermediate section 1235. Proximal section 1240 has a widest diameter which is wider than the diameter of intermediate section 1235, and a shoulder 1250 is formed at the intersection of intermediate section 1235 and proximal section 1240. Proximal section 1240 has a hexagonal (or other non-circular) cross-section, whereby to provide the aforementioned drive feature 1220, whereby to enable turning internal retaining pin 1200 into, or out of, compression screw 1100, as will hereinafter be discussed.

As seen in Figs. 20-22, internal retaining pin 1200 is configured to be selectively inserted into compression screw 1100. More particularly, internal retaining pin 1200 is configured to be selectively threaded into compression screw 1100 so that distal cylindrical region 1215 of internal retaining pin 1200 is disposed in intermediate counterbore 1155 of compression screw 1100 when proximal threaded region 1205 of internal retaining pin 1200 is seated in proximal counterbore 1160 of compression screw 1100. As will hereinafter be discussed in further detail, internal retaining pin 1200 is sized so that when compression screw 1100 is strained (i.e., stretched) and proximal threaded region 1205 of internal retaining pin 1200 is fully seated in proximal counterbore 1160 of compression screw 1100, the distal end of distal cylindrical region 1215 of internal retaining pin 1200 abuts annular shoulder 1165 of compression screw 1100. See Fig. 21.

Internal retaining pin 1200 is provided so as to selectively maintain compression screw 1100 in a strained (i.e., stretched) configuration and, when internal retaining pin 1200 is removed from compression screw 1100, to allow compression screw 1100 to attempt to return to its unstrained (i.e., unstretched) configuration.

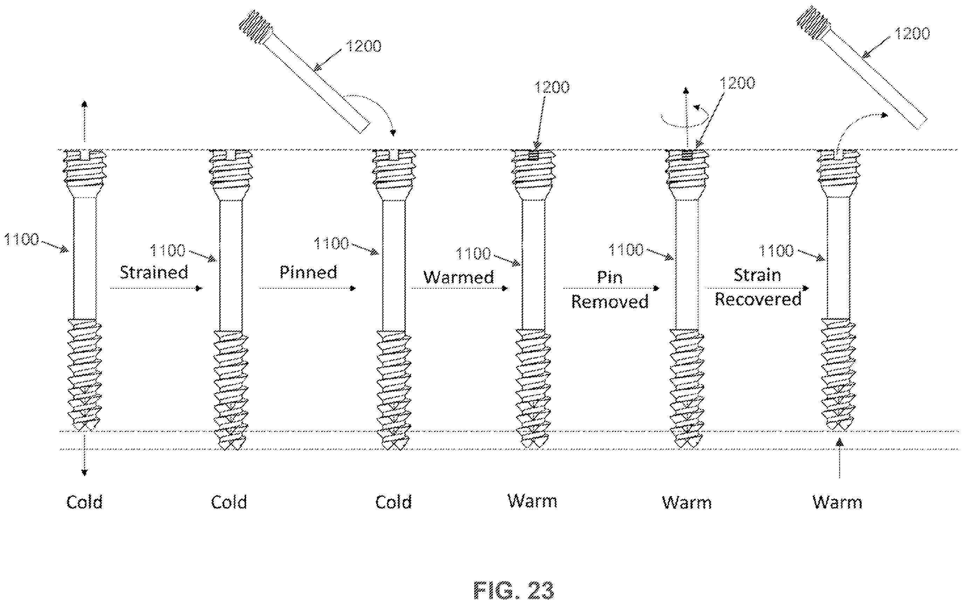

More particularly, and looking now at Fig. 23, with compression screw 1100 maintained at a temperature below its austenite start temperature, more preferably below its martensite start temperature, and most preferably below is martensite finish temperature, compression screw 1100 is gripped by its proximal threaded region 1105 and its distal threaded region 1115 and strained (i.e., stretched) using a stretching mechanism (not shown) of the sort which will be apparent to those skilled in the art in view of the present disclosure. As long as the temperature of compression screw 1100 is maintained below the austenite start temperature, compression screw 1100 will remain strained (i.e., stretched) even after the external strain load is removed.

Straining compression screw 1100 in the "cold" condition is preferable because it takes considerably less force to strain the non-austenitic material. The load that is required to stretch the compression screw may be less than half that required to stress the material in its austenitic phase. Furthermore, the surface finish of the compression screw is critical to its biocompatibility. Prior to straining, the compression screw may be passivated to remove embedded surface contaminants that may have resulted from the manufacturing process. Passivation also creates a biocompatible oxide layer on the surface of the screw. Straining the compression screw with a high load (i.e., the type of high load required to stress the compression screw in an austenitic phase) can damage this biocompatible oxide layer, and can embed particles into the surface of the threaded regions of the compression screw. Lower loads (i.e., the type of loads required to stress the compression screw in a non-austenitic phase) will minimize any damage to the surface finish.

With compression screw 1100 "cold" (i.e., maintained below its austenite start temperature, more preferably below its martensite start temperature, and most preferably below its martensite finish temperature) and strained (i.e., stretched), internal retaining pin 1200 is installed in compression screw 1100. More particularly, with compression screw 1100 maintained below its austenite start temperature, internal retaining pin 1200 is advanced into compression screw 1100 so that proximal threaded region 1205 of internal retaining pin 1200 is fully seated in threaded proximal counterbore 1160 of compression screw 1100, and so that the distal end of internal retaining pin 1200 abuts annular shoulder 1165 in compression screw 1100, whereby to lock compression screw 1100 in its strained (i.e., stretched) state.

Compression screw 1100 can now be warmed above its austenite start temperature and compression screw 1100 will not foreshorten due to the presence of internal retaining pin 1200 within compression screw 1100.

However, when internal retaining pin 1200 is thereafter removed from compression screw 1100, compression screw 1100 will attempt to revert back to its non-strained (i.e., unstretched) length, i.e., compression screw 1100 will attempt to foreshorten. As a result, when a strained compression screw 1100 is deployed across the fracture line in bone and internal retaining pin 1200 is thereafter removed, the compressive force generated by the differential pitch of proximal threaded region 1105 and distal threaded region 1115 is supplemented by the additional compressive force created by the foreshortening of hollow central bridge region 1125.

Note that compression screw 1100 is configured so that the force that is generated as compression screw 1100 foreshortens is less than the pullout force of distal threaded region 1115 and proximal threaded region 1105, so that compression screw 1100 does not "tear through" the bone tissue when attempting to foreshorten. More particularly, compression screw 1100 is specifically engineered so not to "tear through" the bone tissue. The compressive forces of compression screw 1100 can be controlled by modulating the material properties of the compression screw and/or the geometry of the compression screw. The percentage of cold work in the shape memory material forming compression screw 1100 affects the compressive force generated by the compression screw. As the percentage of cold work increases, the compression force declines. A compression screw should, preferably, have between about 15% and 55% cold work to control the recovery force of the compression screw.

Another material property that affects the screw's compression force is the temperature differential between the body that the compression screw will be implanted into (assumed to be 37°C, which is the temperature of a human body) and the austenite finish temperature of the shape memory material forming compression screw 1100. A smaller temperature differential between the two will result in the screw generating a small compressive load; conversely, the larger the temperature differential between the two will result in a screw generating a larger compressive load. The shape memory material that the compression screw is made out of should, preferably, have an austenite finish temperature of greater than about -10°C, resulting in a temperature differential of less than about 47°C when the compression screw is implanted (assuming that the compression screw is implanted in a human body).

Screw geometry also affects the compression force generated. The cross-sectional area of the hollow central bridge region 1125 affects the compression force. As the cross-sectional area increases, so does the compression force that the compression screw 1100 will generate. In this respect it should be appreciated that it is beneficial for the compression force generated by foreshortening compression screw 1100 to be constant as the bone relaxes and remodels. Thus, the cross-section of hollow central bridge region 1125 of compression screw 1100 preferably has a constant cross-section over its entire length. Cross-sections that are not uniform over the length of hollow central bridge region 1125 can result in an increase or decrease in compression as the compression screw shortens.

The screws threads are critical for transmitting the compression force to the bone without "tearing through" the bone. The height of the screw threads, the number of threads per inch (pitch), and the geometry of the screw threads are all critical to the screw's ability to not "tear through" the bone. The proximal and distal threaded regions may be of different lengths. The length of the distal thread region may be equal to or greater than the length of the proximal thread region. The distal thread region should, preferably, be at least 20% of the total screw length. Additionally, the thread height of the distal threads should, preferably, be equal to or greater than the thread height of the proximal threads.

The distal thread geometry may also be mirrored from that of the proximal thread geometry. This creates threaded regions where the load-bearing thread face is nearly perpendicular to the longitudinal axis of the compression screw. The resulting thread form has high shear strength.

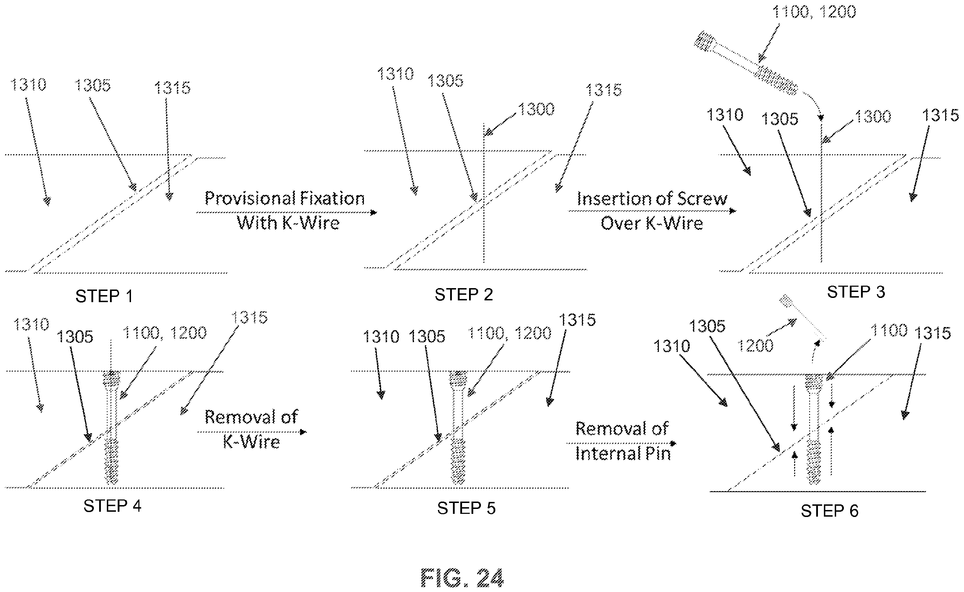

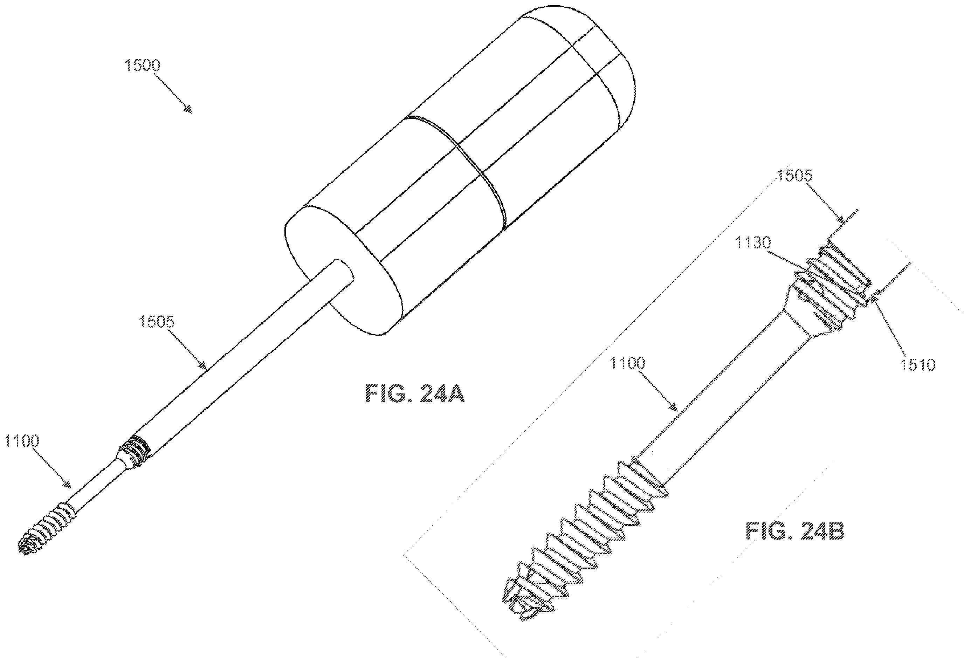

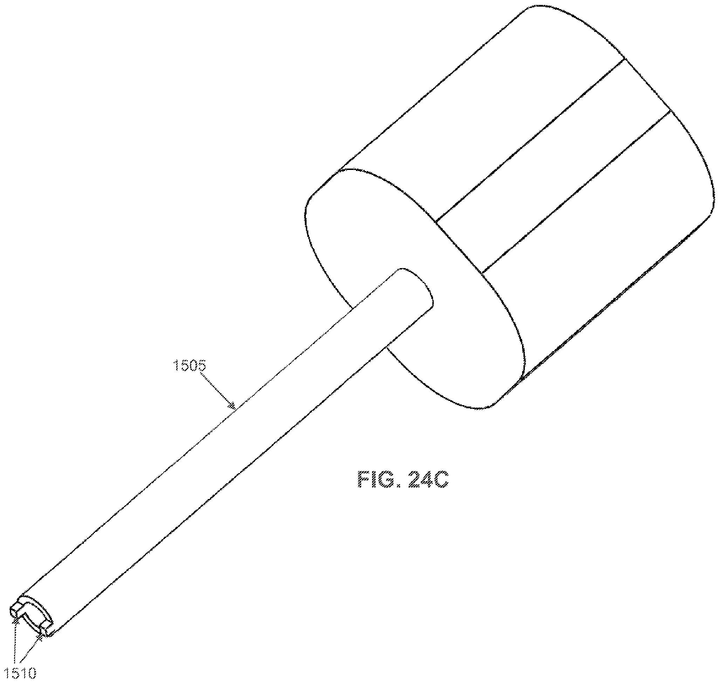

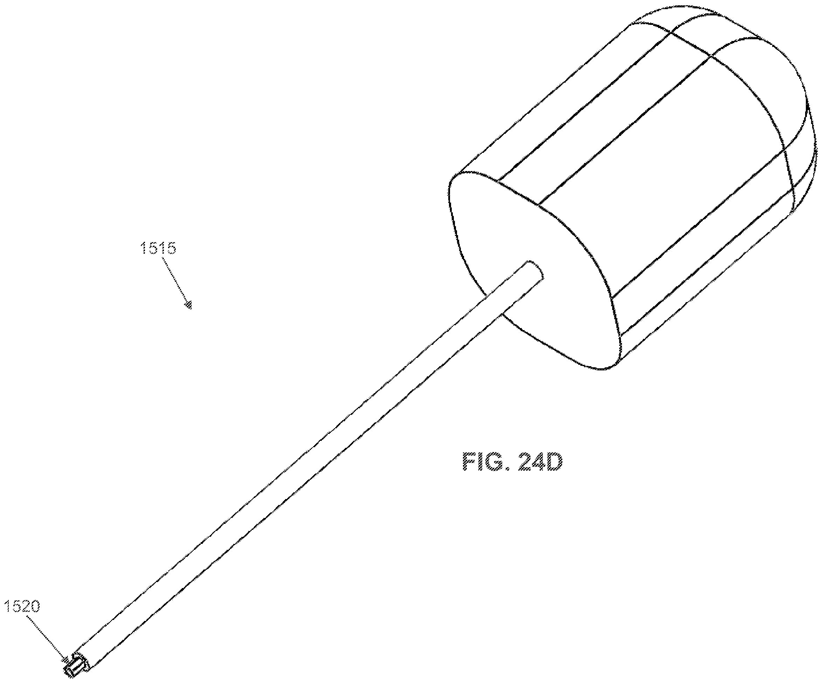

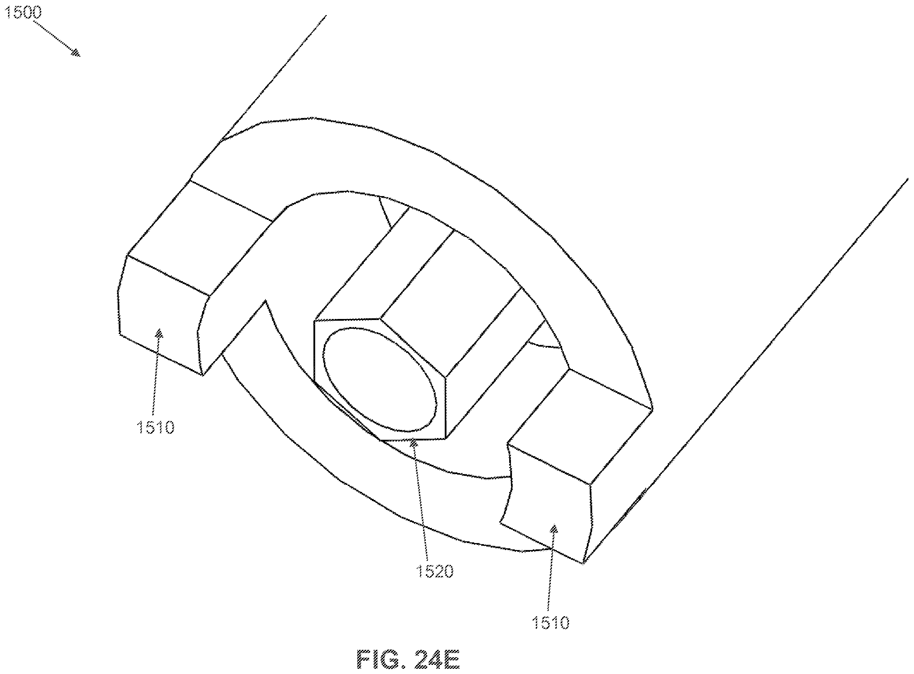

Looking now at Fig. 24, compression screw system 1005 can be used to aid in the healing of fractured bone, e.g., the scaphoid of the wrist, diaphysis of the fifth metatarsal, and the proximal interphalangeal joint of the second, third, fourth or fifth toe. More particularly, in one preferred form of the invention, a k-wire 1300 is inserted across a fracture line 1305 to provisionally stabilize bone fragments 1310, 1315. A strained (i.e., stretched) compression screw 1100, with internal retaining pin 1200 disposed therein, is then slid over k-wire 1300 and threaded into bone fragments 1310, 1315 so that compression screw 1100 extends across fracture line 1305. The differential pitch between proximal threaded region 1105 and distal threaded region 1115 of compression screw 1100 creates compression across fracture line 1305 and reduces the fracture. With compression screw 1100 thoroughly countersunk, k-wire 1300 is removed. Next, internal retaining pin 1200 is removed from compression screw 1100, whereupon the strained (i.e., stretched) compression screw 1100 will attempt to foreshorten to its pre-strained (i.e., pre-stretched) condition. Inasmuch as proximal threaded region 1105 and distal threaded region 1115 of compression screw 1100 are disposed in bone fragments 1310, 1315, respectively, the foreshortening compression screw will further reduce the fracture if a gap exists, and generate and maintain additional compressive load across the fracture line 1305, thereby enhancing healing.

As noted above, compression screw 1100 is provided with a drive feature 1130, whereby to turn compression screw 1100 into bone. Drive feature 1130 can be a standard screw drive feature such as a drive slot, a Philips (cruciform) drive configuration, a hex or hexalobe recess, or other engagement feature of the sort well known in the art.

As also noted above, internal retaining pin 1200 is provided with a drive feature 1220, whereby to advance internal retaining pin 1200 into compression screw 1100 or to remove internal retaining pin 1200 from compression screw 1100. While drive feature 1220 is shown herein as comprising a hex recess, other drive features of the sort well known in the art may also be used.

In addition, as noted above, internal retaining pin 1200 is preferably releasably secured to compression screw 1100 via a screw mechanism (e.g., proximal screw thread 1210 of internal retaining pin 1200 engaging internal thread 1175 of compression screw 1100). However, if desired, other connection mechanisms (e.g., a bayonet mount) may be used to releasably connect internal retaining pin 1200 to compression screw 1100.

It should also be appreciated that compression screw system 1005 need not be delivered over a k-wire. In this case, compression screw 1100 and internal retaining pin 1200 need not be fully cannulated.

In addition to the foregoing, in the preceding section, compression screw 1100 is described as being strained (i.e., stretched) prior to the insertion of internal retaining pin 1200 into the compression screw. However, if desired, internal retaining pin 1200 can be inserted into compression screw 1100 without compression screw 1100 being pre-strained (i.e., without compression screw 1100 being pre-stretched), in which case internal retaining pin 1200 can be used to strain (i.e., stretch) compression screw 1100, i.e., by virtue of the engagement of the advancing distal tip of internal retaining pin 1200 with annular shoulder 1165 of compression screw 1100.

And in the foregoing description, compression screw 1100 is described as being stretched while it is at a temperature below its austenite start temperature, and with the internal retaining pin being inserted into the compression screw while the compression screw is maintained below its austenite start temperature. However, if desired, compression screw 1100 may be stretched while it is at a temperature above its austenite start temperature, whereby to create stress-induced martensite, with the internal retaining pin being inserted into the compression screw while the compression screw is maintained in its stretched condition.

If desired, compression screw system 1005 may be deployed using two separate drivers, i.e., a first driver for deploying compression screw 1100 into the bone and a second driver for removing internal retaining pin 1200 from the deployed compression screw 1100. However, if desired, a single two-part driver may be used, where one part of the two-part driver deploys the compression screw 1100 into the bone and a second part of the two-part driver removes internal retaining pin 1200 from the deployed compression screw 1100.

By way of example but not limitation, and looking now at Figs. 24A-24E, there is shown a two-part driver 1500 which may be used to deploy compression screw 1100. Two-part driver 1500 comprises a first drive element 1505 which is cannulated and includes fingers 1510 at its distal end for engaging drive feature 1130 in compression screw 1100. Two-part driver 1500 also includes a second drive element 1515 which is sized to fit within the cannulation of first drive element 1505 and comprises a hexagonal (or other non-circular) projection 1520 configured to be received within drive feature 1220 of internal retaining pin 1200. In this form of the invention, second drive element 1515 is loaded into first drive element 1505 so that projection 1520 of second drive element 1520 sits adjacent to, but radially inboard, of fingers 1510 of first drive element 1505, the pre-strained compression screw 1100 (carrying internal retaining pin 1200 therein) is mated with two-part driver 1500 so that fingers 1510 of first drive element 1505 mate with drive feature 1130 of compression screw 1100, and so that projection 1520 of second drive element 1515 mates with drive feature 1220 of internal retaining pin 1200. Then two-part driver 1500 is used to install the pre-strained compression screw 1100 (carrying internal retaining pin 1200 therein) in bone, i.e., by first turning compression screw 1100 with first drive element 1505 so as to advance compression screw 1100 into bone, and then turning second drive element 1515 so as to withdraw internal retaining pin 1200 from compression screw 1100, whereby to permit compression screw 1100 to foreshorten. It may be beneficial to hold first drive element 1505 stationary while turning second drive element 1515, so as not to "back-out" the compression screw 1100 while loosening internal retaining pin 1200.

It should be appreciated that with the compression screw system 1005 shown in Fig. 15, internal retaining pin 1200 is held in position relative to compression screw 1100 by virtue of the engagement of proximal screw thread 1210 of proximal threaded region 1205 of internal retaining pin 1200 with internal thread 1175 of proximal counterbore 1160 in compression screw 1100.

In another form of the invention, internal retaining pin 1200 can be incorporated as part of a single-use delivery device, where portions of the single-use delivery device engage compression screw 1100 and hold compression screw 1100 in its stretched condition until compression screw 1100 is released from the delivery device, as will hereinafter be discussed in further detail. Preferably, in this form of the invention, compression screw 1100 is provided pre-strained and pre-loaded onto the delivery device, which is preferably a single-use delivery device that may be disposable.

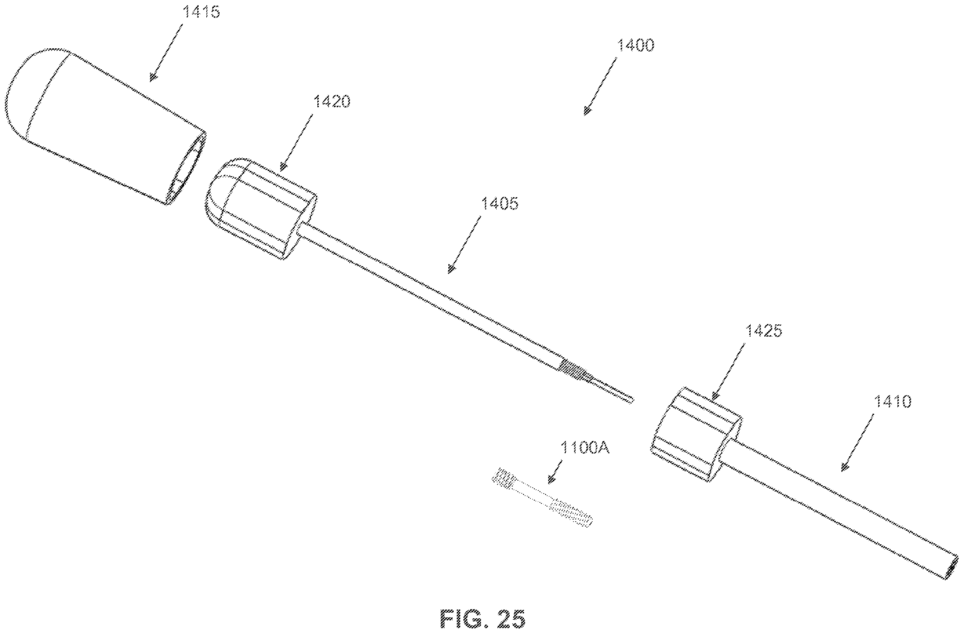

More particularly, and looking now at Fig. 25, there is shown a novel compression screw system 1400 formed in accordance with the present invention. Novel compression screw system 1400 comprises a compression screw 1100A, a cannulated inner driver 1405, a cannulated outer driver 1410, and a coupling cap 1415. Cannulated inner driver 1405, cannulated outer driver 1410, and coupling cap 1415 essentially comprise a delivery device for delivering compression screw 1100A to a fracture site as will hereinafter be discussed. Compression screw 1100A is substantially the same as the compression screw 1100 discussed above, except as will hereinafter be discussed. Pre-strained (e.g., stretched) compression screw 1100A is slidably mounted to cannulated inner driver 1405 and is selectively secured to cannulated inner driver 1405 by cannulated outer driver 1410, as will hereinafter be discussed. Cannulated outer driver 1410 fits over cannulated inner driver 1405, and coupling cap 1415 fits over the drive handle 1420 of cannulated inner driver 1405 and over the drive handle 1425 of cannulated outer driver 1410. With coupling cap 1415 installed, cannulated inner driver 1405 and cannulated outer driver 1410 may be rotated as a unit. When the coupling cap 1415 is removed, cannulated outer driver 1410 may be held stationary and cannulated inner driver 1405 may be rotated independently of cannulated outer driver 1410.

In one preferred form of the invention, compression screw 1100A, cannulated inner driver 1405, cannulated outer driver 1410 and coupling cap 1415 are provided in the form of a sterilized kit. The kit may include additional instruments to aid in the implantation of the screw (e.g., k-wire, drill bit, screw size guide, etc.).

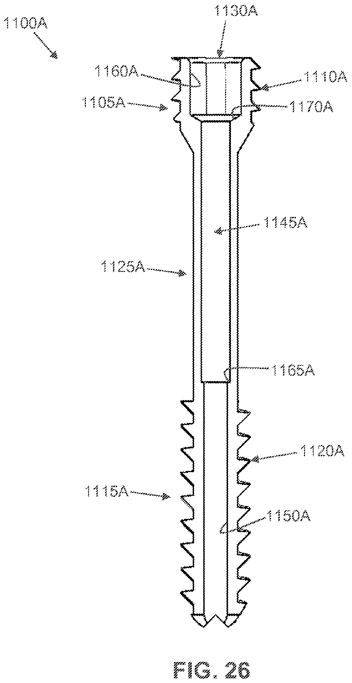

Looking next at Fig. 26, compression screw 1100A is substantially identical to compression screw 1100 discussed above, except that proximal counterbore 1160A of compression screw 1100A (i) omits the internal screw thread 1175 of compression screw 1100, and (ii) comprises the drive feature 1130A. To this end, proximal counterbore 1160A comprises a non-circular cross-section (e.g., hexagonal, hexalobe, etc.). In one preferred form of the invention, proximal counterbore 1160A has a hexagonal cross-section, whereby to provide the drive feature 1130A.

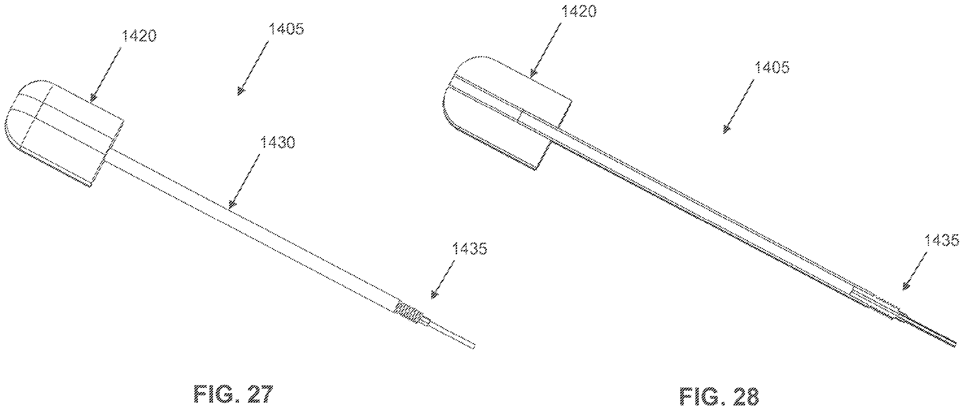

Cannulated inner driver 1405 is shown in greater detail in Figs. 27-29. Cannulated inner driver 1405 comprises a shaft 1430, a compression screw interface region 1435 disposed at the distal end of shaft 1430, and the aforementioned handle 1420. Cannulated inner driver 1405 is fully cannulated so as to allow cannulated inner driver 1405 to be movably mounted on a k-wire, as will hereinafter be discussed.

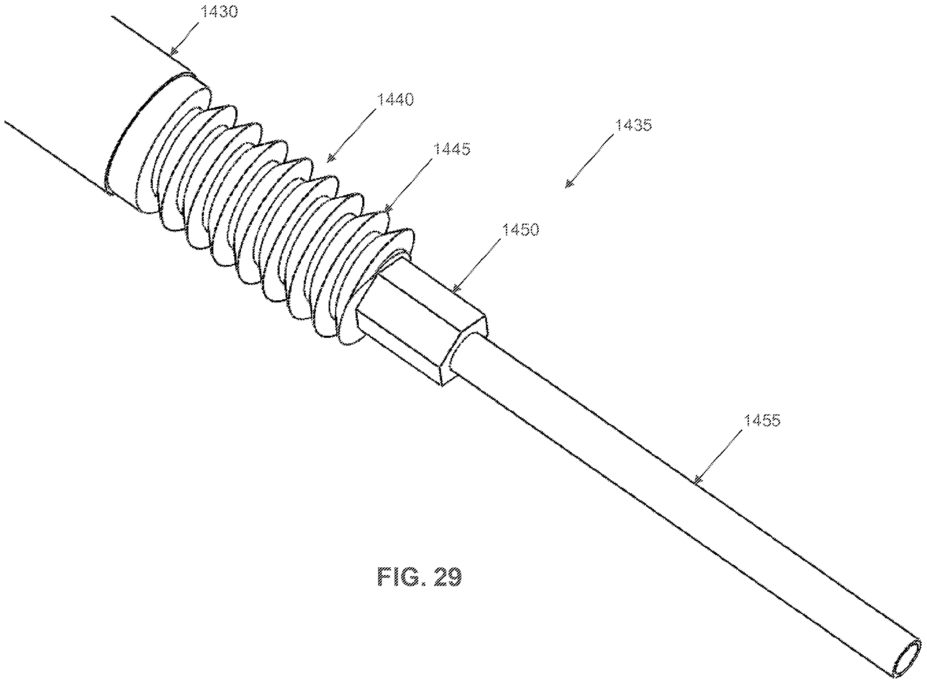

Compression screw interface region 1435 has a proximal threaded region 1440 adjacent to the distal end of shaft 1430. Proximal threaded region 1440 comprises proximal thread 1445 that has an identical pitch to proximal thread 1110A of the proximal threaded region 1105A of compression screw 1100A. Compression screw interface region 1435 also comprises a drive feature 1450 which is disposed distal to proximal thread 1445. Drive feature 1450 is designed to interface with drive feature 1130A on compression screw 1100A. By way of example but not limitation, where drive feature 1130A on compression screw 1100A comprises a hex recess, drive feature 1450 on compression screw interface region 1435 comprises a hex driver (Fig. 29). A hollow distal cylindrical region 1455 is disposed distal to drive feature 1450. Hollow distal cylindrical region 1455 is sized so that when drive feature 1450 of compression screw interface region 1435 is received in drive feature 1130A of a pre-strained (i.e., pre-stretched) compression screw 1100A, the distal end of hollow distal cylindrical region 1455 abuts annular shoulder 1165A of compression screw 1100A.

Thus, in this form of the invention, compression screw interface region 1435 provides a modified form of internal retaining pin for holding compression screw 1100A in a stretched condition. However, inasmuch as drive feature 1450 on compression screw interface region 1435 does not lock, in a longitudinal sense, to drive feature 1130A on compression screw 1100A (although it does lock, in a rotational sense, to drive feature 1130A on compression screw 1100A), cannulated outer driver 1410 is used to hold a strained (i.e., stressed) compression screw 1100A in its strained (i.e., stretched) condition on compression screw interface region 1435 until compression screw 1100A is released from cannulated inner driver 1405 and cannulated outer driver 1410, as will hereinafter be discussed in further detail.

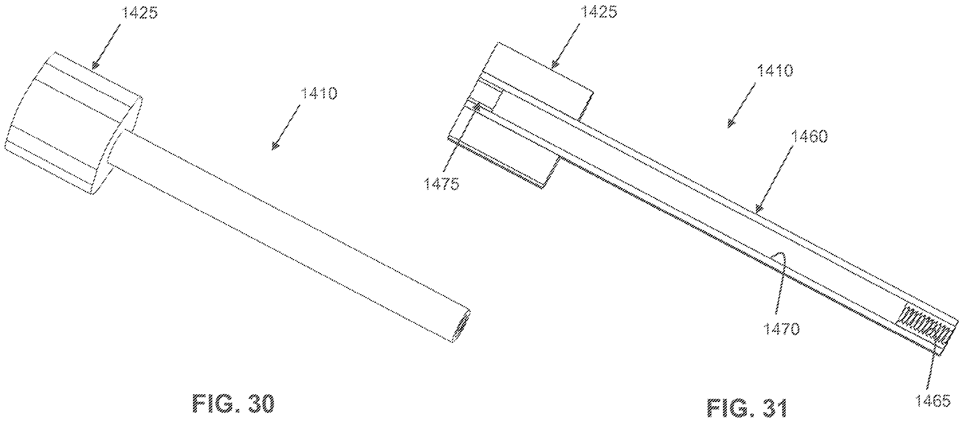

Looking next at Figs. 30 and 31, cannulated outer driver 1410 comprises the aforementioned drive handle 1425 and a length of cannulated rod 1460 extending distal to drive handle 1425. Cannulated rod 1460 comprises an internal thread 1465 at its distal end. Internal thread 1465 is sized to engage proximal thread 1445 of compression screw interface region 1435 of cannulated inner driver 1405, and to engage proximal screw thread 1110A of proximal threaded region 1105A of compression screw 1100A, whereby to allow cannulated outer driver 1410 to be secured to cannulated inner driver 1405 and to compression screw 1100A and, by doing so, to selectively secure compression screw 1100A to cannulated inner driver 1405. Cannulated outer driver 1410 comprises a bore 1470 which receives shaft 1430 of cannulated inner driver 1405, and a bearing 1475 can be pressed into bore 1470 to allow cannulated inner driver 1405 to make a close sliding fit inside cannulated outer driver 1410.

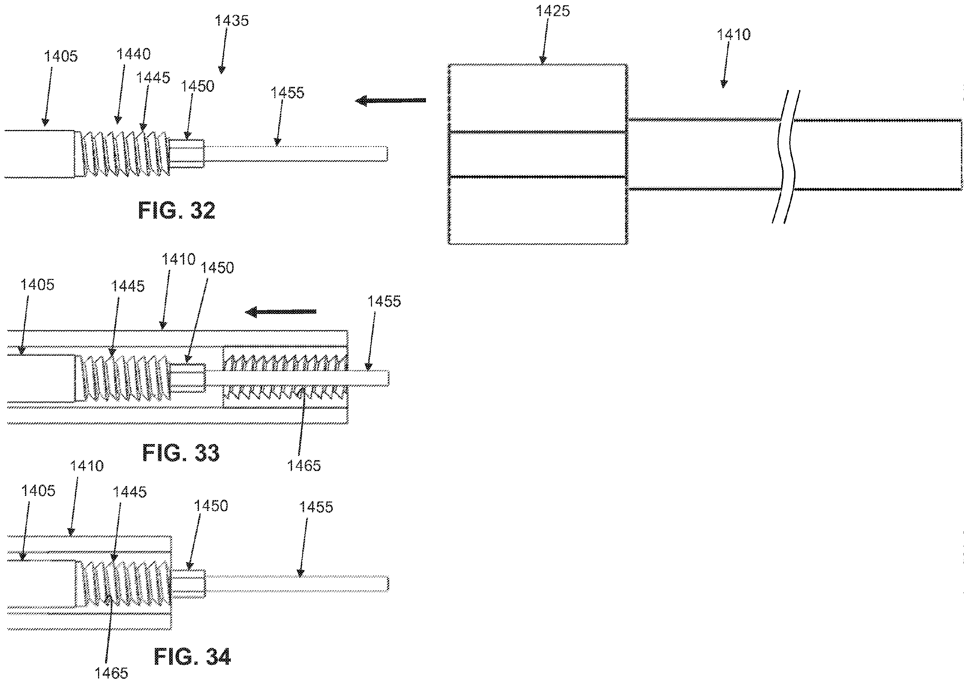

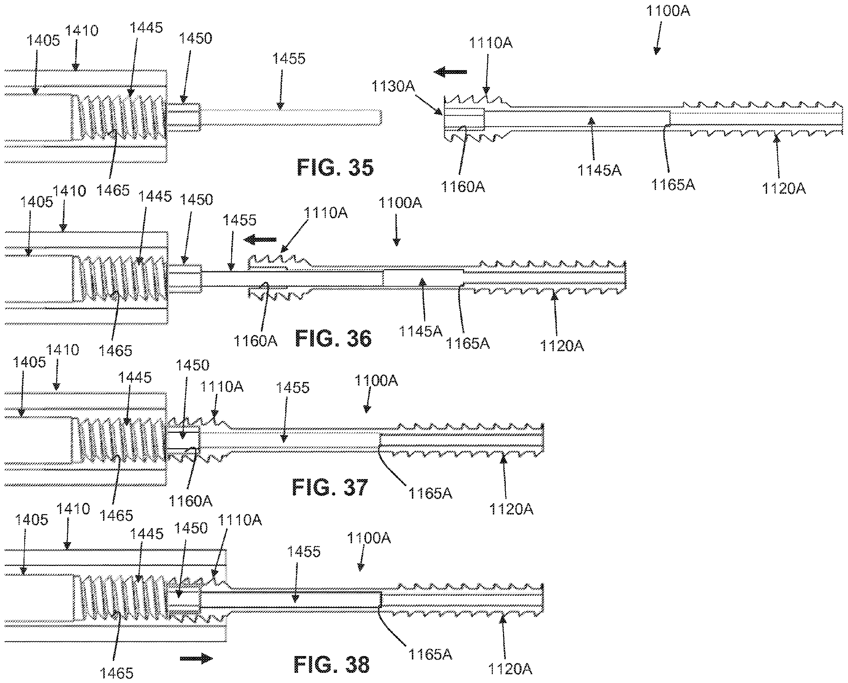

Looking next at Figs. 32-38, a strained (i.e., stretched) compression screw 1100A is intended to be loaded onto compression screw interface region 1435 of cannulated inner driver 1405, and then selectively secured thereto by means of cannulated outer driver 1410, until compression screw 1100A is to be used to secure together bone fragments. More particularly, cannulated outer driver 1410 is moved distally along cannulated inner driver 1405 until internal thread 1465 of cannulated outer driver 1410 engages proximal thread 1445 of cannulated inner driver 1405 (Figs. 32-34). Compression screw 1100A is cooled below its austenite start temperature, more preferably below its martensite start temperature, and most preferably below its martensite finish temperature, and strained (i.e., stretched) using a stretching mechanism (not shown) of the sort which will be apparent to those skilled in the art in view of the present disclosure. While "cold" (i.e., at a temperature below its austenite start temperature, more preferably below its martensite start temperature, and most preferably below its martensite finish temperature), compression screw 1100A is slid over hollow cylindrical region 1455 of compression screw interface region 1435 of cannulated inner driver 1405 until drive feature 1450 of cannulated inner driver 1405 is received in drive feature 1130A of compression screw 1100A. See Figs. 35-37. Then cannulated outer driver 1410 is advanced distally along cannulated inner driver 1405, and rotated, so that internal thread 1465 of cannulated outer driver 1410 engages proximal screw thread 1110A of compression screw 1100A. See Fig. 38. As this occurs, internal thread 1465 of cannulated outer driver 1410 remains in contact with proximal thread 1445 of cannulated inner driver 1405. Thus it will be seen that internal thread 1465 of cannulated outer driver 1410 simultaneously engages the proximal thread 1445 of cannulated inner driver 1405 and the proximal screw thread 1110A of compression screw 1100A. As a result, internal thread 1465 of cannulated outer driver 1410 effectively secures compression screw 1100A to cannulated inner driver 1405.

It will be appreciated that when compression screw 1100A is secured to cannulated inner driver 1405 by means of cannulated outer driver 1410, the distal end of hollow cylindrical region 1455 of cannulated inner driver 1405 abuts annular shoulder 1165A of compression screw 1100A, so that compression screw 1100A can be warmed to a temperature above its austenite start temperature without the compression screw shortening.

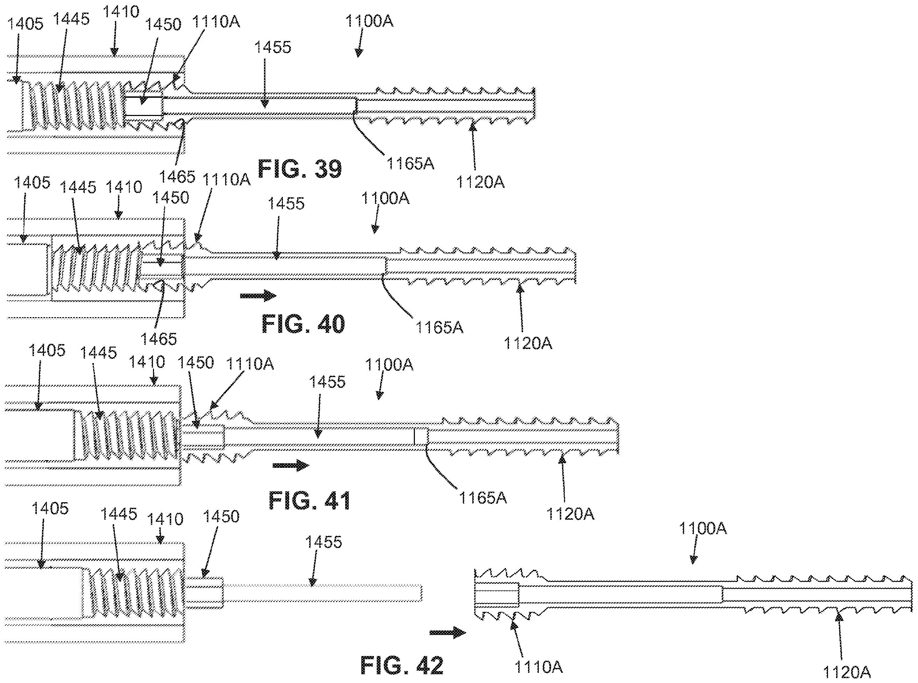

Looking next at Figs. 39-42, compression screw 1100A can be released from cannulated outer driver 1410 and cannulated inner driver 1405 by rotating cannulated inner driver so as to bring compression screw 1100A distal to cannulated outer driver 1410 (and hence disengage proximal screw thread 1110A of compression screw 1100A from internal thread 1465 of cannulated outer driver 1410). When this occurs, compression screw 1100A will no longer be mechanically prevented from foreshortening (Fig. 41) and may longitudinally separate from cannulated inner driver 1405 (Fig. 42).

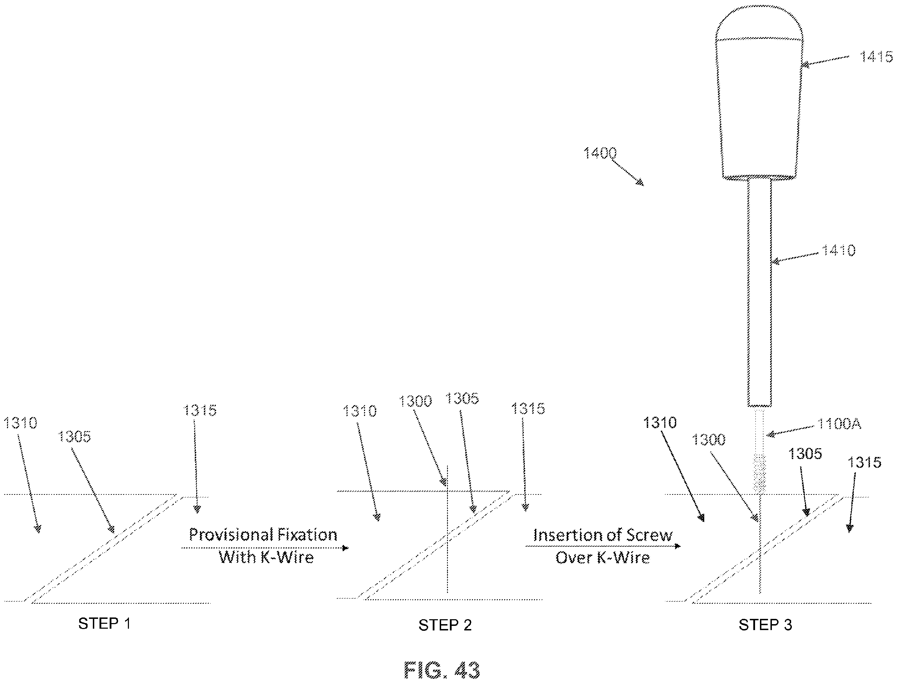

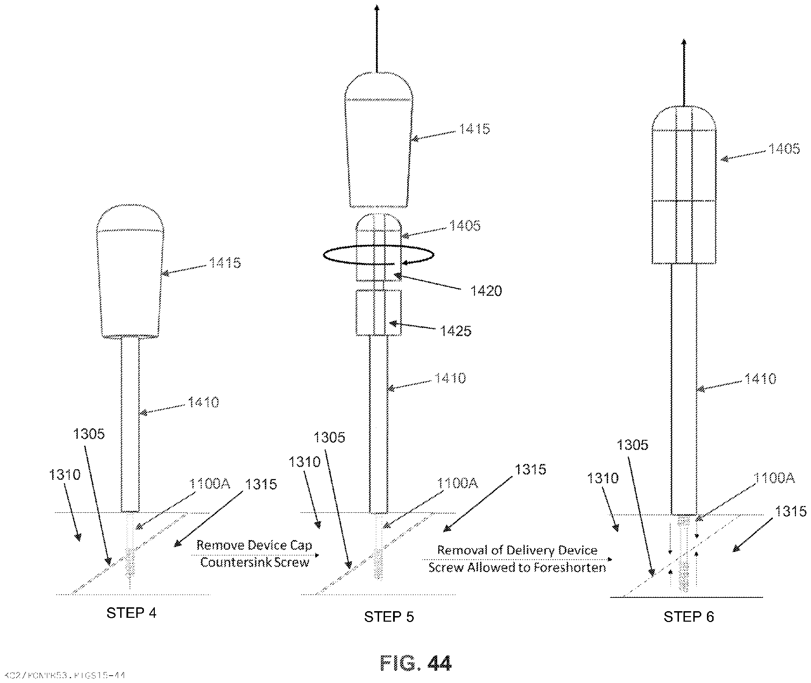

Compression screw system 1400 can be used to aid in the healing of fractured bone, e.g., the scaphoid of the wrist, diaphysis of the fifth metatarsal, and the proximal interphalangeal joint of the second, third, fourth or fifth toe. More particularly, and looking now at Figs. 43 and 44, a k-wire 1300 is first inserted across a fracture line 1305 to provisionally stabilize bone fragments 1310, 1315. Compression screw system 1400, comprising the strained (i.e., stretched) compression screw 1100A mounted on its delivery device (i.e., cannulated inner driver 1405, cannulated outer driver 1410 and coupling cap 1415), is then slid over k-wire 1300 and turned into the bone by turning coupling cap 1415.

When the distal end of cannulated outer driver 1410 contacts the cortical surface of bone fragment 1310, the compression screw 1100A can be turned further with cannulated outer driver 1410 so as to reduce the fracture and generate compression between the bone fragments 1310, 1315. More particularly, when the cannulated outer driver 1410 is turned further, compression screw 1100A pulls the bone fragments further together (i.e., in the manner of a lag screw).

Thereafter, compression screw 1100A is advanced out of cannulated outer driver 1410 and further into the bone. To this end, coupling cap 1415 is removed, and drive handle 1425 of cannulated outer driver 1410 is held stationary while drive handle 1420 of cannulated inner driver 1405 is turned, so that drive feature 1450 of cannulated inner driver 1405 turns drive feature 1130A of compression screw 1100A so as to advance compression screw 1100A along internal thread 1465 of cannulated outer driver 1410 and further into the bone. This causes cannulated inner driver 1405 to countersink compression screw 1100A. As this occurs, the differential pitch between proximal screw thread 1110A and distal screw thread 1120A further reduces the fracture and generates further compression through the aforementioned differential thread pitch.

As compression screw 1100A exits cannulated outer driver 1410 and becomes thoroughly countersunk, compression screw 1100A becomes disengaged from internal thread 1465 of cannulated outer driver 1410, thereby allowing compression screw 1100A to attempt to foreshorten. Inasmuch as proximal threaded region 1105A and distal threaded region 1115A of compression screw 1100A are disposed in bone fragments 1310, 1315, respectively, the compression screw will generate and maintain an additional compressive load across the fracture line 1305 as the compression screw foreshortens, thereby enhancing healing.

It should be appreciated that compression screw system 1400 need not be delivered over a k-wire. In this case, compression screw 1100A, cannulated inner driver 1405 and coupling cap 1415 need not be fully cannulated.

In addition to the foregoing, in the preceding section, compression screw 1100A is described as being strained (i.e., stretched) prior to being mounted to cannulated inner driver 1405. However, if desired, compression screw 1100A can be mounted to cannulated inner driver 1100A without compression screw 1100A being pre-strained (i.e., pre-stretched), in which case cannulated inner driver 1405 can be used to strain (i.e., stretch) compression screw 1100A, i.e., by virtue of the engagement of the distal tip of cannulated inner driver 1405 with annular shoulder 1165A of compression screw 1100 when cannulated outer driver 1410 engages compression screw 1100A and pulls compression screw 1100A proximally.

And in the foregoing description, compression screw 1100A is described as being stretched while it is at a temperature below its austenite start temperature, and then being loaded onto the cannulated inner driver and locked into position (i.e., with the cannulated outer driver) while the compression screw is maintained below its austenite start temperature. However, if desired, compression screw 1100A may be stretched while it is at a temperature above its austenite start temperature, whereby to create stress-induced martensite, and the compression screw mounted onto the cannulated inner driver and locked into position (i.e., with the cannulated outer driver) while the compression screw is maintained in its stretched condition.

### Test Data

It has been reported that the "mean time to union" for a non-displaced scaphoid fracture is 6 to 8 weeks. At the time of insertion, nominally similar dimensioned conventional compression screws (between 3.0 and 3.6mm distal major diameter), generate on average about 155N of compressive force. Comparatively, the compression screw system of the present invention is capable of generating additional compression (for example, about 190N) because of an increased thread pullout resistance.

Following the first 12 hours after implantation, compression decays on average by about 43% for conventional compression screws. For some conventional compression screws, that decay is as high as 55%. The compression screw system of the present invention loses only about 20% of its compression over a 12 hour period. This occurs as the bone relaxes and remodels around the threads. After this 12 hour period, a new steady state of compression of around 100N or greater of compression is achieved and maintained, and correlates with the force generated by the novel compression screw as it recovers its strain in the hollow central bridge region. This compressive load is actively maintained until the novel compression screw has fully restored the strain. It should be appreciated that the force generated by the novel compression screw as it recovers can be modified for different bone qualities and indications by adjusting the parameters discussed above.

It should be noted that larger diameter compression screws of the present invention can generate and maintain greater compressive loads, and conversely, smaller diameter compression screws of the present invention will generate and maintain lesser compressive loads. Since the recovery force is a function of the cross-sectional area of the novel compression screw's central counterbore 1145A, a cannulated 7.5mm screw (distal major diameter) can generate and maintain about 400N of force. Larger diameter compression screws of the present invention can support greater compressive loads since the threaded regions may be longer and/or deeper and thus have greater surface area to distribute the force over.

### Further Aspects

In one preferred embodiment of the present invention, the force generated by the compression screw is controlled by stretching (i.e., straining) the compression screw approximately 8%, and then allowing the compression screw to partially recover (e.g., to shorten) so that the compression screw is approximately 6% longer than its original length. With the compression screw stretched 6% longer than its original length, a constraint (e.g., the aforementioned internal retaining pin 1200) can be removably installed on the compression screw to prohibit further recovery. The partial recovery of strain (i.e., from an 8% stretch to a 6% stretch) occurs in the Load Transition zone at stress levels that may be in excess of the shear strength of bone. In other words, by allowing the compression screw to partially recover from strain prior to implantation (i.e., from an 8% stretch to a 6% stretch), the highest stress levels associated with the upper plateau at 8% stretch may be prevented from being applied to the bone, and only the lower plateau stress levels associated with the remaining 6% stretch may be applied to the bone (i.e., partial recovery of strain prior to implantation occurs in the load transition zone). Following implantation of the compression screw, the constraint (e.g., the aforementioned internal retaining pin 1200) can be removed from the compression screw, thereby allowing the compression screw to attempt to recover the remaining strain. The subsequent strain recovery will occur directly at the stress level of the lower plateau of the mechanical hysteresis curve of the Nitinol compression screw. The stress level of the lower plateau may be less than the shear strength of the bone, thereby keeping the screw threads from stripping in the bone.

By way of example but not limitation, a cannulated Nitinol headless compression screw can manufactured. The Nitinol material properties can have an upper plateau stress of 400 MPa and a lower plateau stress of 100 MPa. The hollow central region of the compression screw may have an outer diameter of 2.3 mm and an inner cannulation of 1.5 mm. Thus, the "active region" of the compression screw has a cross-sectional area of 2.4 mm², and would thus generate 955 N at the upper plateau of the mechanical hysteresis curve and 238 N at the lower plateau of the mechanical hysteresis curve.

The compression screw may have proximal threads with a major diameter of 4.6 mm, a thread depth of 0.45 mm, a thread pitch of 1.1 mm and a threaded length of 3.0 mm.

The compression screw may have distal threads with a major diameter of 3.6 mm, a thread depth of 0.65 mm, a thread pitch of 1.25 mm and a threaded length of 12 mm.

The proximal head of the compression screw will sit in cortical bone and the distal thread of the compression screw will sit mainly in cancellous bone. Normal healthy cortical bone may have a shear strength of approximately 60 MPa. Normal healthy cancellous bone may have an ultimate shear strength of approximately 4.3 MPa.

In cortical bone, the pullout force of the proximal threads is approximately 1915 N. In cancellous bone, the pullout force of the distal threads is approximately 467 N. At the level of stress of the upper plateau of the Nitinol's mechanical hysteresis curve, the proximal threads will not strip in the cortical bone as the compression screw attempts to shorten (955 N generated by compression, pullout force 1915 N) but the distal threads will strip as the compression screw attempts to shorten (955 N generated by compression, pullout force 466.9 N). However, by operating at the level of stress of the lower plateau of the Nitinol's mechanical hysteresis curve, the compression screw generates only 238 N of compression, which is less than the pullout force in the healthy cancellous bone, and thus the compression screw will not pull out.

It should be appreciated that unloading on the lower plateau of the Nitinol's mechanical hysteresis curve is even more important when the patient is osteoporotic/osteopenic. Osteoporotic and osteopenic bone will have even a lower shear strength.

### Example 2

### Staples For Generating And Applying Compression Within A Body

### Novel Staple Comprising Elastic Bridge With Two Elastic Legs

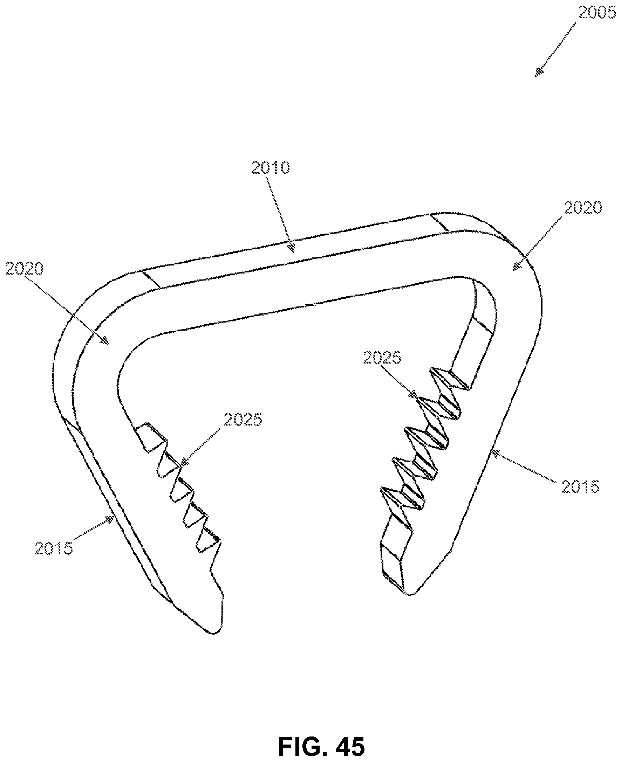

Looking next at Fig. 45, there is shown a novel staple 2005 which is able to bring bone fragments into close proximity with each other, generate a greater, and more uniform (i.e., across the cortical bone and the cancellous bone), compressive load across the fracture line, and maintain that greater, and more uniform, compressive load for a prolonged period of time while healing occurs.

Novel staple 2005 is preferably an integral, monolithic structure manufactured from a single piece of shape memory material (e.g., a material capable of exhibiting superelasticity and/or a temperature-induced shape change). The shape memory material may be a metal alloy (e.g., Nitinol) or a polymer (e.g., appropriately processed PEEK). Staple 2005 is designed to reduce fractures and generate and maintain greater, and more uniform, compression between bone fragments to aid in fracture healing. Staple 2005 comprises an elastic bridge 2010 and two elastic legs 2015. Bridge 2010 and legs 2015 meet at a pair of curved hinge regions 2020 which are also elastic. Legs 2015 may have barbed teeth 2025 to help the legs of the staple grip into the bone after implantation (see below) and prevent the legs of the staple from working their way back out of the bone. In the un-restrained state, legs 2015 of staple 2005 are bent inward with an angle of less than 90°. By way of example but not limitation, in one preferred form of the invention, legs 2015 extend at an angle of about 45° to the longitudinal axis of bridge 2010 when in their unrestrained state.

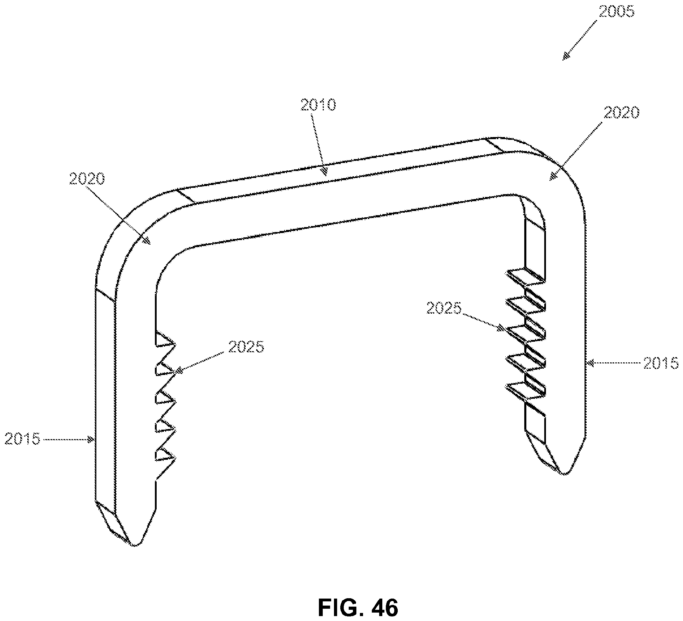

Prior to implantation, bridge 2010 of staple 2005 can be reversibly strained outward (i.e., stretched longitudinally) and legs 2015 of staple 2005 can be reversibly bent to a position substantially perpendicular to bridge 2010 (Fig. 46) so as to allow for insertion of the legs of the staple into a prepared fracture site, with the stretched bridge of the staple spanning across the fracture line (see below). Note that where staple 2005 is formed out of Nitinol, elastic deformations of up to approximately 8% are achievable. A delivery device (see below) can be used to strain bridge 2010 and to bend legs 2015, hold the staple in this strained state prior to implantation, and then insert the staple into the prepared fracture site.

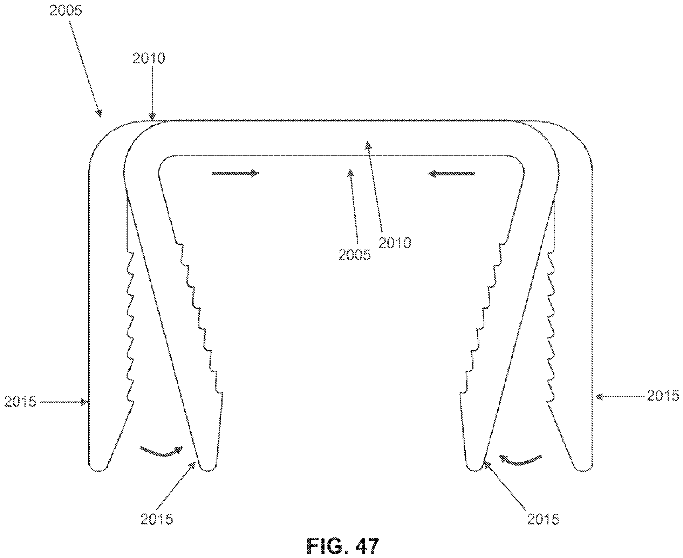

Upon insertion of the strained staple 2005 into the prepared fracture site, the constraint on bridge 2010 and legs 2015 is removed, whereupon staple 2005 attempts to return to its original un-restrained state (Fig. 47), thereby generating a greater compressive load with more uniformity along the fracture line (i.e., through legs 2015 and compressive bridge 2010), and maintaining that compressive load for a prolonged period of time while healing occurs.

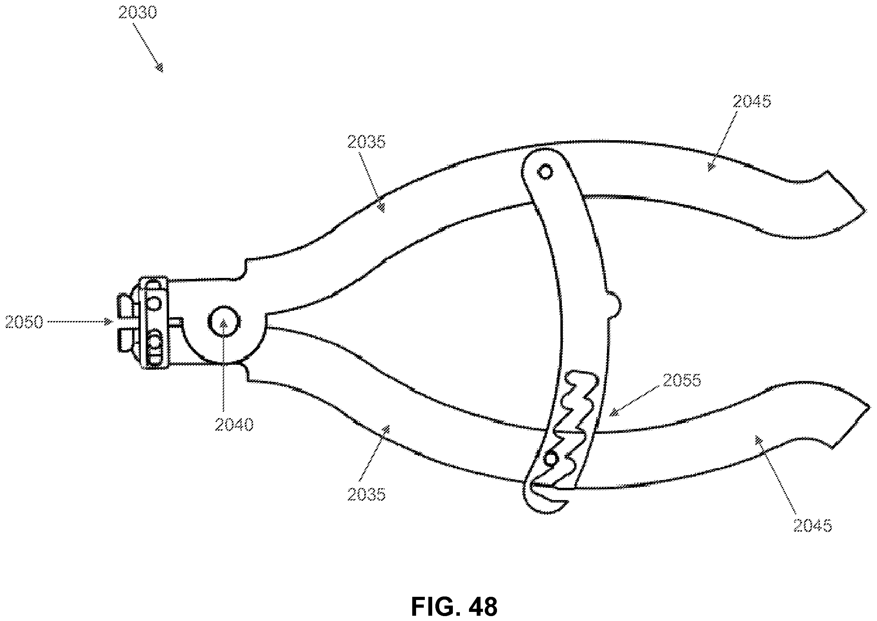

Looking next at Figs. 48-51, there is shown an exemplary delivery device 2030 which may be used to strain (i.e., stretch) bridge 2010 and bend legs 2015 of staple 2005. Delivery device 2030 comprises two arms 2035 which are pivotally connected together at a pivot pin 2040, whereby to provide a pair of handles 2045 on one end for actuating the delivery device, and a staple mount 2050 on the other end for holding and straining staple 2005. When staple 2005 is mounted to staple mount 2050 of delivery device 2030 and handles 2045 are thereafter moved toward one another, staple mount 2050 translates apart, thus stretching bridge 2010 of staple 2005, and also bending legs 2015 of staple 2005 outward to a position substantially perpendicular to the longitudinal axis of bridge 2010. Delivery device 2030 preferably includes a locking feature 2055 that facilitates holding staple 2005 in its strained state and allows for easy insertion of staple 2005 into a prepared fracture site (see below) Note that locking feature 2055 is preferably configured so that the surgeon can strain the staple to different degrees, thereby (i) enabling the surgeon to tailor the compressive force (e.g., by bending only legs 2015, or by bending legs 2015 and straining bridge 2010), and (ii) enabling the surgeon to tailor the amount of recoverable strain established across the fracture line (e.g., by varying the amount that bridge 2010 is stretched), depending on bone quality.

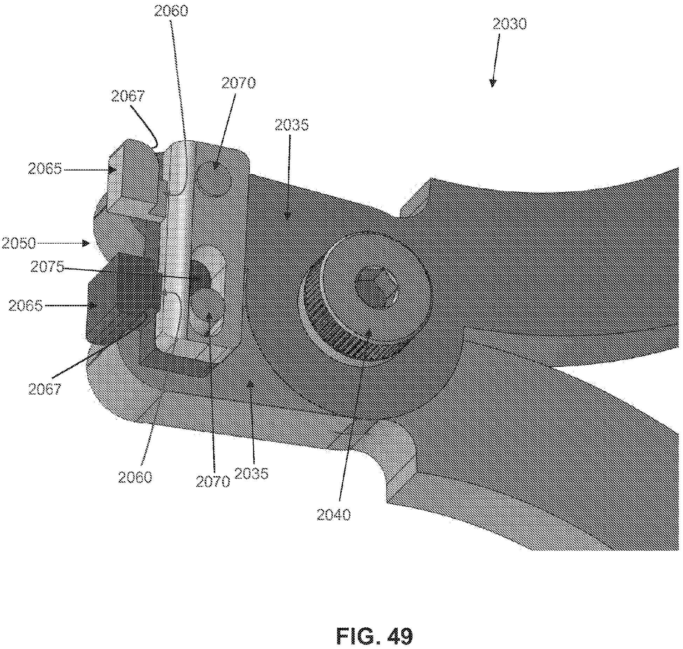

Fig. 49 shows a close-up of staple mount 2050 of delivery device 2030. Staple mount 2050 comprises a channel 2060 that receives bridge 2010 of staple 2005, and two staple-stretching linkages 2065 which sit distal to, and help define, channel 2060. The radii 2067 of staple-stretching linkages 2065 mate with the curved hinge regions 2020 of staple 2005 when the legs 2015 of the staple have been strained (i.e., bent) outward to a position substantially perpendicular to the longitudinal axis of bridge 2010. Each staple-stretching linkage 2065 is connected to the arms 2035 by a pin 2070. Pins 2070 slide in a channels 2075 provided on the staple-stretching linkages 2065 (i.e., a first pin 2070 mounted to a first staple-stretching linkage 2065 slides in a channel 2075 of the second staple-stretching linkage 2065, and a second pin 2070 mounted to the second staple-stretching linkage 2065 slides in the channel 2075 of the first staple-stretching linkage 2065). Channels 2075 are sized to limit the maximum amount of strain which may be imposed on bridge 2010 of staple 2005 by delivery device 2030 (i.e., channels 2075 limit the extent to which bridge 2010 of staple 2005 may be stretched).

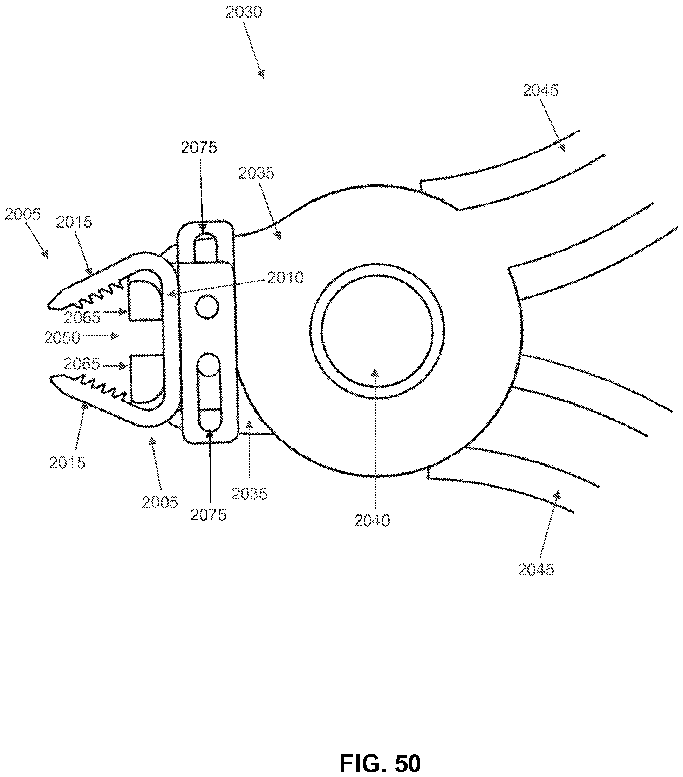

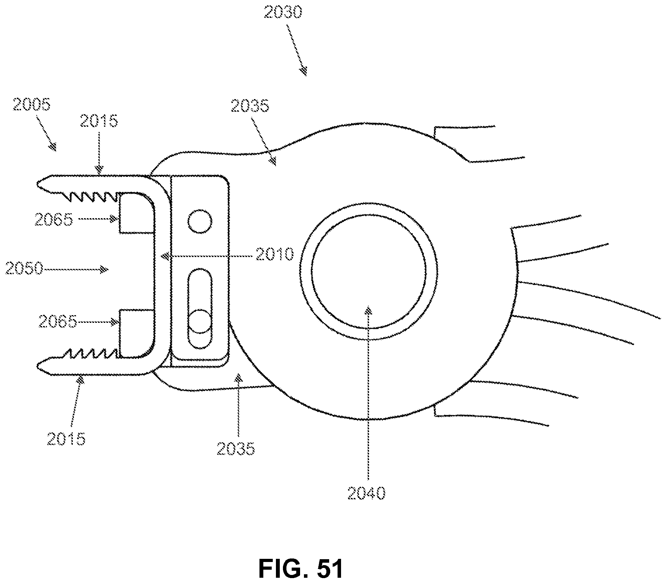

Figs. 50 and 51 show staple 2005 being loaded onto delivery device 2030 and staple 2005 being strained, i.e., bridge 2010 being stretched and legs 2015 being bent so that they are perpendicular to the longitudinal axis of bridge 2010. More particularly, Fig. 50 shows staple 2005 loaded onto staple mount 2050 of delivery device 2030 while staple mount 2050 of delivery device 2030 is in its closed (i.e., non-staple-straining) position. This is done by positioning bridge 2010 of staple 2005 in channel 2060 of staple mount 2050. Note that in this position, legs 2015 of staple 2005 are in their unbiased, pointed inward position. Fig. 51 shows staple 2005 after handles 2045 of delivery device 2030 have been moved together, so that staple mount 2050 is in its open (i.e., staple-straining) position. This is done by moving handles 2045 of delivery device 2030 together, thereby forcing staple-stretching linkages 2065 of staple mount 2050 apart, and causing bridge 2010 of staple 2005 to be stretched and causing legs 2015 of staple 2005 to be positioned substantially perpendicular to the longitudinal axis of bridge 2010.

Note that with delivery device 2030, the delivery device is constructed so that upon squeezing handles 2045, the legs of the staple are first bent to perpendicular and then, when the legs of the staple are substantially perpendicular, the bridge of the staple is elongated.

Note that staple 2005 is configured so that the force that is generated as staple 2005 reconfigures (i.e., as bridge 2010 foreshortens and legs 2015 bend inward) is less than the "tear through" force of the bone receiving legs 2015, i.e., staple 2005 is specifically engineered so as to not "tear through" the bone tissue when attempting to reconfigure. Delivery device 2030 preferably includes the aforementioned locking feature 2055 which enables the surgeon to control the extent to which the staple is strained (e.g., to bend only the legs of the staple, or to both bend the legs of the staple and strain the bridge of the staple, and to control the extent to which the bridge is stretched), thereby allowing the surgeon to tailor the compressive forces and recoverable strain imposed on the anatomy, depending on bone quality. The compressive forces of staple 2005 can be controlled by modulating the material properties of the staple and/or the geometry of the staple.

The percentage of cold work in the shape memory material forming staple 2005 affects the compressive force generated by the reconfiguring staple. As the percentage of cold work increases, the compression force declines. A staple should, preferably, have between about 15% and 55% cold work to control the recovery force of the staple; however, other degrees of cold work may be used, and/or the material may not be cold worked at all.

Another material property that affects the staple's compression force is the temperature differential between the body that the staple will be implanted into (assumed to be 37°C, which is the temperature of a human body) and the austenite finish temperature of the shape memory material forming staple 2005. A smaller temperature differential between the two will result in the staple generating a smaller compressive load; conversely, a larger temperature differential between the two will result in the staple generating a larger compressive load. The shape memory material that the staple is made out of should, preferably, have an austenite finish temperature of greater than about -10°C, resulting in a temperature differential of about 47°C when the staple is implanted (assuming that the staple is implanted in a human body).

Staple geometry also affects the compression forces generated. The cross-sectional area of bridge 2010, and the cross-sectional area of legs 2015, affects the compression forces generated by the reconfiguring staple. As the cross-sectional areas increase, so do the compression forces that the reconfiguring staple will generate.

The staple legs are critical for transmitting the compression force to the bone without "tearing through" the bone. The height, width, and length of the staple legs, and the geometry of the staple legs, are all significant relative to the staple's ability to not "tear through" the bone. Staple legs with greater surface area are better able to distribute the compression force and thus not "tear through" the bone.

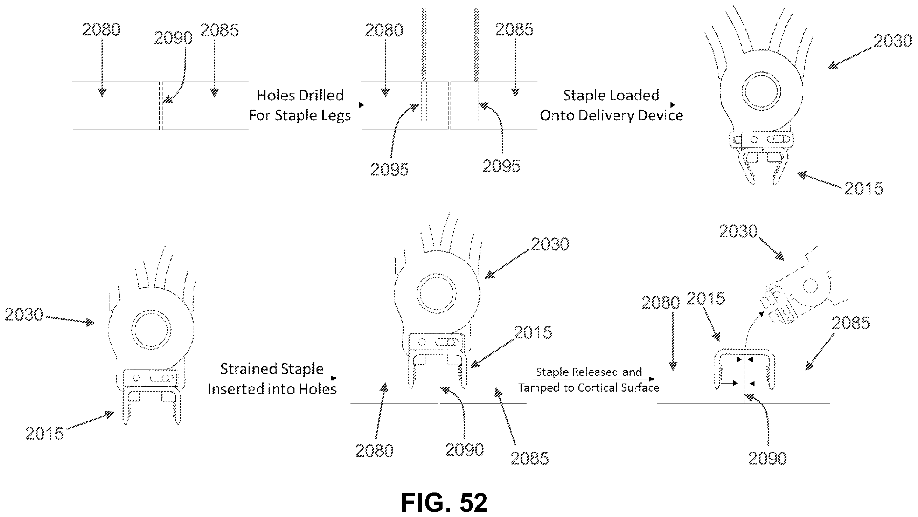

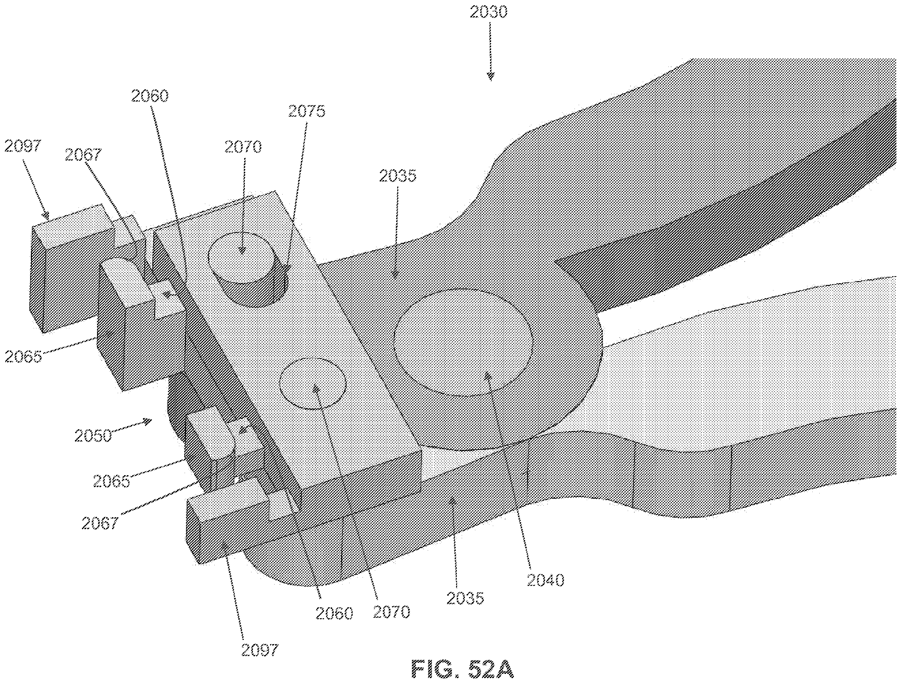

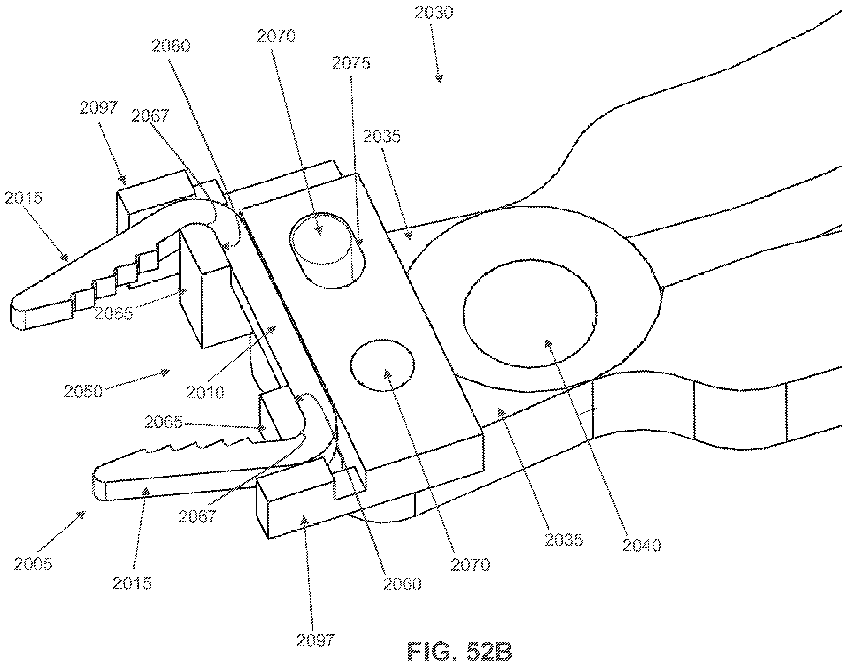

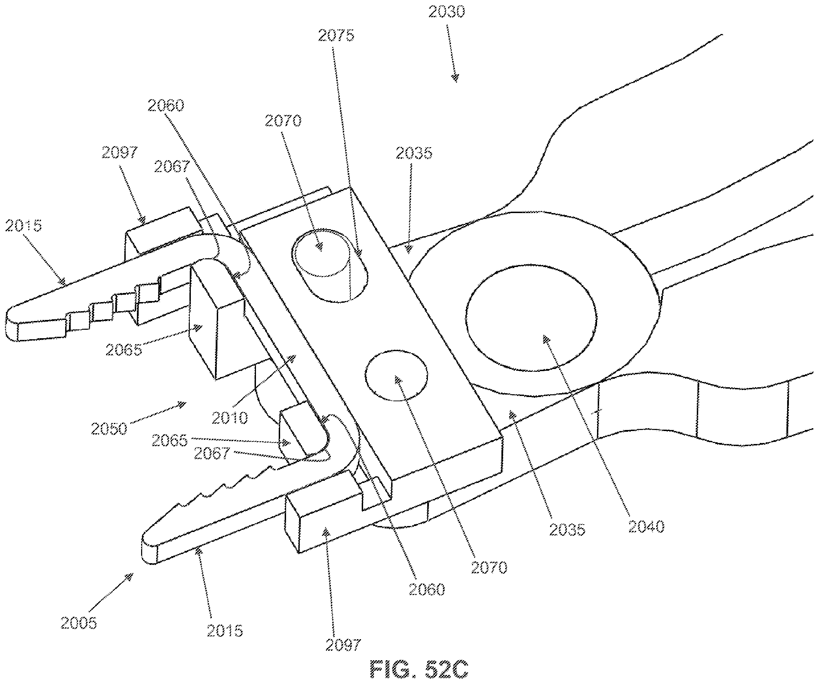

Fig. 52 shows how staple 2005 may be used to reduce a fracture and generate and maintain greater, and more uniform, compression between bone fragments 2080 and 2085 to aid in fracture healing.

More particularly, the fracture 2090 to be fused is first re-approximated and reduced. A drill guide (not shown) of the sort well known in the art is used to drill two holes 2095 the correct distance apart to accommodate the legs 2015 of the strained staple 2005. Staple 2005 is loaded onto delivery device 2030, and delivery device 2030 is used to stretch bridge 2010 and straighten legs 2015 of staple 2005 (i.e., by squeezing together handles 2045). While still on delivery device 2030, legs 2015 of staple 2005 are placed into the pre-drilled holes 2095. Staple 2005 is then released from delivery device 2030, which allows the stretched bridge 2010 of staple 2005 to foreshorten so as to apply compression to the fracture line, and which allows the strained legs 2015 of staple 2005 to "kick in" and thereby apply additional inward pressure across the fracture line 2090. Thus, staple 2005 applies more uniform compression across the fracture site, generating compression across both the cortical and intramedullary surfaces, using the compressive forces generated by the foreshortening bridge 2010 of the strained staple 2005 and using the compressive forces generated by inwardly bending legs 2015 of the strained staple 2005.

Significantly, when bridge 2010 and legs 2015 of staple 2005 generate a compressive force, both the cortical regions of the bone fragments and the cancellous regions of the bone fragments are pulled together. This provides a superior balance of compression across different regions of the bone.

It should also be appreciated that, if desired, staple 2005 can be used to attach soft tissue to bone (e.g., to attach a rotator cuff to bone).

It should be appreciated that delivery device 2030 may not always seat the staple with the bridge of the staple seated directly against the cortical surface of the bone (i.e., the bridge of the staple may sit slightly above the cortical surface of the bone). Therefore, a tamp of the sort well known in the art may be used to fully seat the staple bridge against the cortical surface of the bone.

In some circumstances it can be desirable to modify delivery device 2030 so as to ensure that legs 2015 do not be bent past 90 degrees (relative to the longitudinal axis of bridge 2010) when staple 2005 is strained. More particularly, in some constructions, staple 2005 can require more force to stretch bridge 2010 than to bend legs 2015. In this circumstance, there is the possibility that legs 2015 will be bent to 90 degrees (relative to the longitudinal axis of bridge 2010) and then, as bridge 2010 is stretched, legs 2015 may be bent past 90 degrees (relative to the longitudinal axis of bridge 2010). Therefore, it can be desirable to provide means for preventing legs 2015 from being bent past 90 degrees (relative to the longitudinal axis of bridge 2010). To this end, and looking now at Figs. 52A, 52B and 52C, delivery device 2030 may be constructed so that its staple-straining linkages 2065 are each formed with an outboard constraint 2097, whereby to prevent legs 2015 from being bent past 90 degrees (relative to the longitudinal axis of bridge 2010) when the staple is strained.

In one preferred form of the invention, staple 2005 and delivery device 2030 are provided in the form of a sterilized kit. The kit may include additional instruments to aid in the implantation of the staple (e.g., k-wire, drill bit, staple size guide, tamp, etc.).

In the foregoing discussion, staple 2005 is strained so that, upon deployment in the bone, it will provide compression across a fracture line. However, it should also be appreciated that, if desired, staple 2005 can be configured to provide a distraction force to a bone. In this situation, staple 2005 can be configured and strained so that bridge 2010 can be compressed, and/or legs 2015 can be bent outward, such that when staple 2005 is deployed in bone, the reconfiguring staple can apply a distraction force to the bone, whereby to cause the bone to grow and thereby elongate.

### Novel Staple Comprising Malleable Bridge With Two Elastic Legs

As discussed above, staple 2005 is manufactured from a shape memory material (e.g., a material capable of exhibiting superelasticity and/or a temperature-induced shape change). The shape memory material may be a metal alloy (e.g., Nitinol) or a polymer (e.g, appropriately processed PEEK). In this respect it should be appreciated that staple 2005 can be manufactured out of a single piece of shape memory material (i.e., so as to create an integral, monolithic structure), and the different regions of the staple worked differently, in a metallurgical sense, so that different regions of the staple have different mechanical properties and exhibit different mechanical characteristics, even as they form a single, integral, monolithic structure.

In one form of the invention, and as discussed above, staple 2005 can be manufactured so that bridge 2010 is elastic, legs 2015 are elastic, and curved hinge regions 2020 are elastic, in which case bridge 2010 can be elastically deformed, and legs 2015 can be elastically deformed, so that both bridge 2010 and legs 2015 provide compression to the fracture site after implantation. In this form of the invention, bridge 2010 and legs 2015 may be worked, metallurgically, so that they have the same or different mechanical properties.

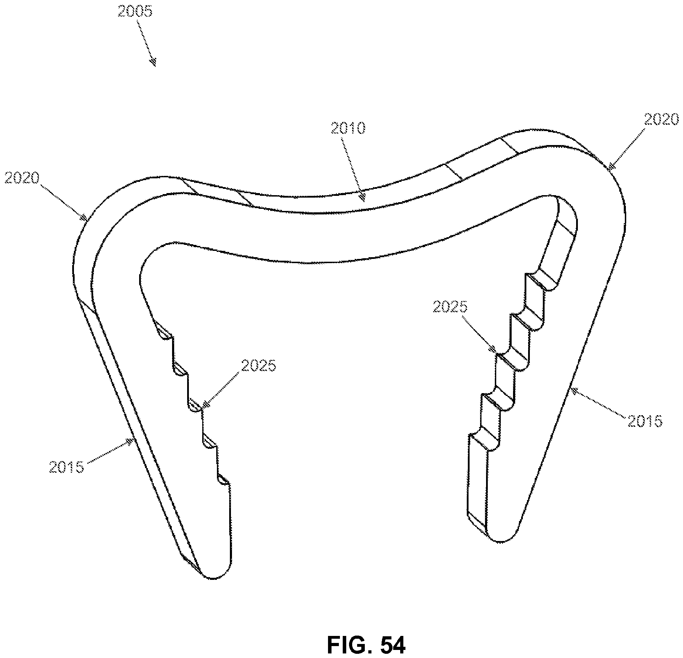

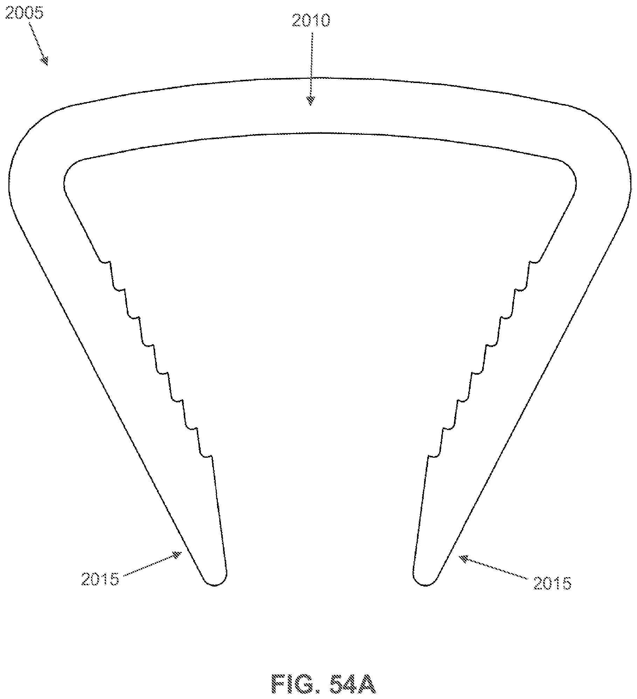

However, in another form of the invention, staple 2005 can be manufactured so that bridge 2010 is malleable and non-superelastic (e.g., fully annealed Nitinol, or martensitic Nitinol with an austenite start temperature greater than body temperature), and legs 2015 and hinge regions 2020 are superelastic (e.g., austenite but capable of forming stress-induced martensite). This allows the malleable bridge 2010 of staple 2005 to be inelastically bent (i.e., to take a set) to accommodate a particular geometry of the cortical anatomy, while still allowing the superelastic legs 2015 of the staple to generate compression. By way of example but not limitation, many bones exhibit an hour-glass surface profile; moreover, certain orthopedic indications (e.g., an Akin Osteotomy) often results in a cortical surface that is concave when the bones are re-approximated. In these situations, a staple with a straight bridge will not sit flush on the bone surface, which can lead to patient discomfort. In this respect it should also be appreciated that where bridge 2010 is malleable and legs 2015 are superelastic, legs 2015 of staple 2005 may be manufactured at a more acute angle (Fig. 54) so as to allow for adequate fracture compression and reduction in the event that bridge 2010 must be bent downward (e.g., deformed to a concave position) to meet the anatomical structure of the cortical bone.

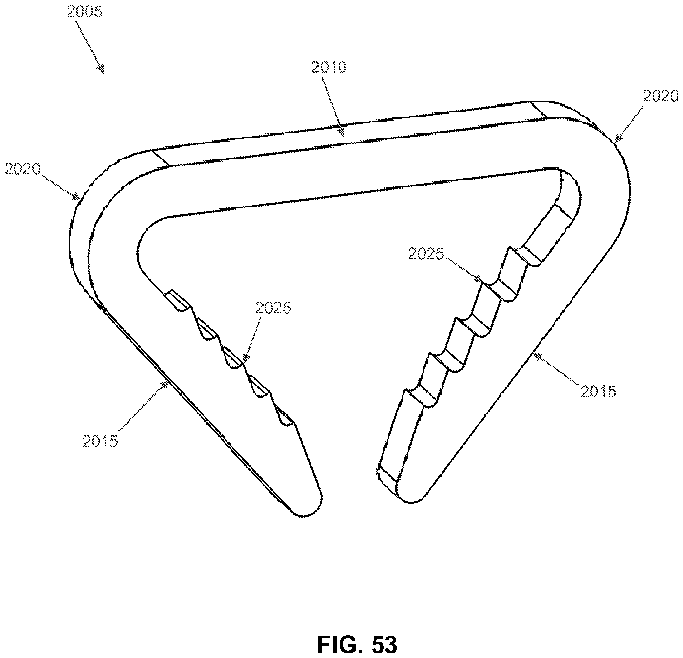

See Fig. 53, which shows a monolithic staple 2005 where bridge 2010 is malleable and legs 2015 are superelastic, and where staple 2005 is shown in its unbent and unstrained condition; and Fig. 54, where bridge 2010 of staple 2005 has been bent to give it an altered configuration. Note that staple 2005 shown in Figs. 53 and 54 is preferably formed out of a single piece of shape memory material, whereby to form a single, integral, monolithic structure, with the single piece of shape memory material having different regions of the staple worked differently, in a metallurgical sense, so that different regions of the staple have different mechanical properties and exhibit different mechanical characteristics, i.e., bridge 2010 is malleable and legs 2015 are superelastic.

It may be desirable for staple 2005 to start with a bridge that is convex, e.g., such as the staple 2005 shown in Fig. 54A. This will allow the bridge of the implanted staple to sit flush with the cortical bone surface if the bone surface is largely planar. More particularly, if the bridge of staple 2005 were to be linear, and the legs strained and the staple inserted into a prepared fracture site where the cortical surface is largely planar, the resulting implanted staple could have two small "humps" at the outer ends of the bridge, i.e., at the bridge-hinge interface. Starting with a convex-shaped bridge (i.e., such as is shown in Fig. 54A) largely eliminates these "humps".

Thus, in a second form of the invention, staple 2005 is formed out of a single piece of shape memory material (i.e., so as to form a single, integral, monolithic structure), with the shape memory material being worked so that bridge 2010 is malleable (e.g., fully annealed Nitinol, or martensitic Nitinol with an austenite start temperature greater than body temperature) and legs 2015 are superelastic (e.g., austenite but capable of forming stress-induced martensite), such that bridge 2010 of staple 2005 may be bent to contour to the surface of the bone while the compressive force generated by the superelastic legs 2015 of the staple are used to help fuse the bone.

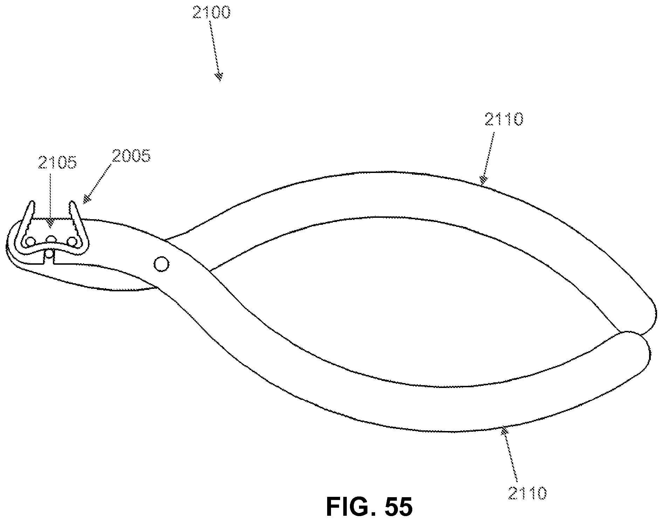

A bending device can be used to bend bridge 2010 of staple 2005 prior to implantation of the staple. An exemplary bending device 2100 is shown in Fig. 55. Bending device 2100 is essentially a modified plier assembly. Staple 2005 is placed into the bending fixture 2105 of bending device 2100; compressing the handles 2110 causes bridge 2010 of staple 2005 to be bent to better meet the shape of the cortical bone surface.

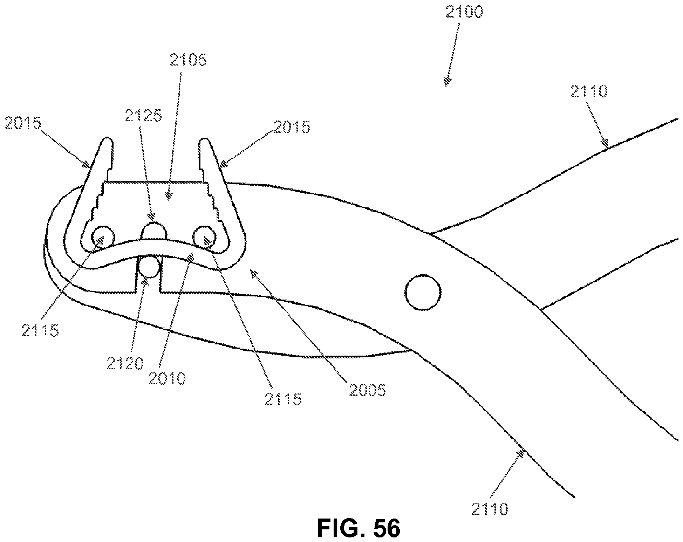

More particularly, Fig. 56 shows a close-up of bending fixture 2105 of bending device 2100. Two pins 2115 are used to locate the staple, and a third pin 2120 is used to bend the bridge of the staple when the handles 2110 of bending device 2100 are compressed. A channel 2125 in bending fixture 2105 both directs the shape of the contour while also serving to limit the maximum bend imposed on the bridge of the staple.

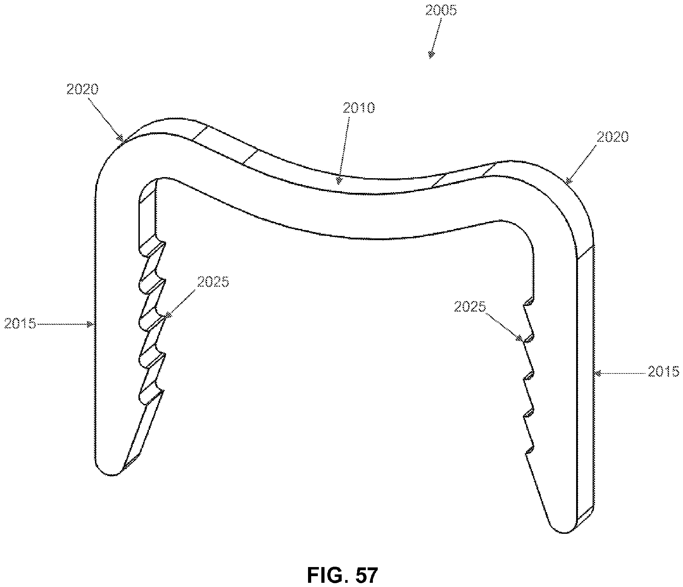

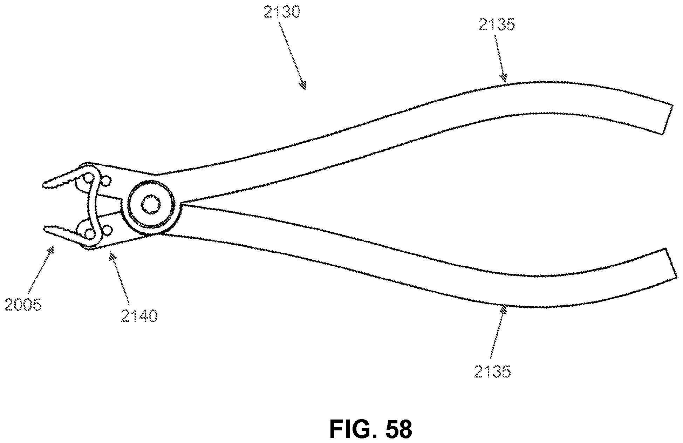

After the bridge of the staple has been bent to the desired geometry (e.g., the geometry shown in Fig. 54), the legs of the staple can be strained open (e.g., to the geometry shown in Fig. 57) so as to allow the bent, strained staple to be inserted into the prepared fracture site. By way of example but not limitation, and looking now at Fig. 58, the bent staple may be strained using a plier assembly 2130 comprising a pair of handles 2135 and a straining fixture 2140. The previously-bent staple is placed into straining fixture 2140, and compressing handles 2135 causes the staple's legs 2015 to be strained opened to parallel.

Plier assembly 2130 is also used to insert the staple into the bone after the legs of the staple have been strained open to substantially parallel.

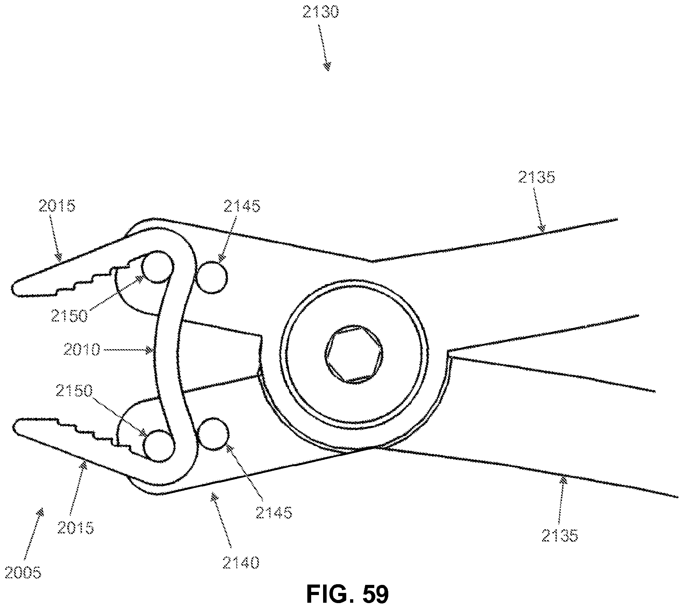

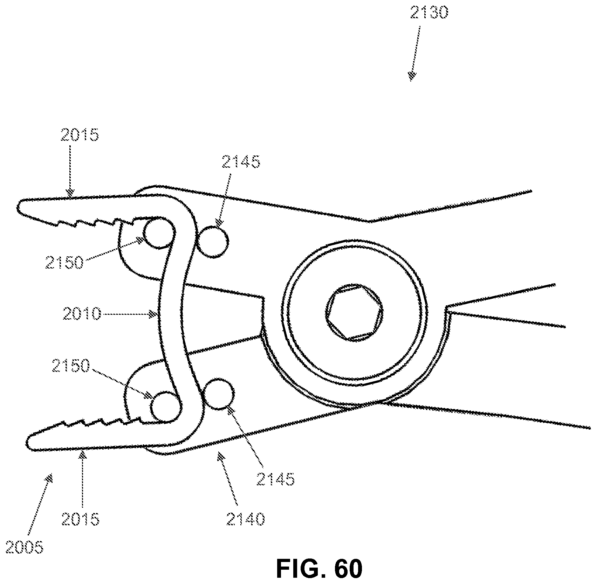

Figs. 59 and 60 show the construction and function of straining fixture 2140 in greater detail. Staple 2005 is supported by two internal pins 2145 and two external pins 2150. Compressing handles 2135 cause the staple legs to move from an inward-pointing configuration (Fig. 59) to a more open (e.g., parallel) state (Fig. 60). The previously-bent staple, with the legs now strained to the open state, is then ready for implantation across the fracture line. When implanted in bone and thereafter released from plier assembly 2130, the strained legs 2015 of staple 2005 then kick inward, reducing the fracture and generating and maintaining compression across the fracture.

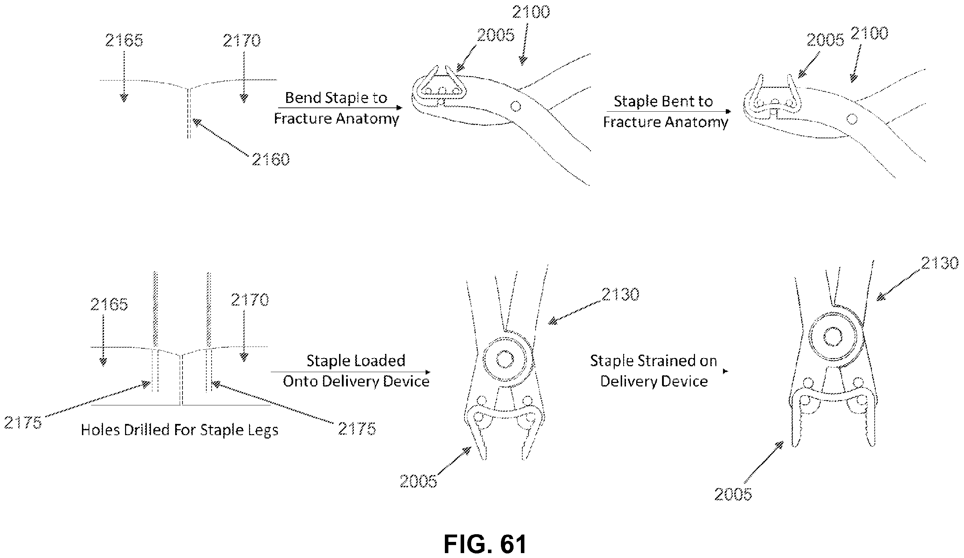

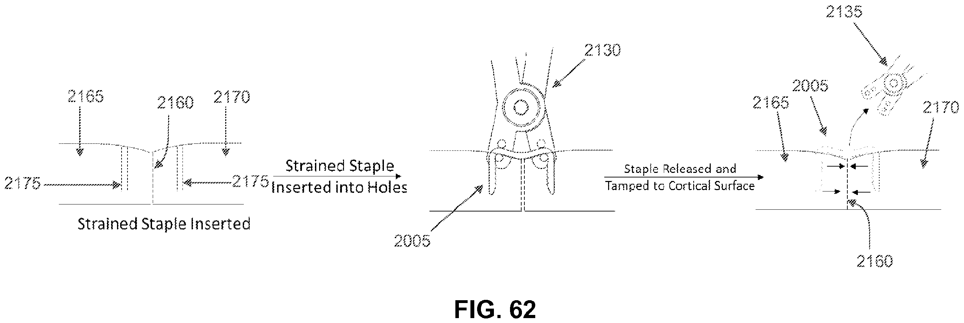

Figs. 61 and 62 show how a staple formed out of a shape memory material, with its bridge being malleable (e.g., fully annealed Nitinol, or martensitic Nitinol with an austenite start temperature greater than body temperature) and its legs being superelastic (e.g., austenite but capable of forming stress-induced martensite), may be used to reduce a fracture 2160 between two bone fragments 2165, 2170 and generate and maintain compression across the fracture. Significantly, because the bridge of the staple is malleable and the legs of the staple are superelastic, the bridge of the staple can be first bent to match the surface profile of the bone while enabling the superelastic legs of the staple to be elastically strained to provide the compressive force across the fracture.

Looking now at Figs. 61 and 62, staple 2005 is first loaded onto bending device 2100 and the bridge of the staple is bent to accommodate the surface profile of the patient's cortical bone anatomy. The surgeon may use fluoroscopy or trial-and-error to bend the bridge of the staple to the appropriate configuration. With the bridge of the staple appropriately bent, a drill guide (not shown) is used to drill holes 2175 into the bone fragments 2165, 2170 at the appropriate locations on either side of the fracture line 2160 to accommodate the strained staple legs. Staple 2005 is then loaded onto plier assembly 2130, and superelastic legs 2015 are then elastically bent to the open state.

With the bridge of the staple inelastically bent into the appropriate configuration and with the legs of the staple elastically strained to substantially parallel, the staple can be inserted into the pre-drilled holes 2175 in bone fragments 2165, 2170. The staple is then released from plier assembly 2130 and tamped to sit flush with the cortical surface, with the inelastically bent bridge 2010 of the staple more closely matching the surface contour of the bone. The elastically-strained superelastic legs 2015 of the staple applies a compressive force across the fracture.

If desired, where the staple is provided with a malleable bridge, the malleable bridge may be bent, or further bent, after the staple has been deployed in bone, e.g., to match, or to more closely match, the surface profile of the bone.

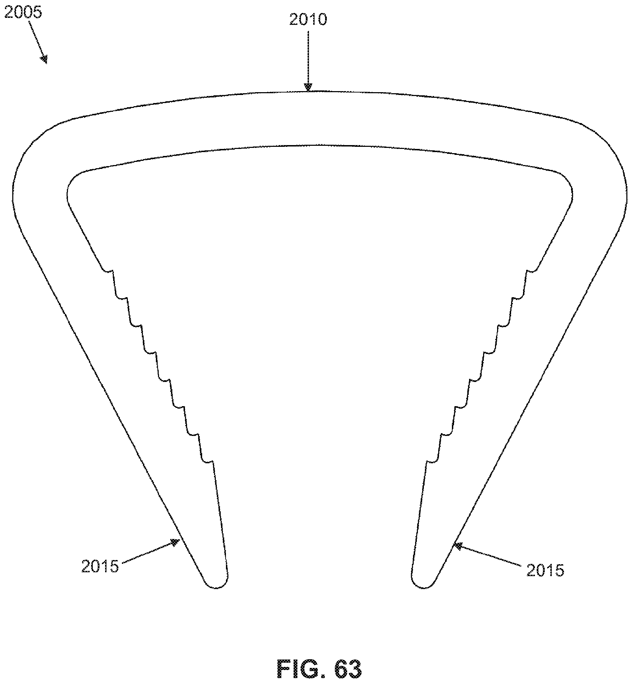

In some circumstances the bone may have a convex profile. In this circumstance, it may be desirable to set the staple so that its bridge has a convex configuration. To this end, and looking now at Fig. 63, there is shown a staple 2005 which has been inelastically bent to have a convex bridge 2010 and two legs 2015.

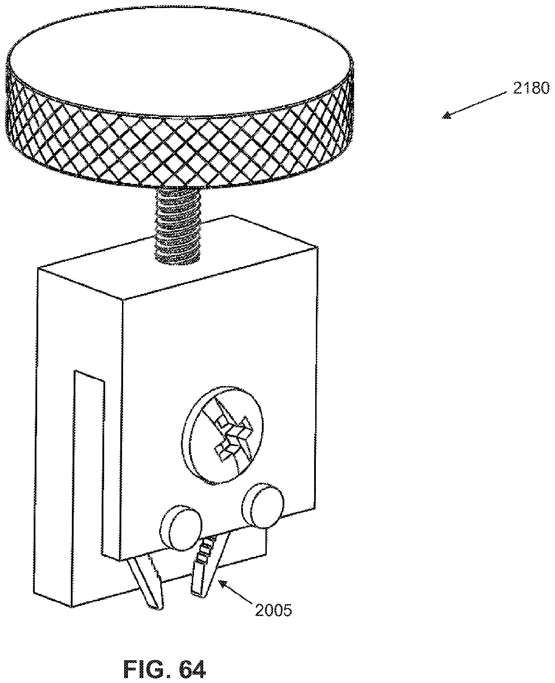

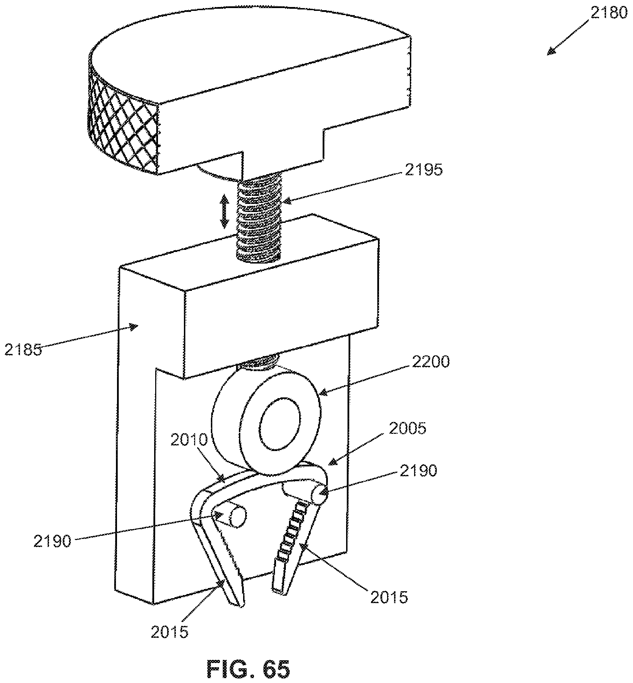

Figs. 64 and 65 show another bending device 2180 which may be used to bend the bridge of a staple, e.g., the bridge 2010 of the staple 2005 shown in Fig. 54A. Bending device 2180 generally comprises a housing 2185 supporting a pair of pins 2190. Pins 2190 receive staple 2005 in the manner shown in Fig. 65. Bending device 2180 also comprises a screw mechanism 2195 which selectively advances an element 2200 toward pins 2190 or retracts element 2200 away from pins 2190. As a result of this construction, when staple 2005 is mounted on pins 2190, screw mechanism 2195 can be used to drive element 2200 against bridge 2010 of staple 2005, whereby to bend the bridge of the staple.

It should also be appreciated that, if desired, staple 2005 can be used to attach soft tissue to bone (e.g., to attach a rotator cuff to bone).

It should be appreciated that delivery device 2130 discussed above may not always seat the staple with the bridge of the staple seated directly against the cortical surface of the bone (i.e., the bridge of the staple may sit slightly above the cortical surface of the bone). Therefore, a tamp of the sort well known in the art may be used to fully seat the staple bridge against the cortical surface of the bone.

In one preferred form of the invention, staple 2005, bending device 2100 and/or bending device 2180, and delivery device (i.e., plier assembly) 2130 are provided in the form of a sterilized kit. The kit may include additional instruments to aid in the implantation of the staple (e.g., k-wire, drill bit, staple size guide, tamp, etc.).

### Test Data

Conventional shape memory staples typically generate between about 20N and about 120N of compressive force from the staple legs kicking inward.

The novel staple of the present invention having a stretched bridge as described herein generates a compressive load of greater than the 20N to 120N generated by other like-sized conventional staples, thereby providing significantly increased compressive forces without tearing through or otherwise damaging the bone. Additionally, the compressive force provided by the stretched bridge staple of the present invention is more uniformly distributed across the fracture line (i.e., across the cortical bone and the cancellous bone).

### Further Aspects

In one preferred embodiment of the present invention, the force generated by the staple is controlled by stretching (i.e., straining) the bridge of the staple approximately 8%, and then allowing the bridge of the staple to partially recover (e.g., to shorten) so that the bridge of the staple is approximately 6% longer than its original length. With the staple stretched 6% longer than its original length, a constraint (e.g., delivery device 2030) can be removably used to prohibit further recovery of the stretched staple bridge. The stretched staple can then be implanted. The partial recovery of strain (i.e., from an 8% stretch to a 6% stretch) occurs in the Load Transition zone at stress levels that may be in excess of the shear strength of bone. In other words, by allowing the stretched staple bridge to partially recover from strain prior to implantation (i.e, from an 8% stretch to a 6% stretch), the highest stress levels associated with the upper plateau at 8% stretch may be prevented from being applied to the bone, and only the lower plateau stress levels associated with the remaining 6% stretch may be applied to the bone (i.e., partial recovery of strain prior to implantation occurs in the load transition zone). Following implantation of the staple, the constraint (e.g., the aforementioned delivery device 2030) can be removed from the staple, thereby allowing the staple to attempt to recover the remaining strain. The subsequent strain recovery will occur directly at the stress level of the lower plateau of the mechanical hysteresis curve of the Nitinol staple. The stress level of the lower plateau may be less than the shear strength of the bone, thereby keeping the staple legs from stripping in bone.

In another preferred embodiment of the present invention, shape memory materials may be used to manufacture staples that generate and maintain compression between bone fragments while also having a malleable bridge that may be bent to conform to the unique anatomical structure of each patient. Like traditional shape memory staples, this novel staple can have legs that are implanted in a parallel state and are allowed to "kick in" after implantation. However, during manufacturing, the staple bridge may be selectively heat treated at 400°C for 70 minutes. This will selectively age the shape memory material so that the austenite finish temperature of only the bridge region of the staple is 40°C (i.e., above body temperature). Thus the bridge will be martensitic and malleable. The surgeon can then bend the bridge of the staple so that it matches the contour of the bone.

### Example 3

### Intramedullary Devices For Generating And Applying Compression Within A Body

The present invention comprises the provision and use of novel intramedullary devices that are better able to bring bone fragments into close proximity with each other, generate a compressive load, and maintain that compressive load for a prolonged period of time while healing occurs.

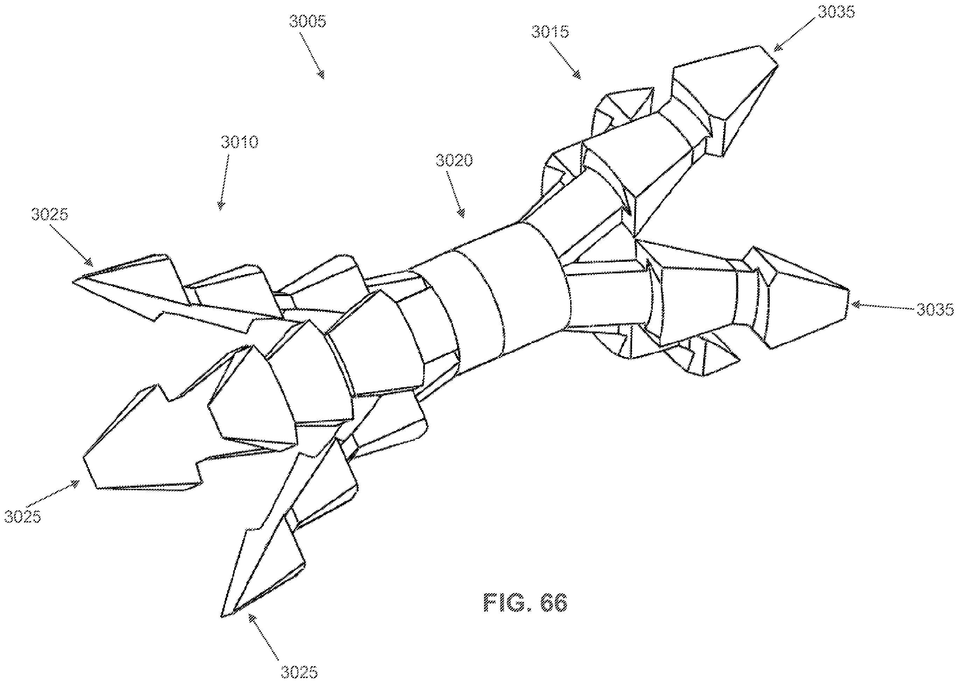

Looking next at Figs. 66-69, there is shown an intermedullary fusion device 3005 manufactured from a shape memory material (e.g., a material capable of exhibiting superelasticity and/or a temperature-induced shape change). The shape memory material may be a metal alloy (e.g., Nitinol) or a polymer (e.g., appropriately processed PEEK). Intramedullary fusion device 3005 comprises a first barbed end region 3010, a second barbed end region 3015, and a central bridge region 3020 connecting first barbed end region 3010 to second barbed end region 3015. Intramedullary fusion device 3005 is preferably cannulated so as to allow the intramedullary fusion device to be installed over a k-wire if desired, while also allowing a k-wire to be passed through the intramedullary fusion device following implantation if the surgeon desires to fuse a distal or proximal joint. The first and second barbed regions flare outward in multiple planes, preferably engaging the surrounding bone about the full circumference of the intramedullary fusion device, thereby providing excellent torsional stability to the fusion site.

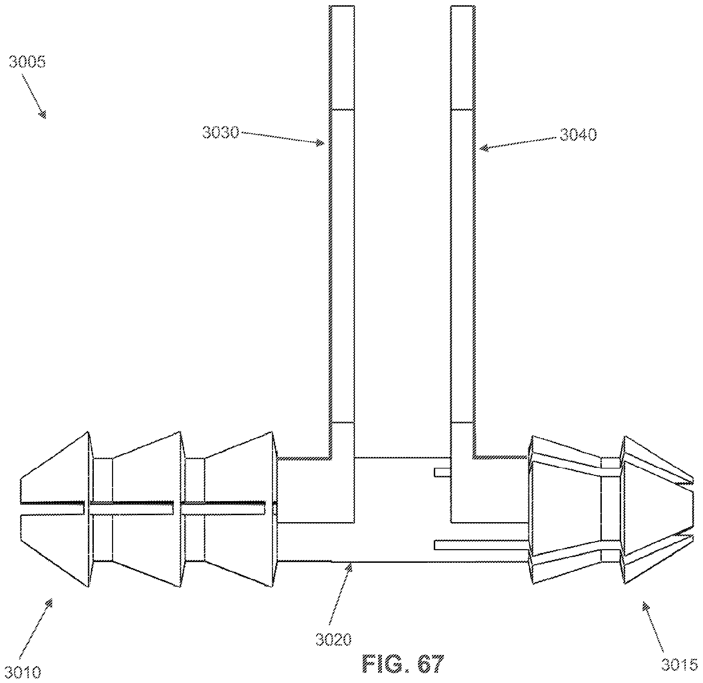

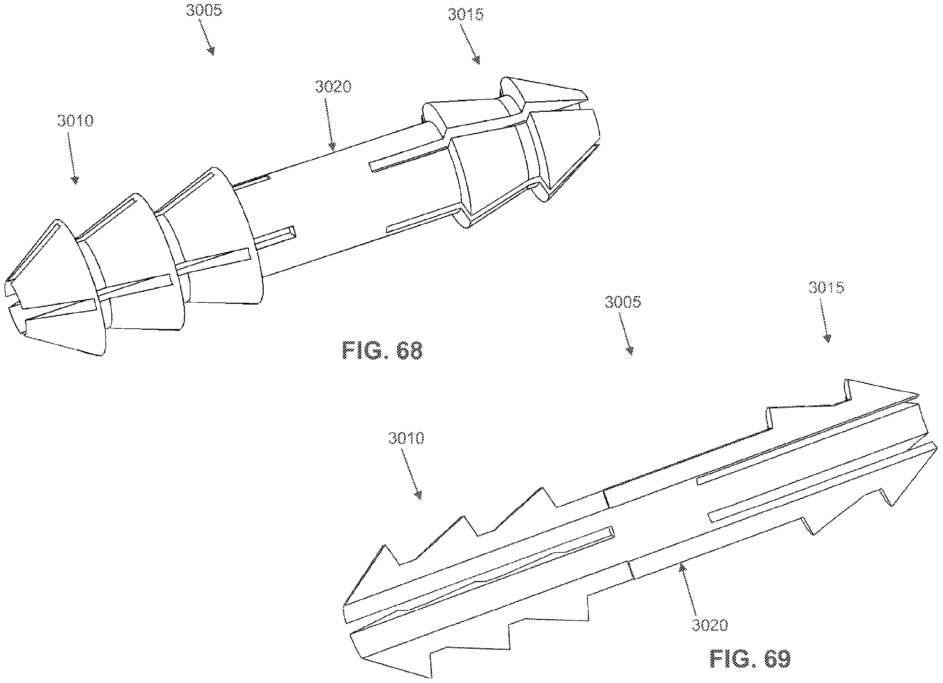

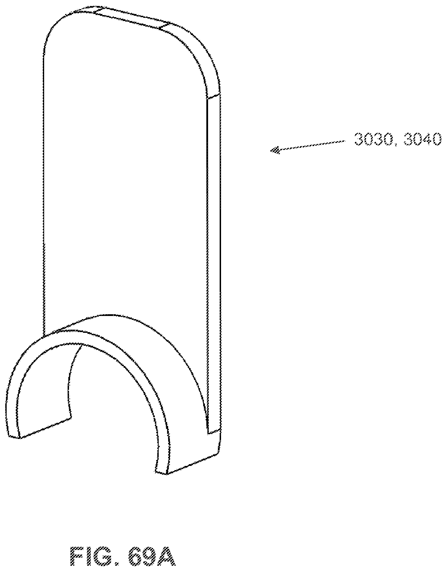

First barbed end region 3010 comprises a plurality of barbs 3025 which, in their unbiased condition, flare outward from the longitudinal axis of intramedullary fusion device 3005 in the manner shown in Fig. 66. The flare may increase linearly over the length of the barb, or it may increase non-linearly over the length of the barb to enable first barbed end region 3010 to better engage the "hourglass-shaped" intramedullary canal. The better that first barbed end region 3010 engages the intramedullary canal, the more even the pressure distribution will be. However, barbs 3025 can be strained to a position parallel to the longitudinal axis of intramedullary fusion device 3005 and constrained in that position (e.g., via a removable retaining tab 3030, Figs. 67 and 69A) so as to reduce the cross-sectional profile of first barbed end region 3010, whereby to allow for insertion into a drilled hole in bone, as will hereinafter be discussed. Barbs 3025 can be constrained in a state where they partially occupy the cannulation of intramedullary fusion device 3005 (i.e., where barbs 3025 are strained to a point past parallel to the longitudinal axis of intramedullary fusion device 3005), thereby further reducing the cross-sectional area of first barbed end region 3010. This is beneficial for accessing the intramedullary canal through a small drilled hole.

While Fig. 66 illustrates a device with four barbs 3025 on first barbed end region 3010, it should be appreciated that first barbed end region 3010 can be made with more or fewer barbs. Upon removing retaining tab 3030, barbs 3025 are allowed to flare outward again, whereby to grip the side wall of the drilled hole and intramedullary canal receiving first barbed end region 3010, whereby to lock first barbed end region 3010 to a bone fragment via an expansive force on the intramedullary canal, as will also hereinafter be discussed.

In one preferred form of the invention, barbs 3025 of first barbed end region 3010 are separated from one another by relatively small longitudinal gaps when barbs 3025 are strained to a position parallel to the longitudinal axis of intramedullary fusion device 3005, such that barbs 3025 collectively provide a substantially full circumferential structure for first barbed end region 3010 (i.e., when barbs 3025 are strained to a position parallel to the longitudinal axis of intramedullary fusion device 3005, barbs 3025 collectively provide a substantially continuous extension of central bridge region 3020 of intramedullary fusion device 3005). See Figs. 68 and 69.

Second barbed end region 3015 comprises a plurality of barbs 3035 which, in their unbiased condition, flare outward from the longitudinal axis of intramedullary fusion device 3005 in the manner shown in Fig. 66. The flare may increase linearly over the length of the barb, or it may increase non-linearly over the length of the barb to enable second barbed end region 3015 to better engage the "hourglass-shaped" intramedullary canal. The better that second barbed end region 3015 engages the intramedullary canal, the more even the pressure distribution will be. However, barbs 3035 can be strained to a position parallel to the longitudinal axis of intramedullary fusion device 3005 and constrained in that position (e.g., via a removable retaining tab 3040, Figs. 67 and 69A) so as to reduce the cross-sectional profile of second barbed end region 3015, whereby to allow for insertion into a drilled hole in bone, as will hereinafter be discussed. Barbs 3035 can be constrained in a state where they partially occupy the cannulation of intramedullary fusion device 3005 (i.e., where barbs 3035 are constrained to a point past parallel to the longitudinal axis of intramedullary fusion device 3005), thereby further reducing the cross-sectional area of second barbed region 3015. This is beneficial for accessing the intramedullary canal through a small drilled hole.

While Fig. 66 illustrates a device with four barbs 3035 on the second barbed end region 3015, it should be appreciated that second barbed end region 3015 can be made with more or fewer barbs. Upon removing retaining tab 3040, barbs 3035 are allowed to flare outward, whereby to grip the side wall of the drilled hole and intramedullary canal receiving second barbed end region 3015, whereby to lock second barbed end region 3015 to a bone fragment via an expansive force on the intramedullary canal, as will also hereinafter be discussed.

In one preferred form of the invention, barbs 3035 of second barbed end region 3015 are separated from one another by relatively small longitudinal gaps when barbs 3035 are strained to a position parallel to the longitudinal axis of intramedullary fusion device 3005, such that barbs 3039 collectively provide a substantially full circumferential structure for second barbed end region 3015 (i.e., when barbs 3035 are strained to a position parallel to the longitudinal axis of intramedullary fusion device 3005, barbs 3035 collectively provide a substantially continuous extension of central bridge region 3020 of intramedullary fusion device 3005). See Figs. 68 and 69.

It should be appreciated that first barbed end region 3010 and second barbed end region 3015 may have different numbers of barbs, e.g., first barbed end region 3010 may comprise four barbs 3025 and second barbed end region 3015 may comprise three barbs 3035. However, it should be appreciated that regardless of the number of barbs 3025 provided on first barbed end region 3010, and regardless of the number of barbs 3035 provided on second barbed end region 3015, the barbs 3025 of first barbed end region 3010 preferably engage the surrounding bone about the full circumference of the intramedullary fusion device, and the barbs 3035 of second barbed end region 3015 preferably engage the surrounding bone about the full circumference of the intramedullary fusion device.

Central bridge region 3020 preferably comprises a generally cylindrical shape and is preferably sized so as to have an outer diameter somewhat less than the major diameters of first barbed end region 3010 and second barbed end region 3015 when their barbs 3025, 3035, respectively, have been strained to a position parallel to the longitudinal axis of intramedullary fusion device 3005.

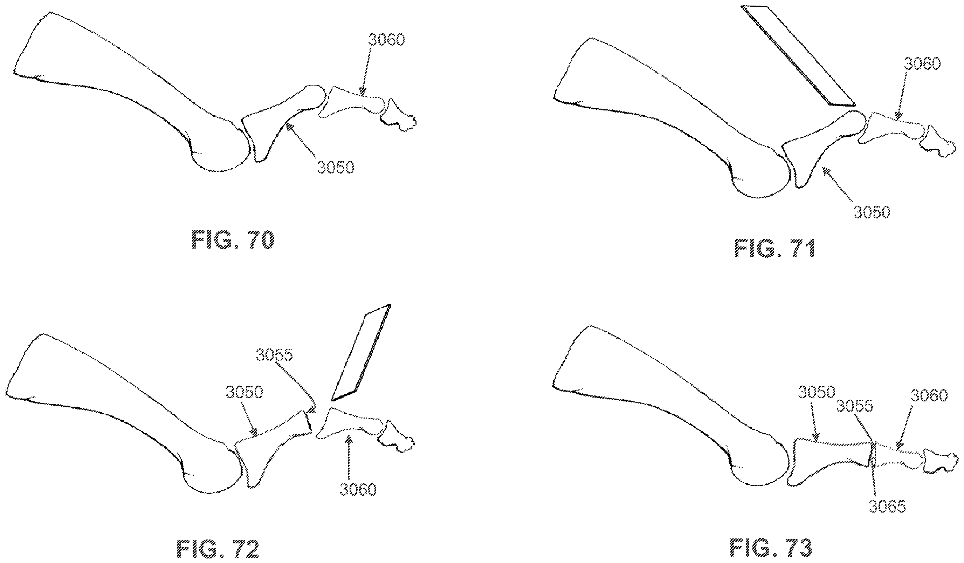

Intramedullary fusion device 3005 may be used to secure together two bone fragments under compression. By way of example but not limitation, and looking now at Figs. 70-80, intramedullary fusion device 3005 may be used to treat a hammer toe deformity (Fig. 70).

First the distal end of the metatarsal 3050 is cut off to correct the deformity and create a bone face 3055 suitable for fusion (Figs. 71 and 72). Then the proximal end of the phalange 3060 is removed to correct the deformity and create a bone face 3065 suitable for fusion (Figs. 72 and 73). With these two cuts complete, the bones of the metatarsal-phalange joint can be properly aligned (Fig. 73) for subsequent fusion, as will hereinafter be discussed.

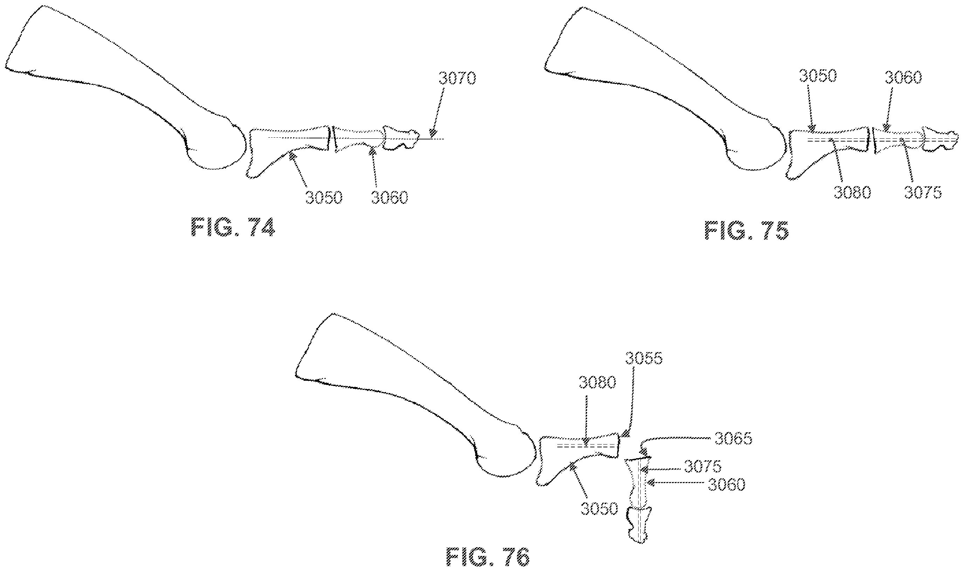

Next, the surgeon inserts a k-wire 3070 through the distal end of the toe, phalange 3060 and into the metatarsal 3050 (Fig. 74). Preferably k-wire 3070 passes down the intramedullary canals of phalange 3060 and metatarsal 3050. Removal of k-wire 3070 leaves a canal 3075 (i.e., the opened intramedullary canal) in phalange 3060 and a canal 3080 in metatarsal 3050, with canal 3075 in phalange 3060 being aligned with canal 3080 in metatarsal 3050 when phalange 3060 is aligned with metatarsal 3050 (Fig. 75). Canals 3075 and 3080 receive intramedullary fusion device 3005 as will hereinafter be discussed.

Following removal of k-wire 3070, phalange 3060 is flexed downward so as to expose the prepared metatarsal face 3055 (Fig. 76).

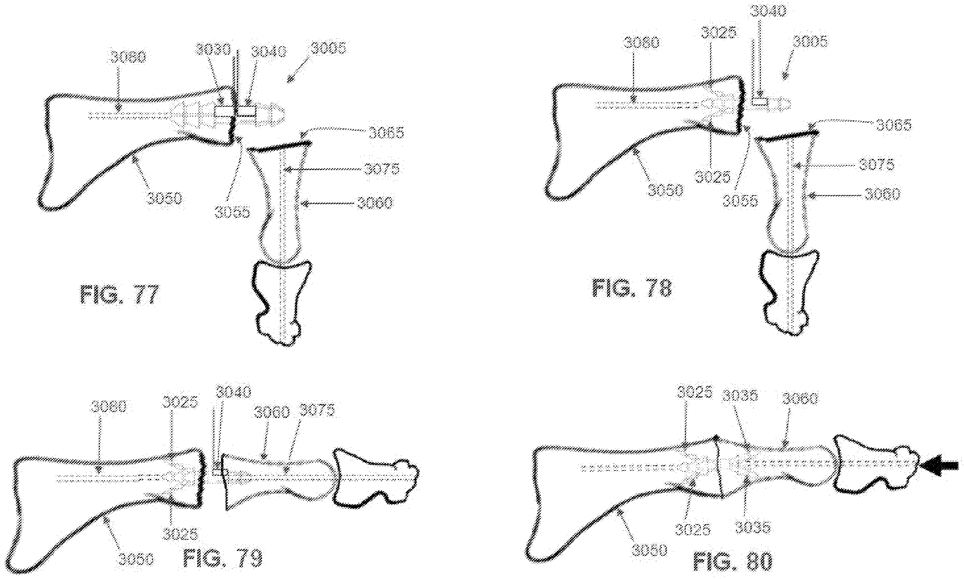

Next, with retaining tabs 3030, 3040 constraining barbs 3025, 3035, respectively, of first barbed end region 3010 and second barbed end region 3015, respectively, to their "inboard" position (i.e., as shown in Fig. 67), intramedullary fusion device 3005 has its first barbed end region 3010 advanced into canal 3080 of metatarsal 3050 (Fig. 77). Then removable retaining tab 3030 is removed, allowing barbs 3025 of first barbed end region 3010 to expand outwardly and grip the side wall of canal 3080, whereby to securely fasten intramedullary fusion device 3005 to metatarsal 3050 through an expansive force against the intramedullary surface (Fig. 78). Phalange 3060 is then pressed over second barbed region 3015 of intramedullary fusion device 3005, with second barbed region 3015 being received in canal 3075 of phalange 3060 (Fig. 79). This action brings face 3065 of phalange 3060 against face 3055 of metatarsal 3050, with just enough room being left for retaining tab 3040 to extend from intramedullary device 3005 to a region outside of the bone.

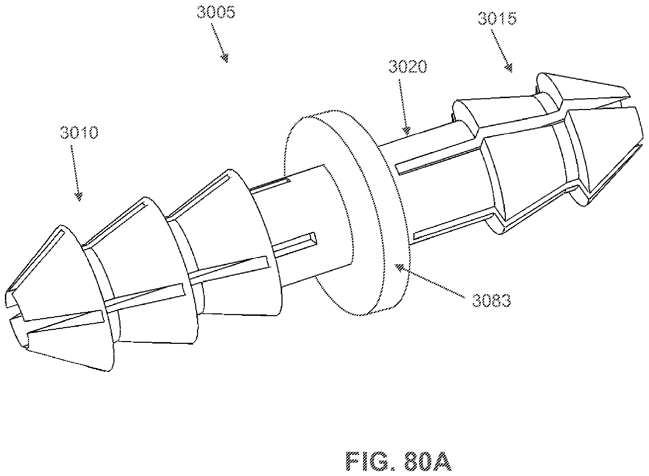

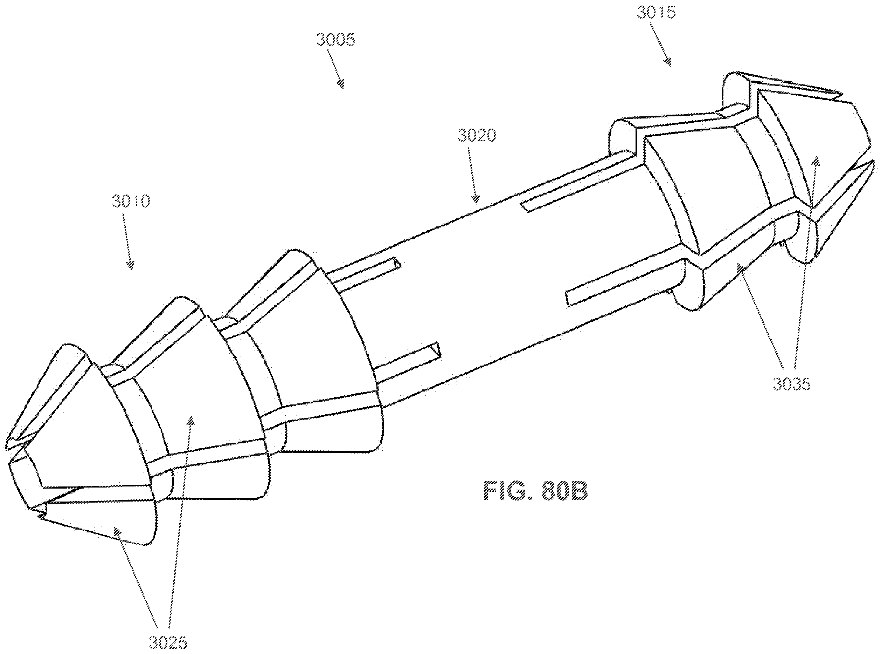

Next, retaining tab 3040 is removed so that barbs 3035 of second barbed end region 3015 are allowed to expand outwardly and grip the side wall of canal 3075 of phalange 3060 (Fig. 80). At this point barbs 3025 of first barbed end region 3010 are securely engaging metatarsal 3050, and barbs 3035 of second barbed end region 3015 are securely engaging phalange 3060, with central bridge region 3020 extending across the fracture line. A force can be applied to reduce any gap left after removing retaining tab 3040.

It should be appreciated that novel intramedullary fusion device 3005 can first be implanted into phalange 3060 and then implanted into metatarsal 3050 if the surgeon so chooses.

If desired, and looking now at Fig. 80A, central bridge region 3020 may have an enlargement 3083 intermediate its length to act as a stop, limiting how far intramedullary fusion device 3005 can be pushed into either side of the intramedullary canal during implantation.

In Figs. 66-69, barbs 3025 of first barbed end region 3010 are shown as being circumferentially offset from barbs 3035 of second barbed end region 3015, i.e., barbs 3025 and barbs 3035 are not axially aligned with one another. However, if desired, and looking now at Fig. 80B, barbs 3025 of first barbed end region 3010 may not be circumferentially offset from barbs 3035 of second barbed end region 3015, i.e., barbs 3025 and barbs 3035 may be axially aligned with one another in the manner shown in Fig. 80B.

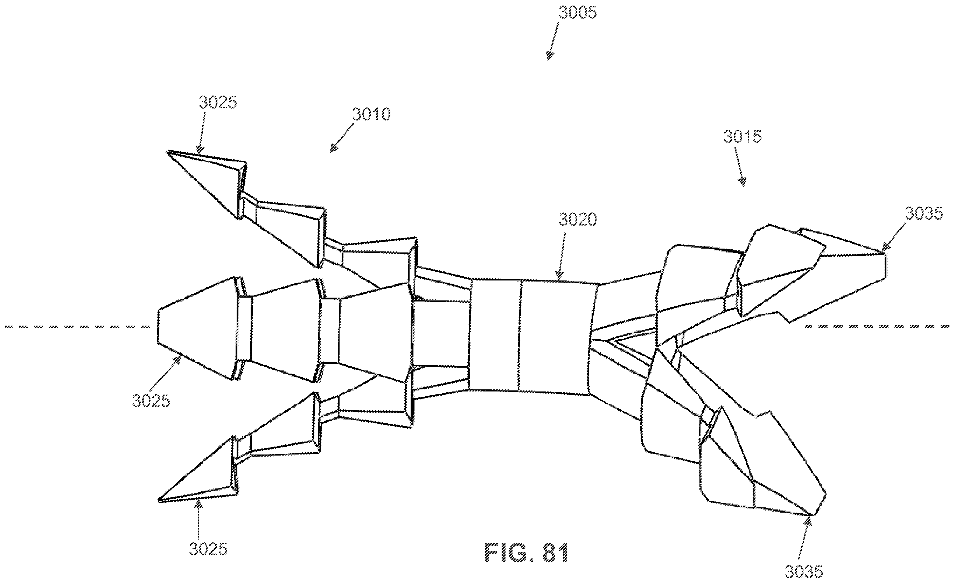

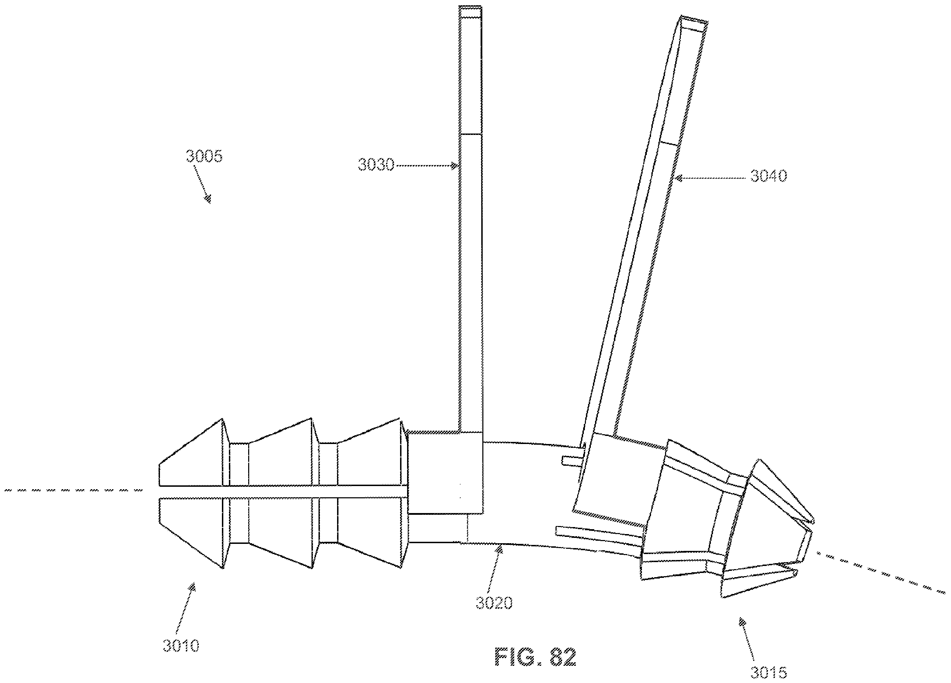

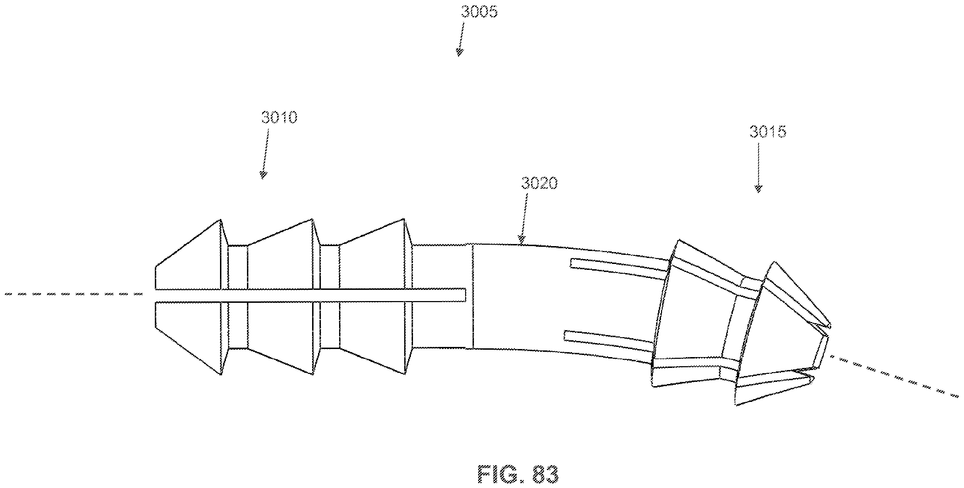

If desired, and looking now at Figs. 81-83, intramedullary fusion device 3005 can have a slight bend from central bridge region 3020 to one or both of its first barbed end region 3010 and second barbed end region 3015. By way of example but not limitation, in the metatarsal-phalange fusion shown in Figs. 70-80, it may be desirable to provide a slight bend to second barbed end region 3015 so as to facilitate the restoration of the normal anatomy. In this form of the invention, intramedullary fusion device 3005 may be bent after machining and during the working of the shape memory material, e.g., it may be shape-set at the desired angulation through heat treatment.

It should also be appreciated that the central bridge region 3020 can be processed so as to be malleable (i.e., to take a set). At body temperature, the barb regions 3010 and 3015 can be superelastic while central bridge region 3020 can be fully annealed Nitinol or martensitic Nitinol, such that central bridge region 3020 is malleable and can take a set. This allows the surgeon to deform central bridge region 3020 at the time of surgery so that it assumes the bend desired.

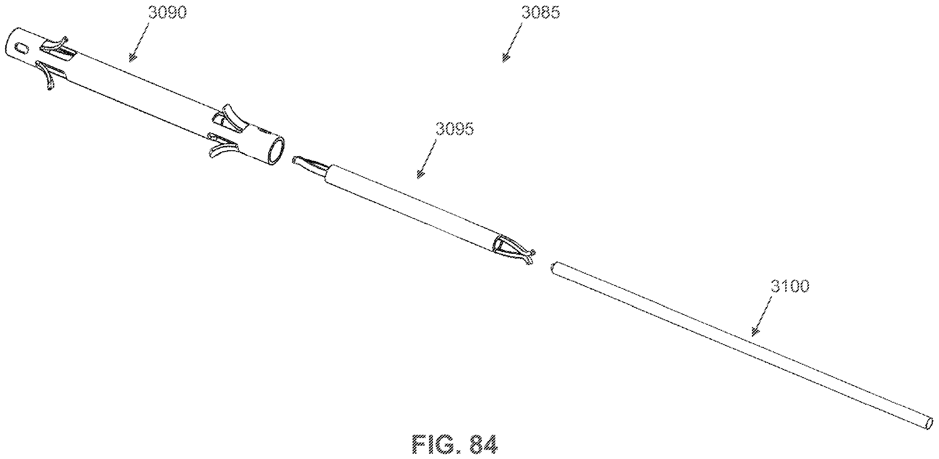

Looking next at Fig. 84, there is shown an intramedullary fusion system 3085 which generally comprises an intramedullary fusion device 3090, an internal restrainer 3095 and a locking pin 3100.

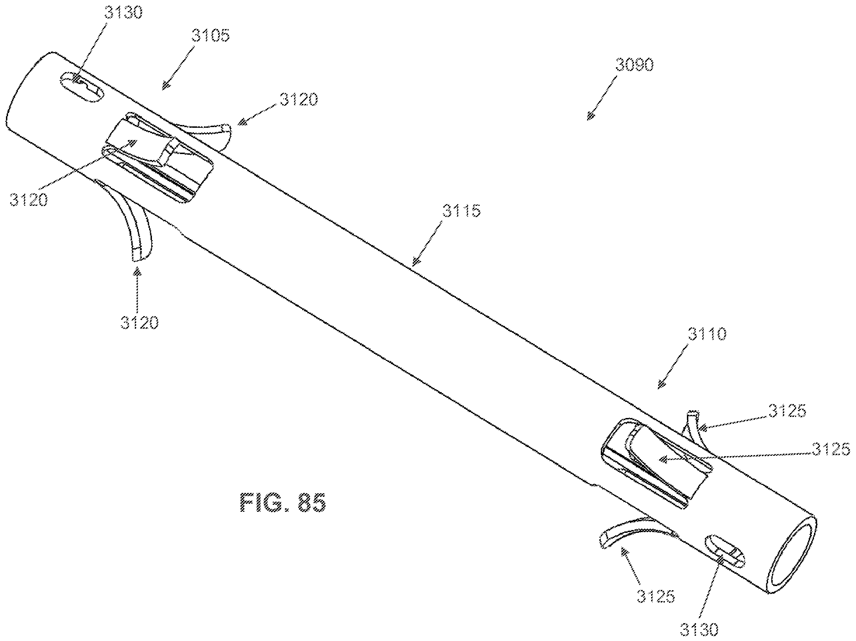

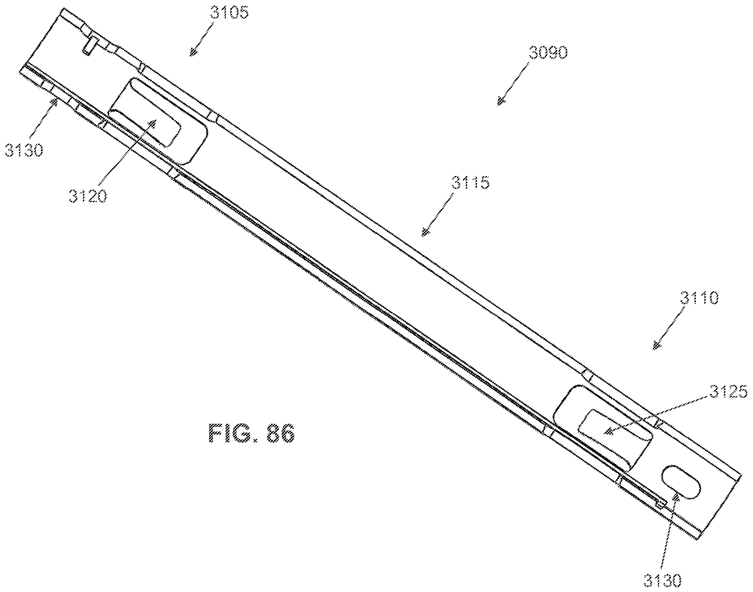

Intramedullary fusion device 3090 is manufactured from a shape memory material (e.g., a material capable of exhibiting superelasticity and/or a temperature-induced shape change). The shape memory material may be a metal alloy (e.g., Nitinol) or a polymer (e.g., appropriately processed PEEK). Looking now at Figs. 85 and 86, intramedullary fusion device 3090 comprises a first barbed end region 3105, a second barbed end region 3110, and a central bridge region 3115 connecting first barbed end region 3105 and second barbed end region 3110. Intramedullary fusion device 3090 is preferably cannulated so as to allow the intramedullary fusion device to be installed over a k-wire if desired, while also allowing a k-wire to be passed through the device following implantation if the surgeon desires to fuse a distal or proximal joint. The first and second barbed regions flare outward in multiple planes, preferably engaging the surrounding bone about the full circumference of the intramedullary fusion device, thereby providing excellent torsional stability to the fusion site.

First barbed end region 3105 comprises a plurality of barbs 3120 which, in their unbiased condition, flare outward from the longitudinal axis of intramedullary fusion device 3090 in the manner shown in Fig. 85. The flare may increase linearly over the length of the barb, or it may increase non-linearly over the length of the barb to enable first barbed end region 3105 to better engage the "hourglass-shaped" intramedullary canal. The better that first barbed region 3105 engages the intramedullary canal, the more even the pressure distribution will be. While Fig. 85 illustrates a device with four barbs 3120 in first barbed end region 3105, it should be appreciated that the device can have more or fewer barbs. Barbs 3120 can be strained to a position parallel to the longitudinal axis of intramedullary fusion device 3090, e.g., during insertion into a hole drilled in bone. Once inserted into the intramedullary canal, barbs 3120 flare outwardly so as to engage with the side wall of the drilled hole receiving first barbed end region 3105. By angling barbs 3120 relative to the longitudinal axis of intramedullary fusion device 3090, i.e., with an arrowhead configuration, barbs 3120 can ensure that first barbed end region 3105 is insertable into a hole in a bone but not withdrawable from the hole in the bone. In this way barbs 3120 can selectively lock first barbed end region 3105 to a bone fragment, as will hereinafter be discussed.

Second barbed end region 3110 comprises a plurality of barbs 3125, which, in their unbiased condition, flare outward from the longitudinal axis of intramedullary fusion device 3090 in the manner shown in Fig. 85. The flare may increase linearly over the length of the barb, or it may increase non-linearly over the length of the barb to enable second barbed end region 3110 to better engage the "hourglass-shaped" intramedullary canal. The better that second barbed region 3110 engages the intramedullary canal, the more even the pressure distribution will be. While Fig. 85 illustrates a device with four barbs 3125 in second barbed end region 3125, it should be appreciated that the device can have more or fewer barbs. Barbs 3125 can be strained to a position parallel to the longitudinal axis of intramedullary fusion device 3090, e.g., during insertion into a hole drilled in bone, with barbs 3125 flaring outwardly so as to remain in constant engagement with the side wall of the drilled hole receiving second barbed end region 3110. By angling barbs 3125 relative to the longitudinal axis of intramedullary fusion device 3090, i.e., with an arrowhead configuration, barbs 3125 can ensure that second barbed end region 3110 is insertable into a hole in a bone but not withdrawable from the hole in the bone. In this way barbs 3125 can selectively lock second barbed end region 3110 in the intramedullary canal of a bone fragment, as will hereinafter be discussed.

Note that barbs 3120 of first barbed end region 3105 are flared in a direction which is opposite to that of barbs 3125 of second barbed end region 3110.

Central bridge region 3115 preferably comprises a generally cylindrical shape and is configured so that it can be selectively strained (i.e., stretched) longitudinally and constrained in that position (e.g., via the aforementioned internal restrainer 3095 and locking pin 3100). As will hereinafter be discussed, upon removing locking pin 3100, internal restrainer 3095 will release the constraint on central bridge region 3115, whereupon central bridge region 3115 will attempt to foreshorten. As will also hereinafter be discussed, this foreshortening can be harnessed to apply compression between two bone fragments.

In order to allow central bridge region 3115 to be constrained in its longitudinally stretched state, intramedullary fusion device 3090 preferably comprises cutouts 3130, disposed in first barbed end region 3105 and second barbed end region 3110, that allow central bridge region 3115 to be constrained in its longitudinally stretched state by internal restrainer 3095, as will hereinafter be discussed.

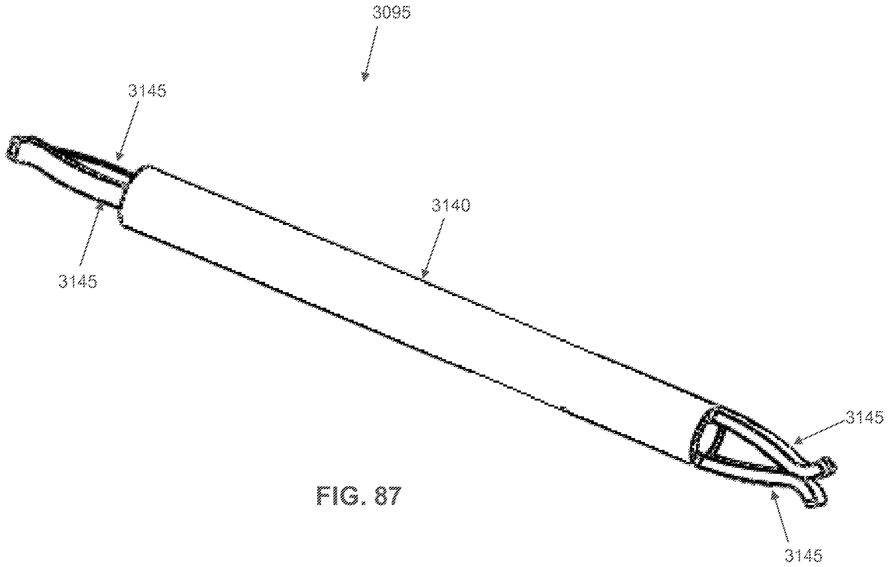

Internal restrainer 3095 is shown in further detail in Fig. 87. Internal restrainer 3095 may comprise a shape memory material if desired. Internal restrainer 3095 generally comprises a cannulated cylindrical body 3140 terminating in a pair of fingers 3145 at each end of cylindrical body 3140. Each pair of fingers 3145 are normally biased together, however, they may be elastically forced apart so that they extend outboard beyond the circumference of cylindrical body 3140, whereby to allow fingers 3145 of internal restrainer 3090 to lock to cutouts 3130 of intramedullary fusion device 3090 when intramedullary fusion device 3090 is in its longitudinally stretched state, as will hereinafter be discussed.

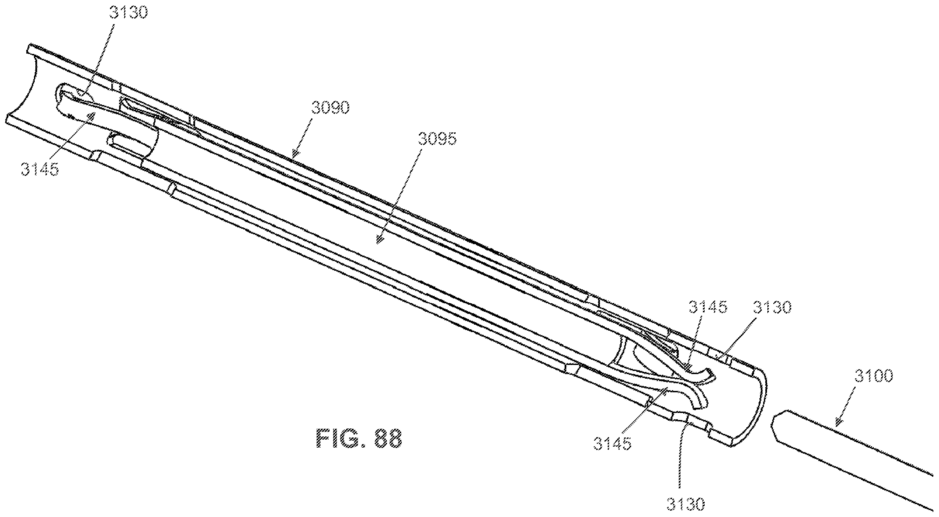

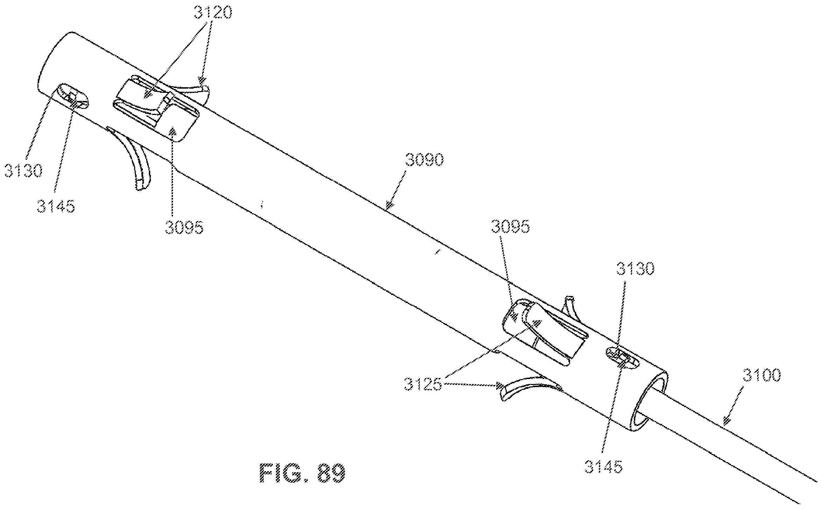

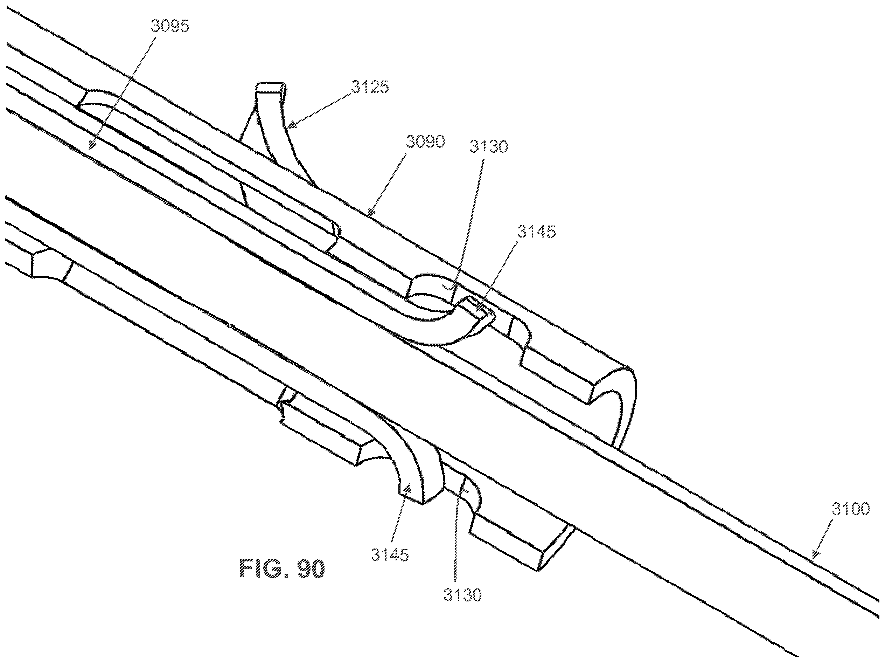

As noted above, central bridge region 3115 of intramedullary fusion device 3090 can be strained (i.e., longitudinally stretched), locked in that position via internal restrainer 3095 and locking pin 3100 and, upon removing locking pin 3100, internal restrainer 3095 will release the constraint on central bridge region 3115 of intramedullary fusion device 3090, whereupon central bridge region 3115 will attempt to foreshorten. More particularly, and looking now at Figs. 88-90, central bridge region 3115 is stretched using a stretching mechanism (not shown) of the sort which will be apparent to those skilled in the art in view of the present disclosure, and internal restrainer 3095 is inserted into intramedullary fusion device 3090. As internal restrainer 3095 is inserted into intramedullary fusion device 3090, the pairs of fingers 3145 disposed at each end of internal restrainer 3095 are aligned with cutouts 3130 in first barbed end region 3105 and second barbed end region 3110. Locking pin 3100 is then inserted into internal restrainer 3095, whereby to cause the pairs of fingers 3145 disposed at each end of internal restrainer 3095 to project through cutouts 3140 of intramedullary fusion device 3090, whereby to lock central bridge region 3115 of intramedullary fusion device 3090 in its strained (i.e., longitudinally stretched) state. The external load stretching central bridge region 3105 (i.e., via the aforementioned stretching mechanism, not shown) can now be removed, and central bridge region 3115 of intramedullary fusion device 3090 will remain in its strained (i.e., stretched) state due to the action of internal restrainer 3095 and locking pin 3100. However, when locking pin 3100 is thereafter removed from the interior of internal restrainer 3095, the pairs of fingers 3145 disposed at each end of internal restrainer 3095 retract from cutouts 3130 by virtue of their inward bias, allowing central bridge region 3115 of intramedullary fusion device 3090 to foreshorten, whereby to generate compression between the bone fragments, as will hereinafter be discussed.

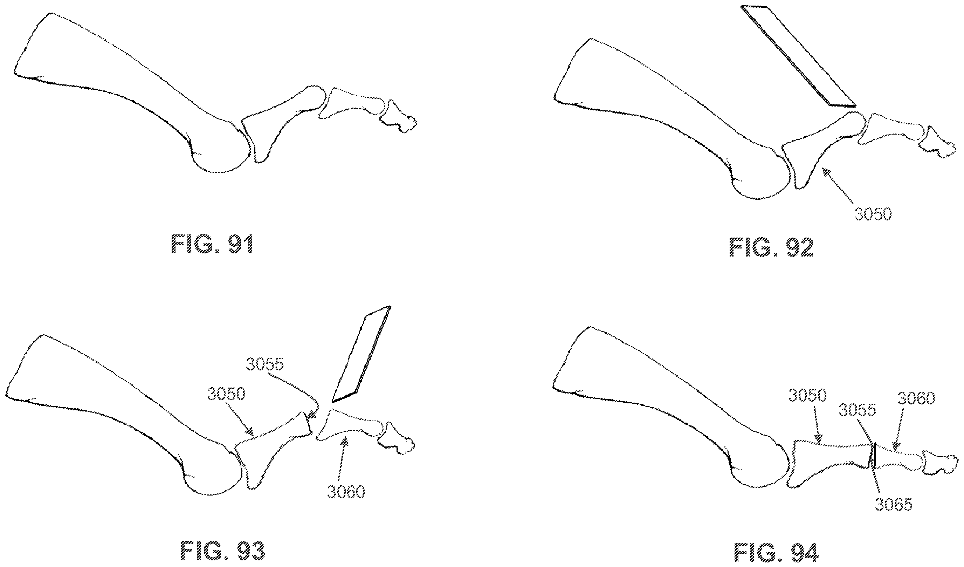

Intramedullary fusion system 3085 may be used to secure together two bone fragments and apply compression to the fracture line. By way of example but not limitation, and looking now at Figs. 91-101, intramedullary fusion system 3085 may be used to treat a hammertoe deformity (Fig. 91).

First, the distal end of metatarsal 3050 is cut off to correct the deformity and create a bone face 3055 suitable for fusion (Figs. 92 and 93). Then the proximal end of phalange 3060 is removed to correct the deformity and create a bone face 3065 suitable for fusion (Figs. 93 and 94). With these two cuts complete, the bones of the metatarsal-phalange joint can be properly aligned (Fig. 94) for subsequent fusion, as will hereinafter be discussed.

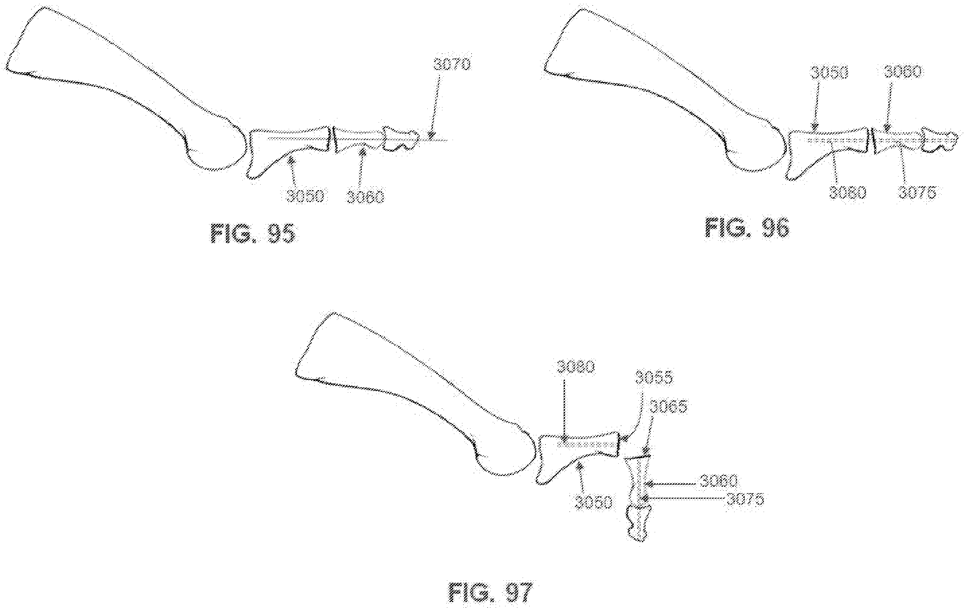

Next, the surgeon inserts k-wire 3070 through the distal end of the toe, then through phalange 3060 and into metatarsal 3050 (Fig. 95). Preferably k-wire 3070 passes down the intramedullary canals of phalange 3060 and metatarsal 3050. Removal of k-wire 3070 leaves a canal 3075 (i.e., the opened intramedullary canal) in phalange 3060 and a canal 3080 in metatarsal 3050, with canal 3075 in phalange 3060 being aligned with canal 3080 in metatarsal 3050 when phalange 3060 is aligned with metatarsal 3050 (Fig. 96). Canals 3075 and 3080 receive intramedullary fusion system 3085 as will hereinafter be discussed.

Following removal of k-wire 3070, phalange 3060 is flexed downward so as to expose the prepared metatarsal face 3055 (Fig. 97).

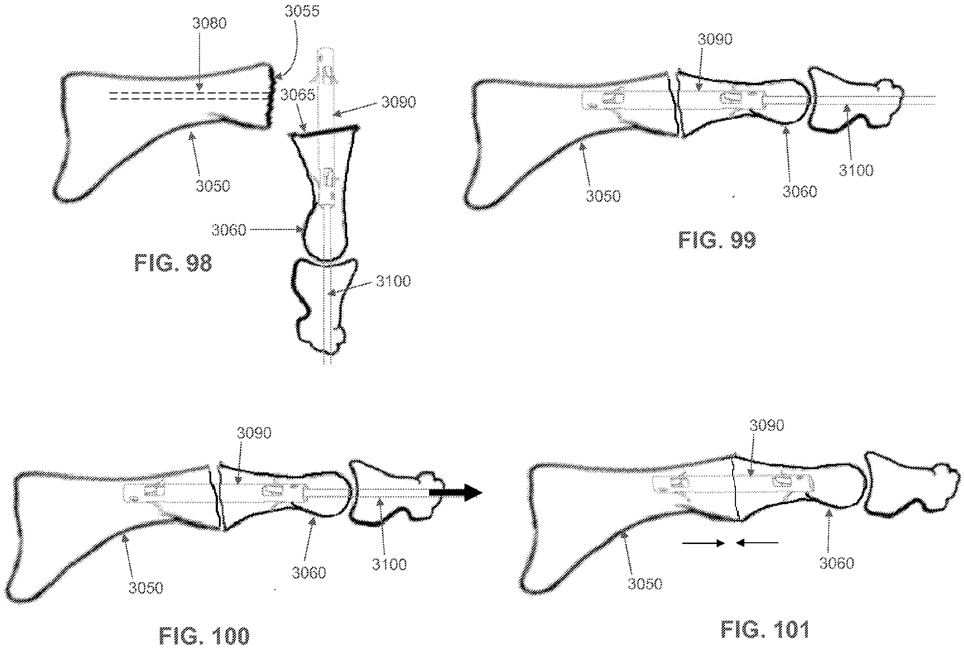

Intramedullary fusion device 3090, which has previously been strained (i.e., its central bridge region 3115 longitudinally stretched) and locked in this state with internal restrainer 3095 and locking pin 3100, is then implanted into canal 3075 in phalange 3060 (Fig. 98), i.e., by advancing the free end of locking pin 3100 and second barbed end region 3110 of intramedullary fusion device 3090 into canal 3075 of phalange 3060. Note that the flare on barbs 3125 of second barbed end region 3110 is such that intramedullary fusion device 3090 can be advanced into canal 3075 of phalange 3060 but not withdrawn. Note also that the free end of locking pin 3100 extends completely out of phalange 3060 and any adjacent bone structure(s) so that it is graspable by the surgeon and able to be retracted when desired through the distal end of the toe.

Phalange 3060 is then reoriented so that first barbed end region 3105 is aligned with canal 3080 in metatarsal 3050, and then phalange 3060 is advanced towards metatarsal 3050 so that first barbed end region 3105 enters canal 3080 of metatarsal 3050 (Fig. 99). Note that the flare on barbs 3120 of first barbed end region 3105 of intramedullary fusion device 3090 is such that intramedullary fusion device 3090 can be advanced into canal 3080 of metatarsal 3050 but not withdrawn.

Phalange 3060 is advanced towards metatarsal 3050 until face 3065 of phalange 3060 engages face 3055 of metatarsal 3050. At this point barbs 3120 of first barbed end region 3105 and barbs 3125 of second barbed end region 3110 prevent phalange 3060 and metatarsal 3050 from moving apart.

With intramedullary fusion device 3090 firmly secured to both phalange 3060 and metatarsal 3050, locking pin 3100 is then removed from internal restrainer 3095 (Fig. 100), i.e., by being retracted through the distal end of the toe. This allows the pairs of fingers 3145 disposed on each end of internal restrainer 3095 to retract from cutouts 3130 of intramedullary fusion device 3090, which allows central bridge region 3115 of intramedullary fusion device 3090 to foreshorten, whereby to generate compression between metatarsal 3050 and phalange 3060 (Fig. 101).

If desired, internal restrainer 3095 may be left in place within intramedullary fusion device 3090 or, more preferably, internal restrainer 3095 may be removed from intramedullary fusion device 3090 after intramedullary fusion device 3090 has been set, e.g., by grasping internal restrainer 3095 with a grasping tool and drawing internal restrainer 3095 longitudinally out of intramedullary fusion device 3090 and then out canal 3075 of phalange 3060. Alternatively, internal restrainer 3095 can be automatically removed from intramedullary fusion device 3090 when locking pin 3100 is removed from internal restrainer 3095, e.g., by providing the distal end of locking pin 3100 and the proximal end of internal restrainer 3095 with an appropriate "catch mechanism" so that the retreating locking pin 3100 engages internal restrainer 3095 and carries internal restrainer 3095 out of intramedullary fusion device 3090 and then out canal 3075 of phalange 3060.

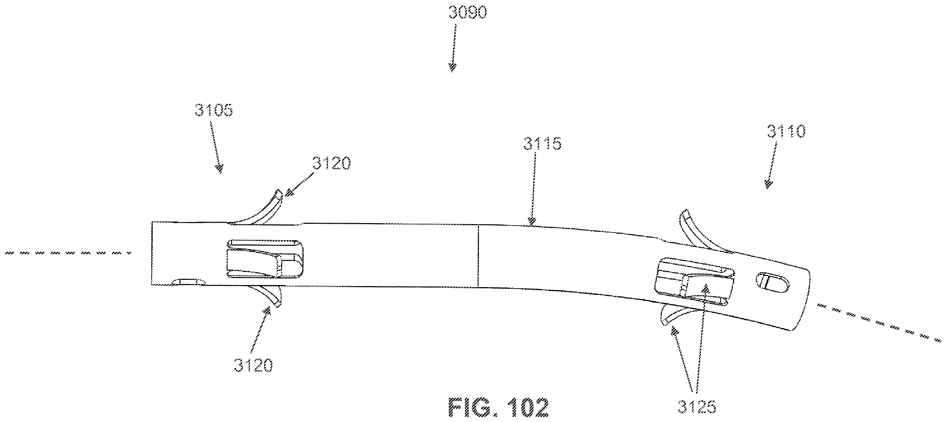

If desired, and looking now at Fig. 102, intramedullary fusion device 3090 can have a slight bend at one or both of its first barbed end region 3105 and second barbed end region 3110. By way of example but not limitation, in the metatarsal-phalange fusion shown in Figs. 91-101, it may be desirable to provide a slight bend to second barbed end region 3110 so as to facilitate restoration of the normal anatomy. In this form of the invention, intramedullary fusion device 3090 may be bent after machining and during the working of the shape memory material, e.g., it may be shape-set at the desired angulation through heat treatment.

Intramedullary fusion device 3090 is specifically engineered so not to "tear through" the bone tissue when central bridge region 3115 foreshortens. The compressive forces of intramedullary fusion device 3090 can be controlled by modulating the material properties of the intramedullary fusion device and/or the geometry of the intramedullary fusion device.

The percentage of cold work in the shape memory material forming intramedullary fusion device 3090 affects the compressive force generated by the intramedullary fusion device. As the percentage of cold work increases, the compression force declines. The intramedullary fusion device should, preferably, have between about 15% and 55% cold work to control the recovery force of the intramedullary device.

Another material property that affects the intramedullary fusion device's compression force is the temperature differential between the body that the intramedullary fusion device will be implanted into (assumed to be 37°C, which is the temperature of a human body) and the austenite finish temperature of the shape memory material forming intramedullary fusion device 3090. A smaller temperature differential between the two will result in the intramedullary fusion device generating a small compressive load; conversely, the larger the temperature differential between the two will result in the intramedullary device generating a larger compressive load. The shape memory material that the intramedullary fusion device is made out of should, preferably, have an austenite finish temperature of greater than about -10°C, resulting in a temperature differential of less than about 47°C when the intramedullary fusion device is implanted (assuming that the intramedullary fusion device is implanted in a human body).

The geometry of the intramedullary fusion device also affects the compression force generated. The cross-sectional area of the hollow central bridge region 3115 affects the compression force. As the cross-sectional area increases, so does the compression force that the intramedullary fusion device 3090 will generate. In this respect it should be appreciated that it is beneficial for the compression force generated by the foreshortening of intramedullary fusion device 3090 to be constant as the bone relaxes and remodels. Thus, the cross-section of hollow central bridge region 3115 of intramedullary fusion device 3090 preferably has a constant cross-section over its entire length. Cross-sections that are not uniform over the length of hollow central bridge region 3115 can result in an increase or decrease in compression as the intramedullary fusion device foreshortens.

The barbs 3120, 3125 are important for transmitting the compression force to the bone without "tearing through" the bone. The height, width, and number of barbs 3120, 3125 on the intramedullary device 3090 are all important to the intramedullary device's ability to not "tear through" the bone.

It should also be appreciated that shape memory material can be processed to exhibit two-way shape memory. The intramedullary fusion device 3090 can be trained to have an austenitic shape (i.e., barbs expanded) and a martensitic shape (i.e., barbs extending parallel to the longitudinal axis of intramedullary fusion device 3090) In this case, the barbs can be in their austenitic shape at about body temperature. The barbs can be deformed via the creation of stress induced martensite to implant the intramedullary fusion device. If the intramedullary fusion device thereafter needs to be removed, the intramedullary fusion device may be cooled (e.g., with cold saline) to a temperature below the austenite start temperature of the shape memory material, and more preferably below the martensite start temperature of the shape memory material, and most preferably below the martensite finish temperature of the shape memory material. When cooled, the intramedullary fusion device 3090 will take on its martensitic shape (i.e., the barbs laying parallel to the longitudinal axis of the intramedullary fusion device), and the surgeon can easily remove the intramedullary fusion device.

Additionally, the intramedullary fusion device can be made such that central bridge region 3115 of intramedullary fusion device 3090 has one austenite start temperature, and such that barbs 3120, 3125 have a lower austenite start temperature. Thus, the intramedullary fusion device can be stretched at a temperature less than the austenite start temperature of central bridge region 3115 but above the austenite start temperature of barbs 3120, 3125. Thus barbs 3120, 3125 will be in the austenite phase and able to undergo a stress induced martensite transformation during insertion of intramedullary fusion device 3090 into a bone canal. Maintaining the intramedullary fusion device at a temperature below the austenite start temperature of central bridge region 3115 allows the intramedullary fusion device to remain in its elongated state. The intramedullary fusion device can then be advanced into a bone canal as discussed above. When the central bridge region 3115 warms (either to body temperature, or to a temperature above body temperature, e.g., through the application of warm saline), central bridge region 3115 will foreshorten, generating and maintaining compression across the fracture line.

It should also be appreciated that central bridge region 3115 of intramedullary fusion device 3090 can be processed so as to be malleable (i.e., to take a set). At body temperature, first barbed end region 3105 and second barbed end region 3110 can be superelastic while central bridge region 3115 can be fully annealed Nitinol or martensitic Nitinol. This allows the surgeon to deform the implant at the time of surgery to the bend desired.

### Further Aspects

In one preferred embodiment of the present invention, the force generated by the intramedullary fusion device (e.g., intramedullary fusion device 3005) is controlled by stretching (i.e., straining) the device approximately 8%, and then allowing the device to partially recover (e.g., to shorten) so that the device is approximately 6% longer than its original length. With the device stretched 6% longer than its original length, a constraint or constraints (e.g., the aforementioned retaining tabs 3030, 3040) can be removably installed on the device to prohibit further recovery. The device can then be implanted. The partial recovery of strain (i.e., from an 8% stretch to a 6% stretch) occurs in the Load Transition zone at stress levels that may be in excess of the shear strength of bone. In other words, by allowing the device to partially recover from strain prior to implantation (i.e., from an 8% stretch to a 6% stretch), the highest stress levels associated with the upper plateau at 8% stretch may be prevented from being applied to the bone, and only the lower plateau stress levels associated with the remaining 6% stretch may be applied to the bone (i.e., partial recovery of strain prior to implantation occurs in the load transition zone). Following implantation of the device, the constraint or constraints (e.g., the aforementioned retaining tabs 3030, 3040) can be removed from the device, thereby allowing the device to attempt to recover the remaining strain. The subsequent strain recovery will occur directly at the stress level of the lower plateau of the mechanical hysteresis curve of the Nitinol device. The stress level of the lower plateau may be less than the shear strength of the bone, thereby keeping the device from damaging in the bone.

### Example 4

### Plates For Generating, Applying And Maintaining Compression Within A Body

### Novel Compression Plate Comprising Elastic Bridge Members

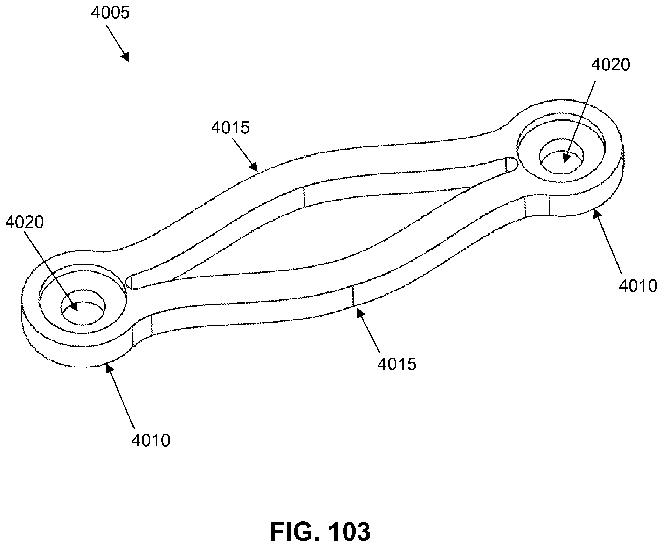

Looking next at Fig. 103, there is shown a novel compression plate 4005 which is able to bring bone fragments into close proximity with each other, generate compressive load across the fracture line (i.e., between the cortical bone and the cancellous bone of the bone fragments), and maintain that compressive load for a prolonged period of time while healing occurs.

Novel compression plate 4005 is preferably a structure manufactured from a single piece of shape memory material (e.g., a material capable of exhibiting superelasticity and/or a temperature-induced shape change). The shape memory material may be a metal alloy (e.g., Nitinol) or a polymer (e.g., appropriately processed PEEK). Compression plate 4005 is designed to reduce fractures and generate and maintain compression between bone fragments in order to aid in fracture healing. Compression plate 4005 generally comprises two opposing regions 4010 joined together by a pair of elastic bridge members 4015, and at least one opening 4020 formed in each of the two opposing regions 4010 for receiving fixation screws. Openings 4020 may have a countersunk feature (e.g., a bore-counterbore configuration) so as to allow the heads of the fixation screws to sit substantially flat with the top surface of compression plate 4005. Additionally, openings 4020 may be threaded so as to allow for positive engagement between the openings and the threaded fixation screws. In the un-restrained state, elastic bridge members 4015 are bowed outwardly, such as in the manner shown in Fig. 103.

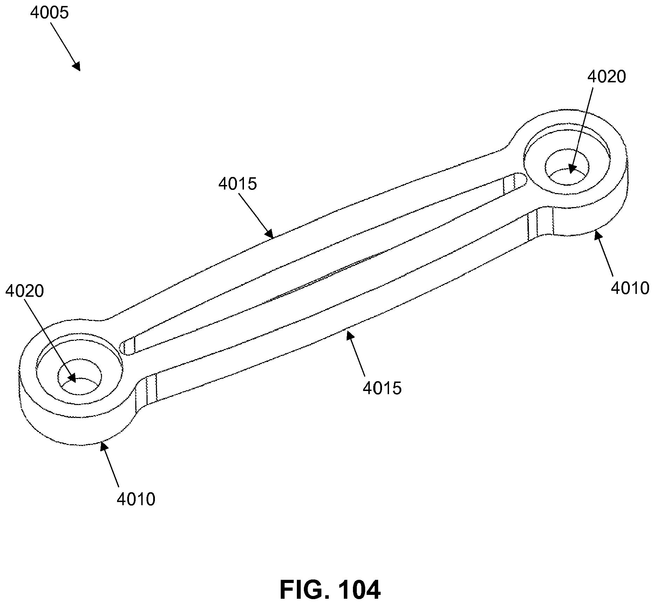

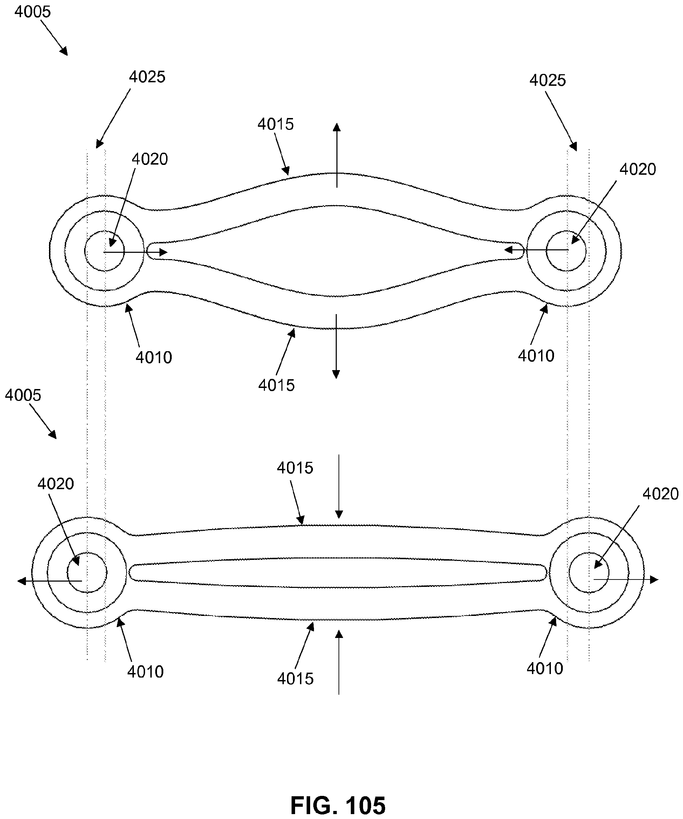

Prior to implantation, elastic bridge members 4015 of compression plate 4005 can be reversibly strained inwardly (i.e., bent laterally inwardly), thus increasing the distance 4025 (Fig. 105) between opposing regions 4010 (and hence openings 4020) of compression plate 4005 (Fig. 104). Note that where compression plate 4005 is formed out of Nitinol, elastic deformations of up to approximately 8% are achievable. A delivery device (see below) can be used to strain elastic bridge members 4015 so as to bring elastic bridge members 4015 to a substantially parallel state.

During implantation, the strained (i.e., elongated) compression plate 4005 is positioned against the bone fragments and secured to those bone fragments by passing fixation screws through openings 4020 and into the bone fragments.

Removal of the induced strain on compression plate 4005 (provided by the delivery device, see below) results in compression plate 4005 attempting to return to its original un-restrained state (Fig. 105), thereby generating a compressive load on the bone (i.e., through elastic bridge members 4015, openings 4020 and fixation screws extending through openings 4020) and maintaining that compressive load on the bone for a prolonged period of time while healing occurs.

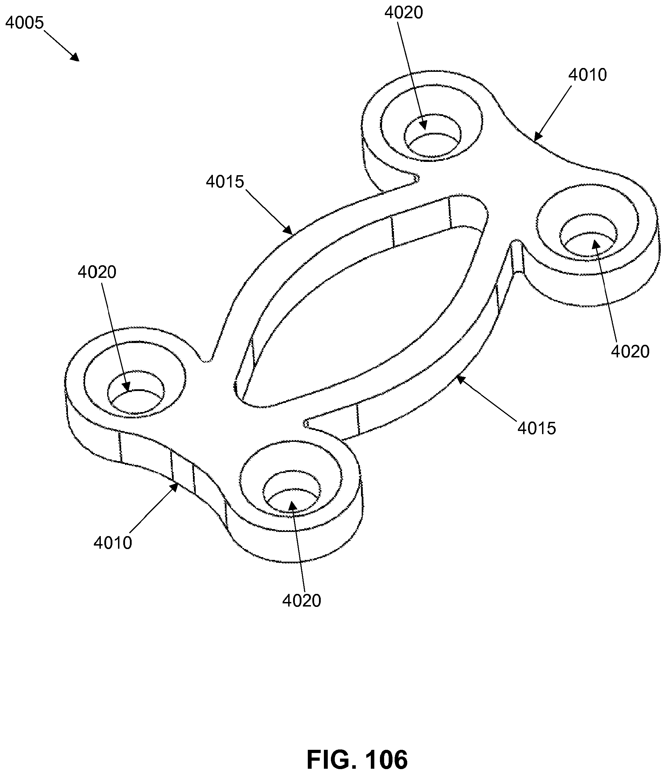

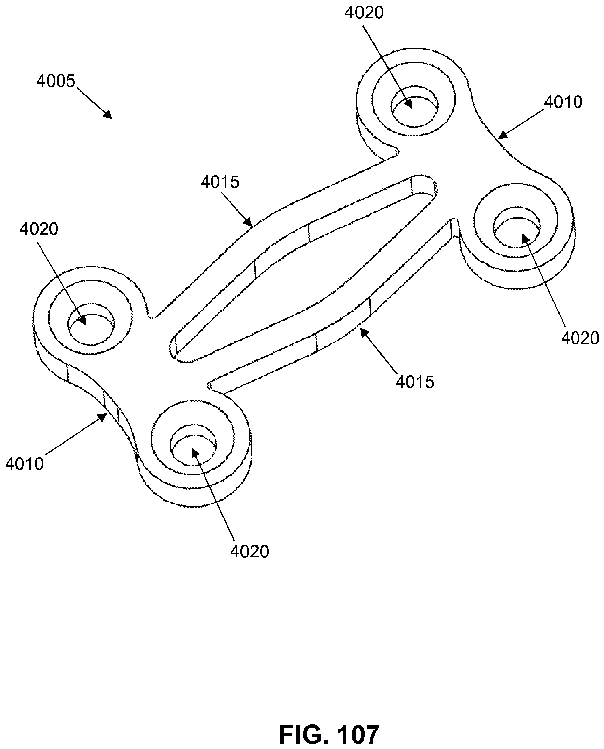

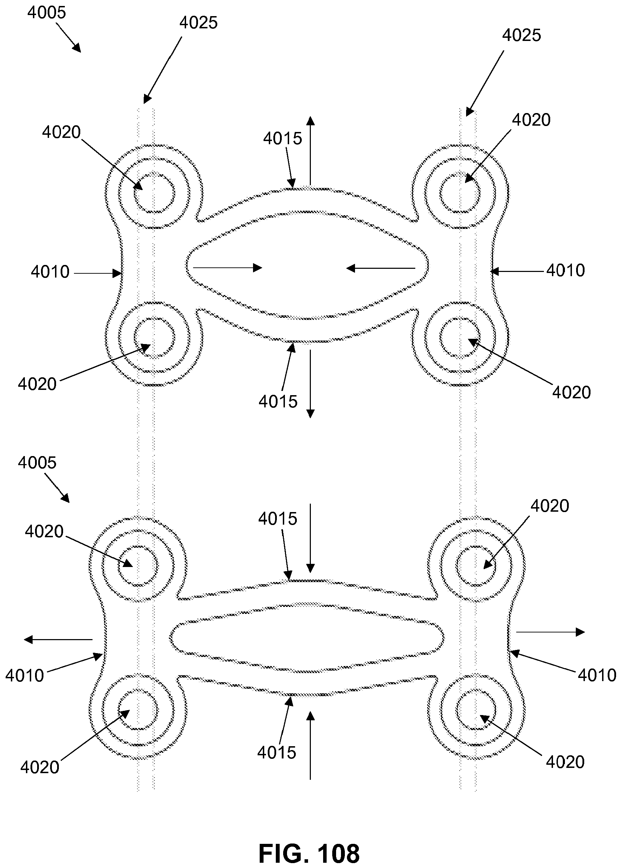

Figs. 106-108 show another compression plate 4005 which is generally similar to the compression plate 4005 shown in Figs. 103-105, except that the opposing regions 4010 of the compression plate shown in Figs. 106-108 each has two openings 4020 for receiving fixation screws.

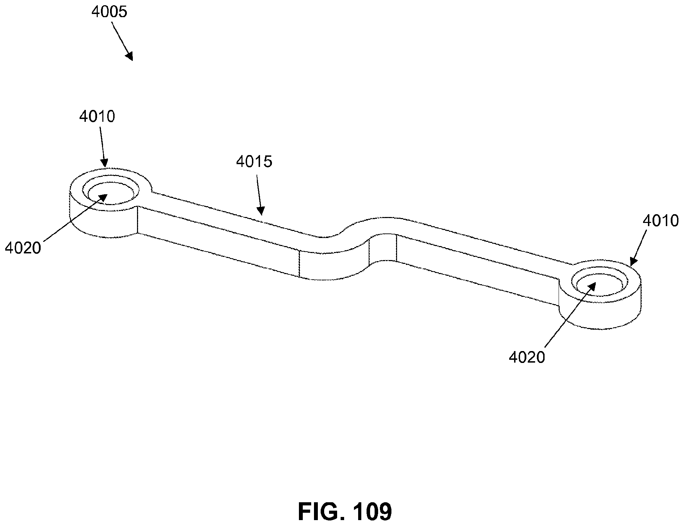

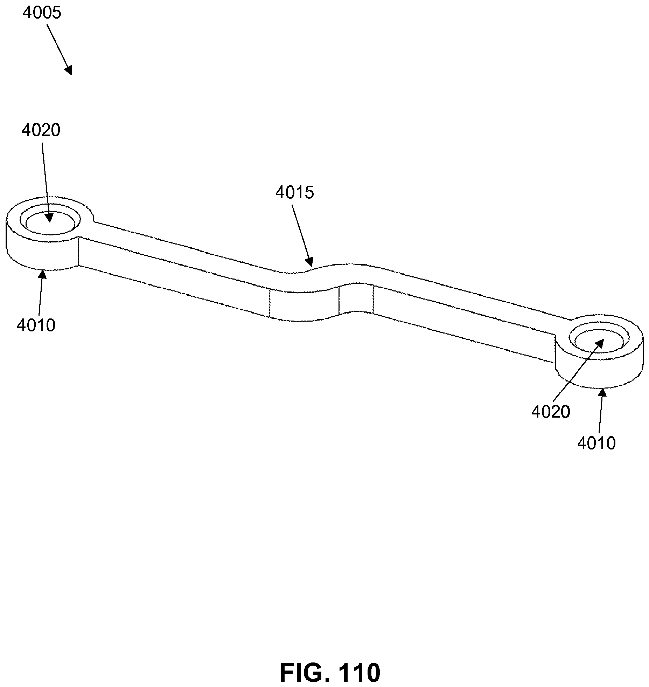

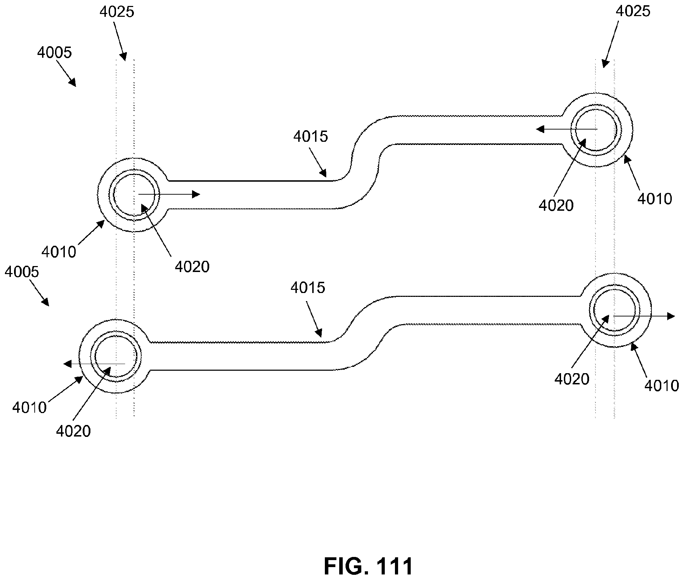

Figs. 109-111 show another novel compression plate 4005 also formed in accordance with the present invention. More particularly, the novel compression plate 4005 of Figs. 109-111 is generally similar to the compression plate 4005 shown in Figs. 103-105, except that the compression plate 4005 of Figs. 109-111 has only one elastic bridge member 4015, and that elastic bridge member 4015 has an "S" configuration. It will be appreciated that the "S" configuration of elastic bridge member 4015 of Figs. 109-111 allows the elastic bridge member to be strained so as to move openings 4020 further apart, and thereafter relaxed so as to cause a foreshortening of the distance between openings 4020. As a result, when compression plate 4005 of Figs. 109-111 is strained so as to move openings 4020 further apart, and fixation screws are thereafter passed through openings 4020 of the strained compression plate and advanced into bone, and the strain on elastic bridge member 4015 is thereafter released, the compression plate and fixation screws will apply a compressive force to the bone.

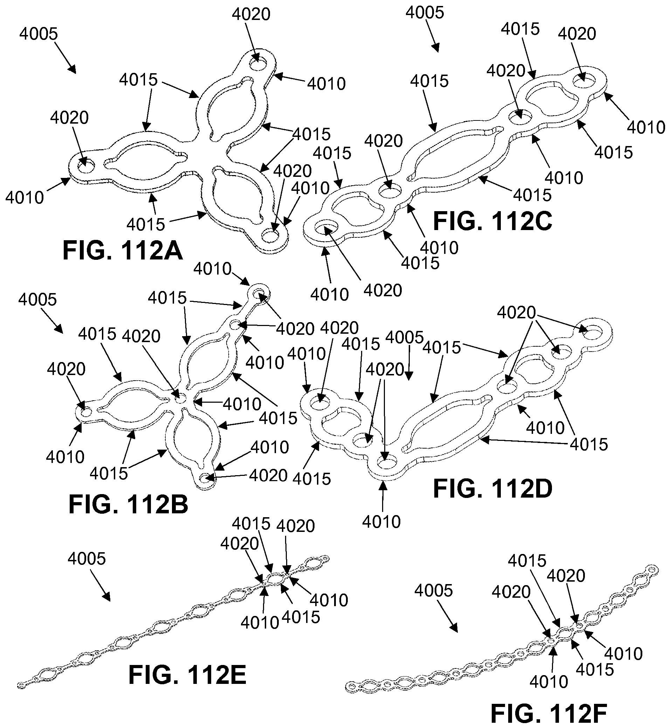

Figs. 112A-112F are schematic views showing additional novel compression plates 4005 also formed in accordance with the present invention - these compression plates may be used when more tailored anatomical fixation is required (e.g., for Cranio-Maxillofacial fracture fixation).

Thus it should be appreciated that compression plate 4005 may be formed in a variety of linear and non-linear configurations, and may include two or more opposing regions 4010 and one or more elastic bridge members 4015. Thus, for example, in one form of the invention, compression plate 4005 may comprise a linear configuration having two opposing regions 4010 connected together by one or more elastic bridge members 4015. In another form of the invention, compression plate 4005 may comprise a linear configuration having three or more opposing regions 4010, each pair of opposing regions being connected together by one or more elastic bridge members 4015. In still another form of the invention, compression plate 4005 may comprise a non-linear configuration (e.g., a curved configuration) having two opposing regions 4010 connected together by one or more elastic bridge members 4015. In still another form of the invention, compression plate 4005 may comprise a non-linear configuration (e.g., a triangular configuration, a L-shaped configuration, etc.) having three or more opposing regions 4010, each pair of opposing regions being connected together by one or more elastic bridge members 4015. It will be appreciated that compression plate 4005 may also comprise still other configurations, e.g., square, circular, etc., all of which are considered to be within the scope of the present invention.

It will be appreciated that the fixation screws used with compression plate 4005 may be conventional fixation screws of the sort well known in the field of fracture fixation.

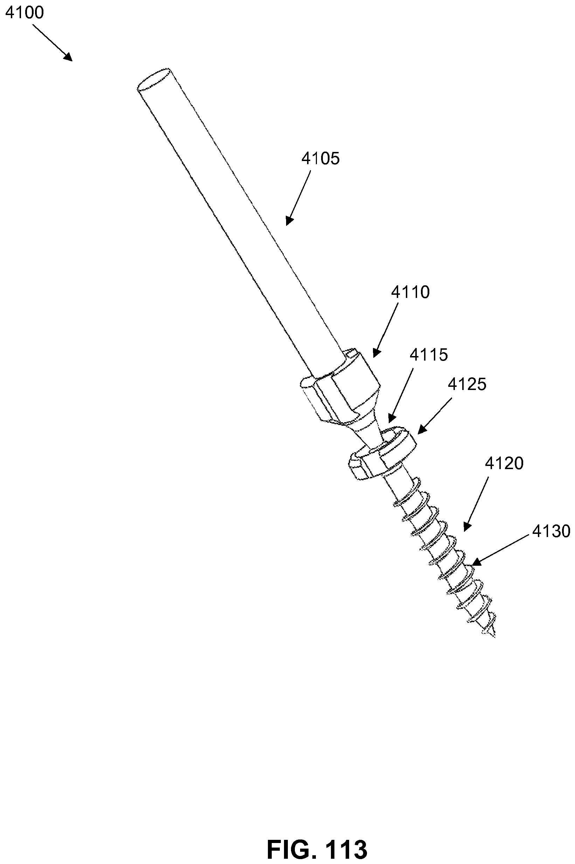

By way of example but not limitation, and looking now at Fig. 113, there is shown a snap-off screw system 4100 of the sort well known in the art. This snap-off screw system can be used to secure compression plate 4005 to bone. The snap-off screw system 4100 has a shaft 4105 to engage a rotary driver (not shown), a drive head 4110 to engage a screw driver (not shown), a preferred shear location 4115, and a fixation screw 4120 provided with a screw head 4125 and screw threads 4130. Screw head 4125 may have external threads (not shown) to engage mating threads on opening 4020 of compression plate 4005, so that fixation screw 4120 may function as a locking screw.

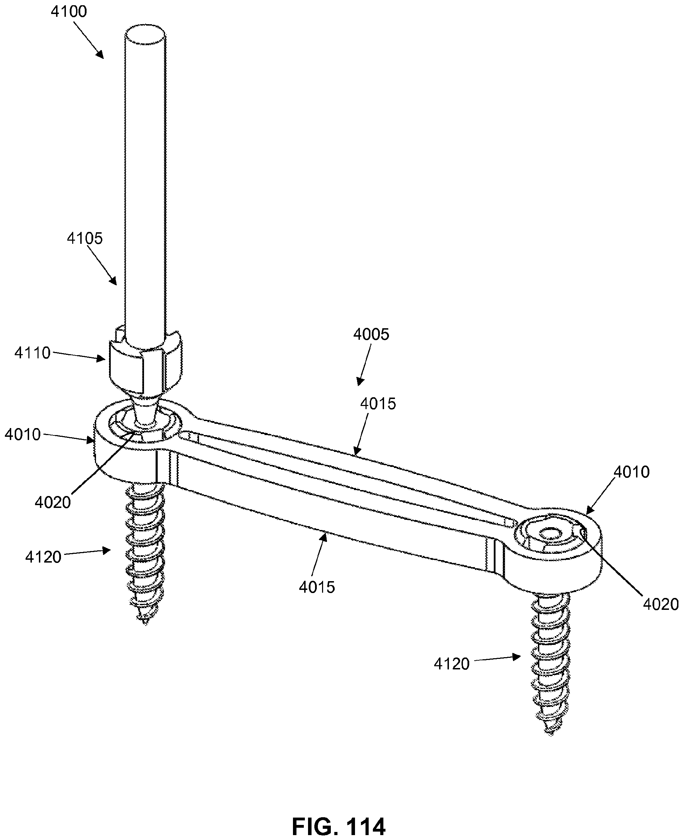

Fig. 114 shows compression plate 4005 with snap-off screw system 4100 being used to deploy fixation screws 4120 through openings 4020 and into bone. Note that in Fig. 114, compression plate 4005 is shown with its elastic bridge members 4035 strained inwardly, although the delivery device (which bends elastic bridge members 4015 inwardly) has been omitted for clarity of illustration.

It will be appreciated that various delivery devices may be used to deploy compression plate 4005 in the body.

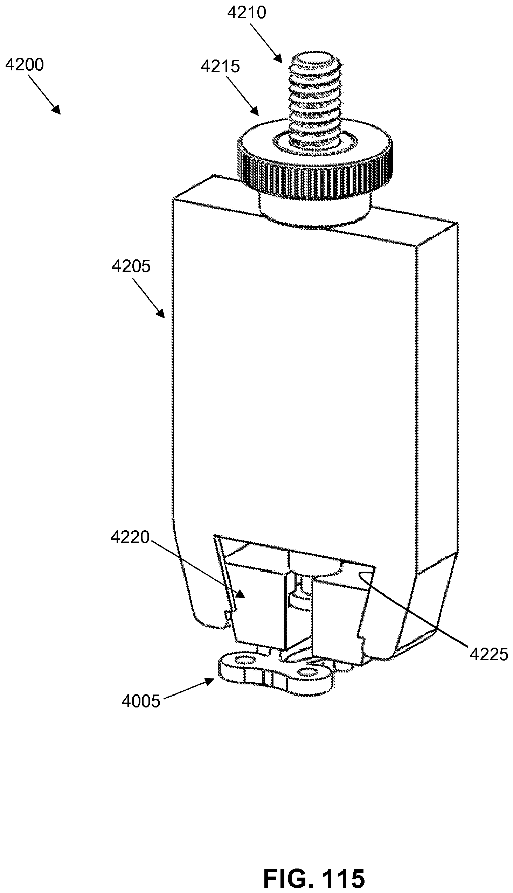

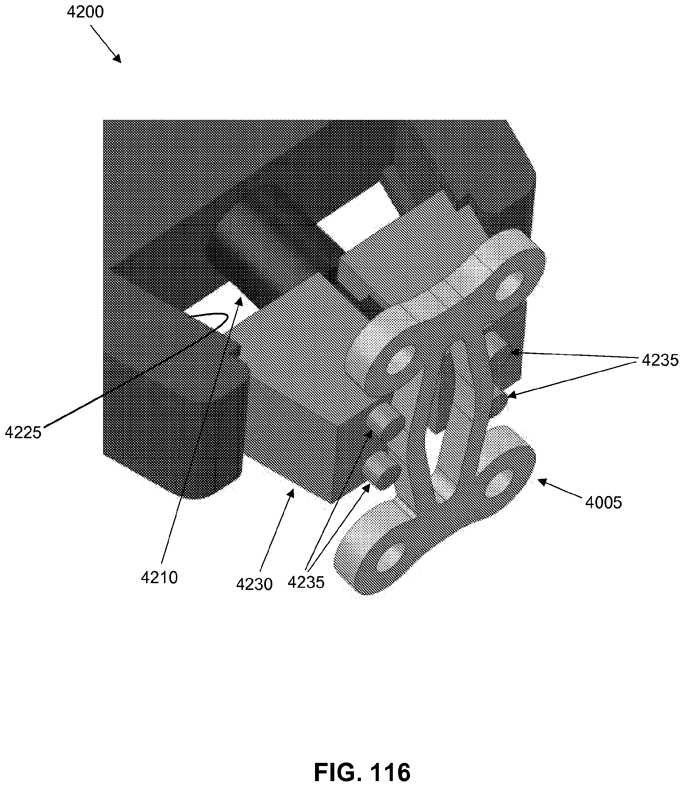

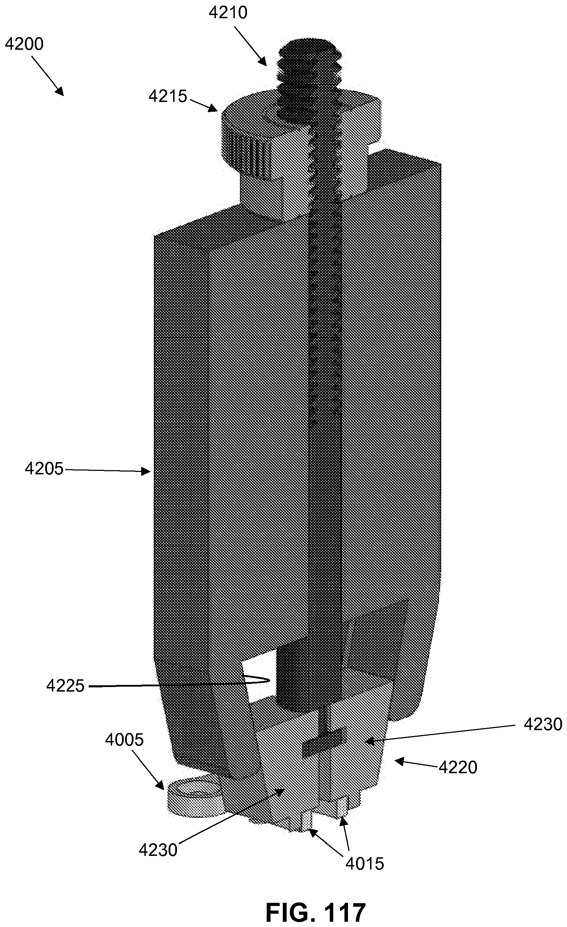

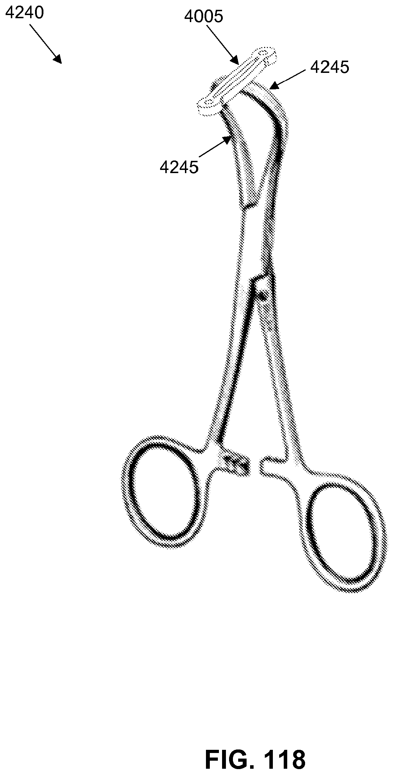

By way of example but not limitation, and looking now at Figs. 115-117, there is shown an exemplary delivery device 4200 which may be used to strain (i.e., compress) elastic bridge members 4015. Delivery device 4200 generally comprises an outer body 4205, a central screw 4210, a nut 4215 and a wedge assembly 4220. Plate 4005 is engaged by wedge assembly 4220 which is movably disposed in a wedge-shaped recess 4225 formed in outer body 4205. Wedge assembly 4220 comprises two individual wedges 4230 that ride in wedge-shaped recess 4225 (formed in outer body 4205) and engage screw 4210. The distal end of each individual wedge 4230 has two pins 4235 that engage elastic bridge members 4015 of compression plates 4005. When screw 4210 is a conventional right-handed thread, loosening nut 4215 causes wedge assembly 4220 to move downward in wedge-shaped recess 4225 of outer body 4205, thereby reducing the distance between pins 4235. This causes elastic bridge members 4015 of compression plate 4005 to elastically deform to a near-parallel (or at least to a more parallel) condition. Tightening nut 4215 causes wedge assembly 4220 to move upward in wedge-shaped recess 4225 of outer body 4205, thereby increasing the distance between pins 4235. This releases the strain on elastic bridge members 4015 of compression plate 4005, thereby allowing the elastic bridge members 4015 to return to their original, outwardly-bowed shape. Fig. 118 shows another delivery device 4240 which may be used to deploy compression plate 4005. The delivery device 4240 shown in Fig. 118 is essentially hemostat pliers, with the working tips 4245 of the hemostat pliers applying the strain on elastic bridge members 4015 of compression plate 4005.

### Novel Method For Applying Compression To A Fracture Using The

### Novel Compression Plate

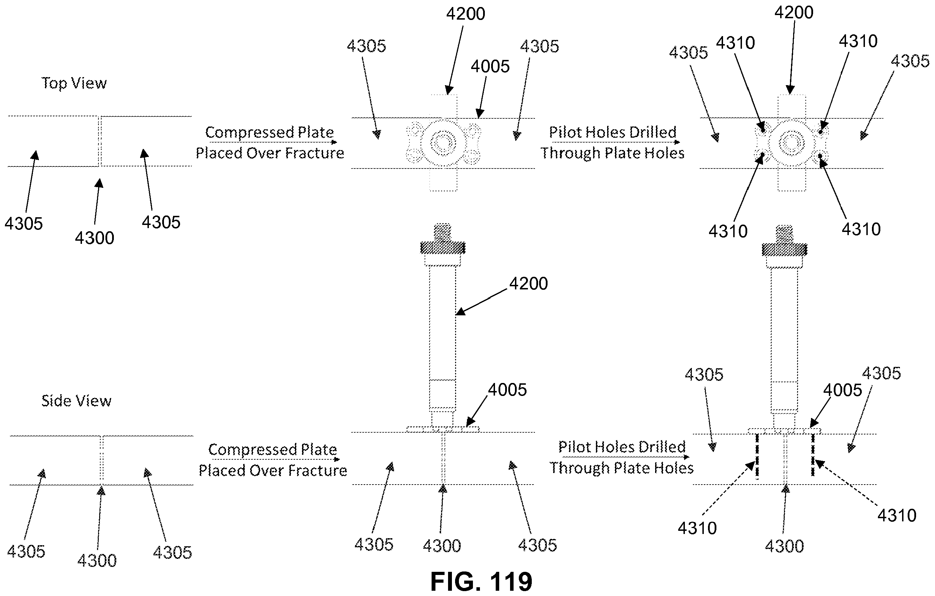

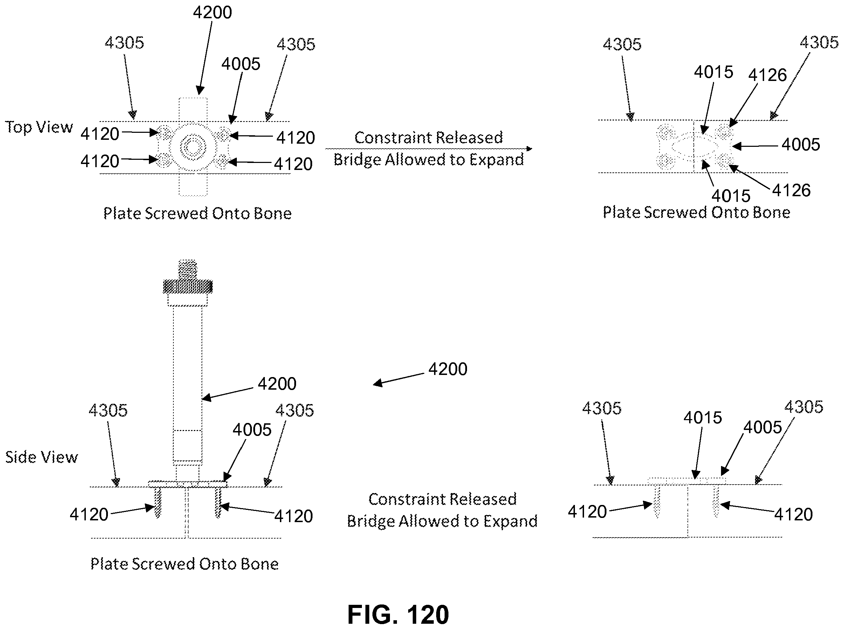

Now next at Fig. 119 and 120, there is shown one preferred method for treating a fracture 4300 formed between two bone fragments 4305 using novel compression plate 4005.

First, compression plate 4005 is loaded onto the delivery device (e.g., delivery device 4200 discussed above) and elastic bridge members 4015 are compressed so that they are near-parallel (or at least more parallel). The constrained compression plate 4005 is then placed over the fracture line 4300 and holes 4310 are drilled through openings 4020. Screws (e.g., snap-off screws 4120 of snap-off screw system 4100) are installed (i.e., they are advanced through openings 4020 and into bone fragments 4305), thereby fixing compression plate 4005 to the bone fragments 4305. The constraining delivery device (e.g., delivery device 4200) is then removed and the compression plate 4005 is allowed to attempt to regain its original unconstrained shape, thereby generating and maintaining compression across the fracture site.

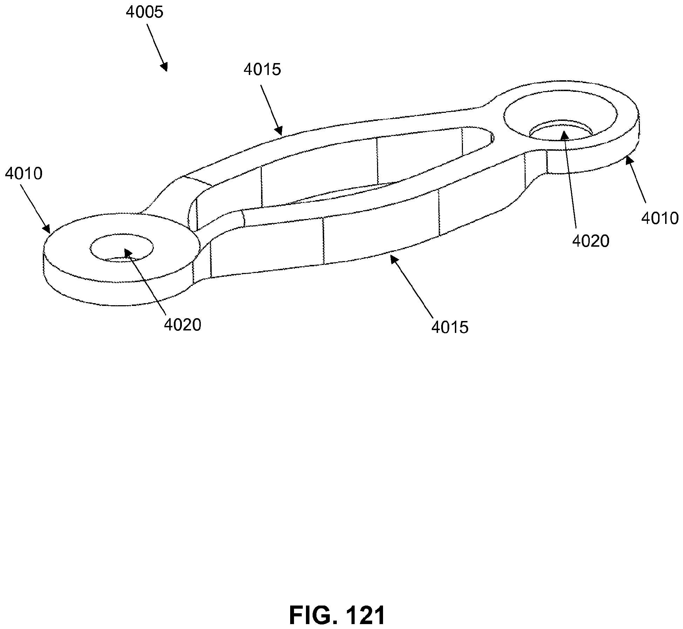

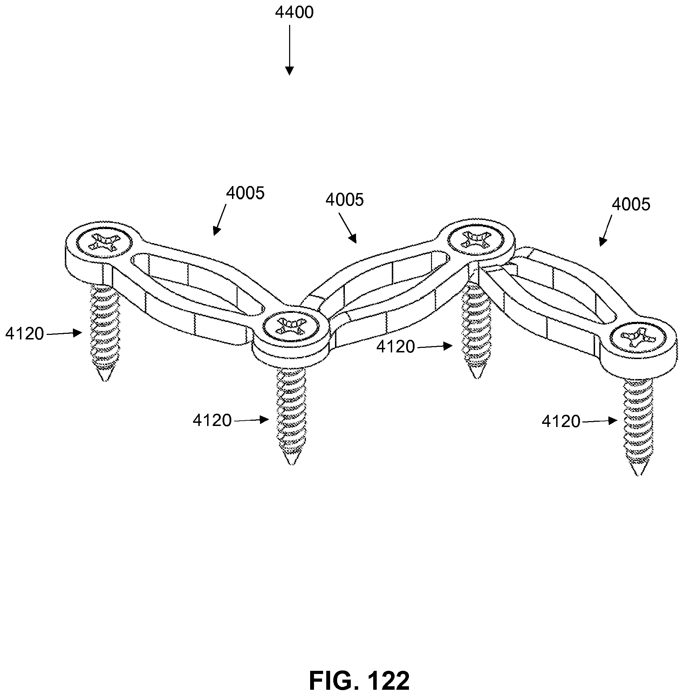

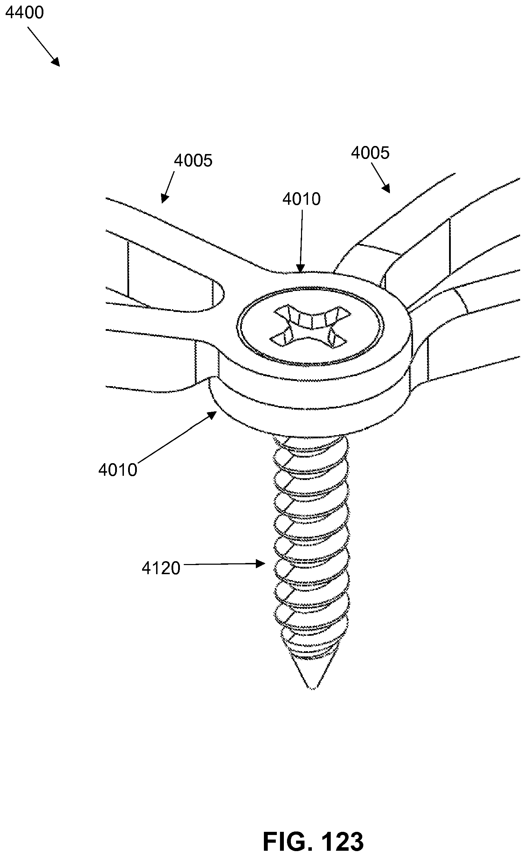

Figs. 121-123 show another novel compression plate 4005 formed in accordance with the present invention. More particularly, in this form of the invention, compression plate 4005 is configured so as to allow multiple compression plates 4005 to be assembled together as a modular plating system 4400. If desired, one or both of the two opposing regions 4010 of each compression plate 4005 may be formed with a height equal to one-half of the height of the two elastic bridge members 4015 and, if desired, an opposing region 4010 may be aligned with the top surface of the elastic bridge members 4015, or the opposing region 4010 may be aligned with the bottom surface of the elastic bridge member 4015. In this way, adjacent regions 4010 can be combined so as to form a lap joint which receives a fixation screw 4120.

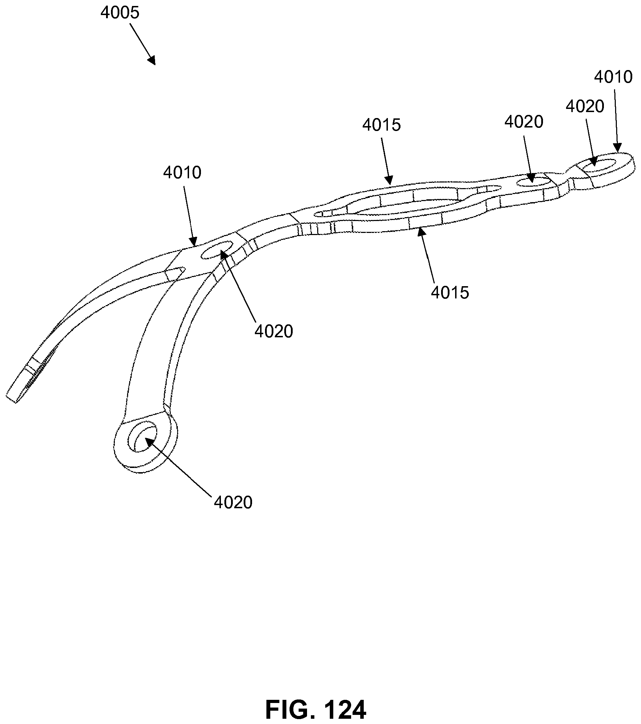

Fig. 124 shows a novel compression plate 4005 that has had areas of the compression plate selectively processed (e.g., heat treated) so as to create plastic areas (e.g., opposing regions 4010) which can be bent to take a set. This construction allows the compression plate to be contourable to the patient's anatomy (using the areas of plastic deformation) while also generating and maintaining compression across fracture sites (using the areas of elastic deformation). In one preferred form of the invention, compression plate 4005 is formed out of Nitinol; the plastic areas of compression plate 4005 (e.g., opposing regions 4010) are formed out of fully annealed Nitinol or martensitic Nitinol with an austenite start temperature greater than body temperature; and the elastic areas of compression plate 4005 (e.g., elastic bridge members 4015) are formed out of austenitic Nitinol but capable of forming stress-induced martensite.

Note that compression plate 4005 is configured so that the force which is generated as compression plate 4005 reconfigures (i.e., as elastic bridge members 4015 return outwardly) is less than the "tear through" force of the bone which receives screws 4120, i.e., compression plate 4005 is specifically engineered so as to not "tear through" the bone tissue when attempting to reconfigure to its original foreshortened configuration. The compressive forces of compression plate 4005 can be controlled by (i) modulating the material properties of the compression plate, and/or (ii) varying the geometry of the compression plate.

The percentage of cold work in the shape memory material forming compression plate 4005 affects the compressive force generated by the reconfiguring compression plate. As the percentage of cold work increases, the compression force declines. A Nitinol compression plate should, preferably, have between about 15% and 55% cold work to control the recovery force of the compression plate; however, other degrees of cold work may be used, and/or the material may not be cold worked at all.

Another material property that affects the plate's compression force is the temperature differential between the body that the compression plate will be implanted into (assumed to be 37°C, which is the temperature of a human body) and the austenite finish temperature of the shape memory material forming compression plate 4005. A smaller temperature differential between the two will result in the compression plate generating a smaller compressive load; conversely, a larger temperature differential between the two will result in the compression plate generating a larger compressive load. When the compression plate is made out Nitinol, the compression plate should, preferably, have an austenite finish temperature of greater than about -10°C, resulting in a temperature differential of about 47°C when the compression plate is implanted (assuming that the compression plate is implanted in a human body).

Plate geometry also affects the compression forces which are generated by the reconfiguring plate. More particularly, the cross-sectional area of elastic bridge members 4015 affects the compression forces generated by the reconfiguring plate. As the cross-sectional areas increase, so do the compression forces that the reconfiguring plate will generate. It should be appreciated that the force generated as an elastic bridge member 4015 attempts to recover from the constrained linear configuration (Fig. 104) to the bowed outward configuration (Fig. 103) is less than the force which would be generated were the plate to be constructed with linear elastic bridge members that are stretched longitudinally in tension. A plate with linear elastic bridge members that are stretched longitudinally in tension may recover with a force that exceeds the pull-out force in bone.

In one preferred form of the invention, compression plate 4005 and delivery device 4200 are provided in the form of a sterilized kit. The kit may include additional instruments to aid in the implantation of the compression plate (e.g., k-wire, drill bit, plate size guide, screws, etc.). In one preferred form of the invention, compression plate 4005 is provided with an associated delivery device (e.g., delivery device 4200) in a sterile package, with elastic bridge members 4015 being pre-constrained (e.g., so that elastic bridge members 4015 are substantially straight, or at least more straight than when the elastic bridge members are in their unconstrained condition) in the sterile package by the delivery device (e.g., delivery device 4200).

### Test Data

Static Elastic Bending: 25mm interaxis width compression plates were tested. A 4-point bending load was applied on a tensile testing machine at a rate of 25.4mm/min. Bending stiffness was calculated as the initial slope of load vs. displacement. The novel compression plate 4005 had comparable Bending Strengths (16.33N/mm vs. 15.86N/mm *p*=*0.818*) to conventional fracture fixation plates.

Compression Force: Greater interfragmentary compression has been found to enhance the healing of fractured bones. The compression plates of the present invention utilize the unique superelastic properties of Nitinol to enhance and sustain compression across the fracture plane. The compressive force generated by the novel compression plates of the present invention generate and maintain 180N of compression over nearly 1mm of bone resorption.

### Use Of Plastic Threaded Inserts Between The Openings Of The

### Compression Plate And The Fixation Screws

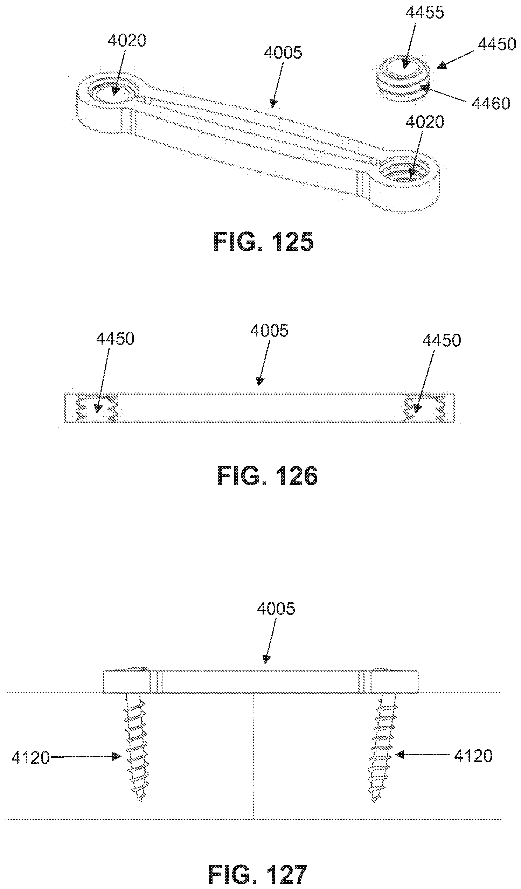

If desired, and looking now at Figs. 125-127, a plastic threaded insert 4450 (with a central hole 4455 and external threads 4460) can be inserted into mating threaded holes 4020 on compression plate 4005. Threaded plastic inserts 4450 can accept a fixation screw (e.g., a fixation screw 4120) with appropriate locking threads on the head of the fixation screw. The threads on the head of the fixation screw are able to cut threads into plastic threaded insert 4450, locking the fixation screw to compression plate 4005 via the intervening plastic threaded insert 4450. Note that the fixation screws can be inserted on an angle (e.g., up to 15 degrees) off the center axis of threaded holes 4020, allowing for polyaxial fixation of compression plate 4005.

Additionally, it should be appreciated that plastic threaded insert 4450 insulates the metallic fixation screw from compression plate 4005, thereby limiting galvanic corrosion between the compression plate 4005 and the metallic fixation screws.

### Use Of Other Types Of Fasteners With The Novel Compression Plate

In the foregoing disclosure, compression plate 4005 is secured to bone fragments using threaded fixation screws. However, if desired, other types of fasteners may also be used to secure compression plate 4005 to bone fragments. By way of example but not limitation, pins may be used to secure compression plate 4005 to bone fragments.

### Applying Distraction To Bone Using A Novel Distraction Plate

In the foregoing disclosure, compression plate 4005 has been discussed in the context of providing compression across a fracture. However, it should also be appreciated that, if desired, the apparatus can be modified so as to provide a distraction force to bone (e.g., to separate two bones or bone fragments, or to induce bone growth in a single bone, etc.).

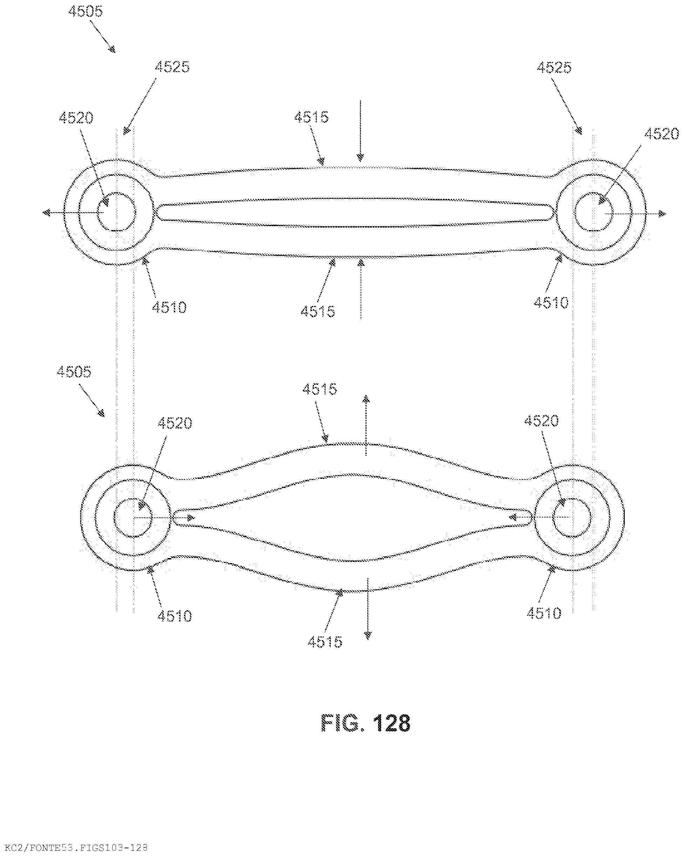

By way of example but not limitation, and looking now at Fig. 128, there is shown a novel distraction plate 4505 formed in accordance with the present invention. Distraction plate 4505 generally comprises two opposing regions 4510 joined together by a pair of elastic bridge members 4515, and at least one opening 4520 formed in each of the two opposing regions 4510 for receiving fixation screws. Openings 4520 may have a countersunk feature (e.g., a bore-counterbore configuration) so as to allow the heads of the fixation screws to sit substantially flat with the top surface of distraction plate 4505. Additionally, openings 4520 may be threaded so as to allow for positive engagement between the openings and the threaded fixation screws. In the un-restrained state, elastic bridge members 4515 are linearly aligned, such as in the manner shown in Fig. 128. Note that this is the inverse of the configuration of compression plate 4005, where elastic bridge members 4015 are bowed outwardly when in their un-restrained state.

Prior to implantation, elastic bridge members 4515 of distraction plate 4505 can be reversibly strained outwardly (i.e., forced outwardly), thus decreasing the distance 4525 between opposing regions 4510 (and hence openings 4520) of distraction plate 4505. A delivery device can be used to strain elastic bridge members 4515 so as to force elastic bridge members 4515 outwardly, e.g., a delivery device generally similar to the delivery device 4200, or the delivery device 4240, discussed above, except that the delivery device is configured to force elastic bridge members 4515 apart when appropriate.

During implantation, the strained (i.e., the forcibly foreshortened) distraction plate 4505 is secured to bone by passing fixation screws through openings 4520 and into the bone.

Removal of the induced strain on distraction plate 4505 (provided by the aforementioned delivery device) results in distraction plate 4505 attempting to return to its original un-restrained state (i.e., with elastic bridge members 4515 linearly aligned), thereby generating a distraction load on the bone to which distraction plate 4505 is secured (i.e., through elastic bridge members 4515, openings 4520 and fixation screws extending through openings 4520), and maintaining that distraction load on the bone for a prolonged period of time while healing occurs.

### Further Aspects

In one preferred embodiment of the present invention, the force generated by the compression plate is controlled by stretching (i.e., straining) the compression plate approximately 8%, and then allowing the compression plate to partially recover (e.g., to shorten) so that the compression plate is approximately 6% longer than its original length. With the compression plate stretched 6% longer than its original length, a constraint (e.g., delivery device 4200) can be removably installed on the compression plate to prohibit further recovery. The compression plate can then be implanted. The partial recovery of strain (i.e., from an 8% stretch to a 6% stretch) occurs in the Load Transition zone at stress levels that may be in excess of the shear strength of bone. In other words, by allowing the compression plate to partially recover from strain prior to implantation (i.e., from an 8% stretch to a 6% stretch), the highest stress levels associated with the upper plateau at 8% stretch may be prevented from being applied to the bone, and only the lower plateau stress levels associated with the remaining 6% stretch may be applied to the bone (i.e., partial recovery of strain prior to implantation occurs in the load transition zone). Following implantation of the compression plate, the constraint (e.g., delivery device 4200) can be removed from the compression plate, thereby allowing the compression plate to attempt to recover the remaining strain. The subsequent strain recovery will occur directly at the stress level of the lower plateau of the mechanical hysteresis curve of the Nitinol compression plate. The stress level of the lower plateau may be less than the shear strength of the bone, thereby keeping the fixation screws (e.g., fixation screws 4120) associated with the compression plate from stripping in the bone.

In another embodiment of the present invention, it is possible to control the unloading of the shape memory compression plate as defined above while also providing regions of the compression plate that are malleable and can be contoured to the patient's specific anatomy. During manufacturing, regions of the compression plate may be selectively heat treated at 400°C for 70 minutes. This will selectively age those regions of the shape memory material so that the austenite finish temperature of only those regions of the compression plate is 40°C (i.e., above body temperature). Thus, those regions of the compression plate will be martensitic and malleable. The surgeon can then bend those regions of the compression plate so that it matches the contour of the bone.

### Other Applications For Novel Compression Plate

The present invention also finds utility as a cervical compression plate, and/or as other spinal compression plates. Additionally, the novel compression plate can be used as a fixation device for sternal closure. The novel compression plate finds particular utility for sternal closures where the design of the compression plate allows the tensile forces on the anterior of the sternum (generated during respiration) to be converted to compressive forces.

### Application To Shape Memory Materials Other Than Nitinol

It should be appreciated that the unloading stress of shape memory materials other than Nitinol can also be controlled using the approaches described herein. More particularly, the approaches described herein are applicable for other shape memory materials that exhibit stress-strain mechanical hysteresis curves similar to that of Nitinol. These other shape memory materials may comprise metal alloys or polymers.

By way of example but not limitation, these other shape memory materials may comprise the following metal alloys:
Ag-Cd 44/49 atomic%Cd;
Au-Cd 46.5/50 atomic% Cd;
Cu-Al-Ni 14/14.5 weight% Al and 3/4.5 weight% Ni;
Cu-Sn approx. 15 atomic% Sn;
Cu-Zn 38.5/41.5 weight% Zn;
Fe-Pt approx. 25 atomic% Pt;
Mn-Cu 5/35 atomic % Cu;
Fe-Mn-Si;
Co-Ni-Al;
Co-Ni-Ga;
Ni-Fe-Ga;
Ti-Nb;
Ni-Ti approx. 55-60 weight% Ni;
Ni-Ti-Hf;
Ni-Ti-Pd; and
Ni-Mn-Ga.

Additionally, it should be appreciated that the approaches described herein can be applied to controlling the unloading stress of shape memory polymers that exhibit stress-strain mechanical hysteresis curves similar to that of Nitinol. These include, but are not limited to:
crosslinked polyurethane;
PEO-PET block copolymers; and
appropriately processed PEEK.

### Additional Approaches to Control Unloading Stress

It should be appreciated that it may sometimes be desirable to "back off" the strain more than just the amount of strain required to operate on the lower plateau of the mechanical hysteresis curve for the shape memory material. In some cases (e.g., for a long compression screw), it may be desirable to have the screw shorten by a fixed number of millimeters following implantation, and this distance may be less than the full distance of the lower plateau of the mechanical hysteresis curve. By way of example but not limitation, for a 100 mm screw to shorten 2 mm, the screw can be strained to 8%, then "backed off' to 6% strain so as to be on the lower plateau of the mechanical hysteresis curve, and then "backed off' further along the lower plateau of the mechanical hysteresis curve until the screw is at only 2% strain, whereup the screw can be constrained. During use, the screw is then released from its 2% strain condition, whereupon it will shorten the desired 2 mm.

Alternatively, it may be desirable to allow the surgeon to control the amount of compressive strain the device imparts. As an example, a staple can be provided to a surgeon that has a bridge that has been stretched to 8% strain, and then allowed to partially recover to a 6% strain prior to constraining the staple (thus allowing the staple to recover the remaining strain at the lower plateau of the mechanical hysteresis curve of the staple). The surgeon may then elect to "back off' the strain on the device to a lesser amount, dependent on the patient's needs. This can be accomplished by allowing for additional strain to be released by the surgeon prior to implantation of the device. Thus, in this form of the invention, the surgeon is able to tailor the specific amount of compression applied by the implanted device, by selectively releasing the strain on the device prior to implantation.

## Claims

1. A method for controlling an unloading stress of a shape memory material orthopaedic device (1100, 2005, 3005, 4005), comprising:
deforming the shape memory material orthopaedic device (1100, 2005, 3005, 4005) using at least one of superelastic properties and shape memory properties of the shape memory material orthopaedic device (1100, 2005, 3005, 4005);
recovering a portion of deformation of the shape memory material orthopaedic device (1100, 2005, 3005, 4005); and
mechanically constraining the shape memory material orthopaedic device (1100, 2005, 3005, 4005) after recovering the portion of the deformation,
**characterized in, that**
the shape memory material orthopaedic device (1100, 2005, 3005, 4005) comprises Nitinol, and the shape memory material orthopaedic device (1100, 2005, 3005, 4005) includes a first region having a first transition temperature and a second region having a second, different transition temperature.

2. The method as recited in claim 1, wherein the shape memory material orthopaedic device (1100, 2005, 3005, 4005) is a compression screw (1100).

3. The method as recited in claim 1, wherein the shape memory material orthopaedic device (1100, 2005, 3005, 4005) is a staple (2005).

4. The method as recited in claim 1, wherein the shape memory material orthopaedic device (1100, 2005, 3005, 4005) is an intramedullary fusion device (3005).

5. The method as recited in claim 1, wherein the shape memory material orthopaedic device (1100, 2005, 3005, 4005) is a compression plate (4005).

6. The method as recited in claim 1, wherein the shape memory material orthopaedic device (1100, 2005, 3005, 4005) is deformed using the superelastic properties until the shape memory material orthopaedic device (1100, 2005, 3005, 4005) generates a first stress level that is at or above an upper plateau of a mechanical hysteresis curve of the shape memory material orthopaedic device (1100, 2005, 3005, 4005).

7. The method as recited in claim 6, wherein the portion of the deformation is recovered until the shape memory material orthopaedic device (1100, 2005, 3005, 4005) generates a second stress level that is substantially at a lower plateau of the mechanical hysteresis curve of the shape memory material orthopaedic device (1100, 2005, 3005, 4005).

8. The method as recited in claim 1, wherein deforming the shape memory material orthopaedic device (1100, 2005, 3005, 4005) using the shape memory properties includes:
deforming the shape memory material orthopaedic device (1100, 2005, 3005, 4005) while at a temperature below a martensite start temperature to form detwinned martensite.

9. The method as recited in claim 8, wherein recovering the portion of the deformation of the shape memory material orthopaedic device (1100, 2005, 3005, 4005) includes:
applying a mechanical constraint to the shape memory material orthopaedic device (1100, 2005, 3005, 4005) in a way that allows the shape memory material orthopaedic device (1100, 2005, 3005, 4005) to partially recover the deformation when the shape memory material orthopaedic device (1100, 2005, 3005, 4005) is heated to a temperature above an austenite start temperature.

10. The method as recited in claim 9, wherein heating the shape memory material orthopaedic device (1100, 2005, 3005, 4005) to a temperature above the austenite start temperature allows the shape memory material orthopaedic device (1100, 2005, 3005, 4005) to recover some strain via free recovery, and wherein mechanically straining the shape memory material orthopaedic device (1100, 2005, 3005, 4005) limits further strain recovery.

11. The method as recited in claim 1, wherein the unloading stress of the shape memory material orthopaedic device (1100, 2005, 3005, 4005) is controlled using both the superelastic properties and the shape memory properties of the shape memory material orthopaedic device (1100, 2005, 3005, 4005).

12. The method as recited in claim 1 wherein one of the first region and the second region is a malleable region.

## Patentansprüche

1. Verfahren zum Steuern einer Entlastungsspannung einer orthopädischen Vorrichtung aus Formgedächtnismaterial (1100, 2005, 3005, 4005), umfassend:
Verformen der orthopädischen Vorrichtung aus Formgedächtnismaterial (1100, 2005, 3005, 4005) unter Verwendung von mindestens einer der superelastischen Eigenschaften und der Formgedächtniseigenschaften der orthopädischen Vorrichtung aus Formgedächtnismaterial (1100, 2005, 3005, 4005);
Wiederherstellen eines Teils der Verformung der orthopädischen Vorrichtung aus Formgedächtnismaterial (1100, 2005, 3005, 4005); und
mechanisches Beschränken der orthopädischen Vorrichtung aus Formgedächtnismaterial (1100, 2005, 3005, 4005) nach dem Wiederherstellen des Teils der Verformung,
**dadurch gekennzeichnet, dass**
die orthopädische Vorrichtung aus Formgedächtnismaterial (1100, 2005, 3005, 4005) Nitinol umfasst und die orthopädische Vorrichtung aus Formgedächtnismaterial (1100, 2005, 3005, 4005) eine erste Region mit einer ersten Übergangstemperatur und eine zweite Region mit einer zweiten, unterschiedlichen Übergangstemperatur beinhaltet.

2. Verfahren nach Anspruch 1, wobei die orthopädische Vorrichtung aus Formgedächtnismaterial (1100, 2005, 3005, 4005) eine Kompressionsschraube (1100) ist.

3. Verfahren nach Anspruch 1, wobei die orthopädische Vorrichtung (1100, 2005, 3005, 4005) aus Formgedächtnismaterial eine Klammer (2005) ist.

4. Verfahren nach Anspruch 1, wobei die orthopädische Vorrichtung aus Formgedächtnismaterial (1100, 2005, 3005, 4005) eine intramedulläre Fusionsvorrichtung (3005) ist.

5. Verfahren nach Anspruch 1, wobei die orthopädische Vorrichtung aus Formgedächtnismaterial (1100, 2005, 3005, 4005) eine Kompressionsplatte (4005) ist.

6. Verfahren nach Anspruch 1, wobei die orthopädische Vorrichtung aus Formgedächtnismaterial (1100, 2005, 3005, 4005) unter Verwendung der superelastischen Eigenschaften verformt wird, bis die orthopädische Vorrichtung aus Formgedächtnismaterial (1100, 2005, 3005, 4005) einen ersten Spannungspegel erzeugt, der sich auf oder oberhalb eines oberen Plateaus einer mechanischen Hysteresekurve der orthopädischen Vorrichtung aus Formgedächtnismaterial (1100, 2005, 3005, 4005) befindet.

7. Verfahren nach Anspruch 6, wobei der Teil der Verformung wiederhergestellt wird, bis die orthopädische Vorrichtung aus Formgedächtnismaterial (1100, 2005, 3005, 4005) einen zweiten Spannungspegel erzeugt, der sich im Wesentlichen auf einem unteren Plateau der mechanischen Hysteresekurve der orthopädischen Vorrichtung aus Formgedächtnismaterial (1100, 2005, 3005, 4005) befindet.

8. Verfahren nach Anspruch 1, wobei das Verformen der orthopädischen Vorrichtung aus Formgedächtnismaterial (1100, 2005, 3005, 4005) unter Verwendung der Formgedächtniseigenschaften beinhaltet:
Verformen der orthopädischen Vorrichtung aus Formgedächtnismaterial (1100, 2005, 3005, 4005) bei einer Temperatur unterhalb einer Martensit-Starttemperatur zur Bildung von entzinntem Martensit.

9. Verfahren nach Anspruch 8, wobei das Wiederherstellen des Teils der Verformung der orthopädischen Vorrichtung aus Formgedächtnismaterial (1100, 2005, 3005, 4005) beinhaltet:
Anwenden einer mechanischen Beschränkung auf die orthopädische Vorrichtung aus Formgedächtnismaterial (1100, 2005, 3005, 4005) in einer Weise, die der orthopädischen Vorrichtung aus Formgedächtnismaterial (1100, 2005, 3005, 4005) ermöglicht, die Verformung teilweise wiederherzustellen, wenn die orthopädische Vorrichtung aus Formgedächtnismaterial (1100, 2005, 3005, 4005) auf eine Temperatur oberhalb einer Austenit-Starttemperatur erwärmt wird.

10. Verfahren nach Anspruch 9, wobei das Erwärmen der orthopädischen Vorrichtung aus Formgedächtnismaterial (1100, 2005, 3005, 4005) auf eine Temperatur oberhalb der Austenit-Starttemperatur der orthopädischen Vorrichtung aus Formgedächtnismaterial (1100, 2005, 3005, 4005) ermöglicht, eine gewisse Spannung durch freie Erholung wiederherzustellen, und wobei das mechanische Belasten der orthopädischen Vorrichtung aus Formgedächtnismaterial (1100, 2005, 3005, 4005) eine weitere Erholung der Spannung begrenzt.

11. Das Verfahren nach Anspruch 1, wobei die Entlastungsspannung der orthopädischen Vorrichtung aus Formgedächtnismaterial (1100, 2005, 3005, 4005) unter Verwendung sowohl der superelastischen Eigenschaften als auch der Formgedächtniseigenschaften der orthopädischen Vorrichtung aus Formgedächtnismaterial (1100, 2005, 3005, 4005) gesteuert wird.

12. Verfahren nach Anspruch 1, wobei entweder die erste Region oder die zweite Region eine verformbare Region ist.

## Revendications

1. Procédé de commande une contrainte de décharge d'un dispositif orthopédique en matériau à mémoire de forme (1100, 2005, 3005, 4005), comprenant :
la déformation du dispositif orthopédique en matériau à mémoire de forme (1100, 2005, 3005, 4005) en utilisant au moins l'une des propriétés superélastiques et des propriétés à mémoire de forme du dispositif orthopédique en matériau à mémoire de forme (1100, 2005, 3005, 4005) ;
la récupération d'une portion de déformation du dispositif orthopédique en matériau à mémoire de forme (1100, 2005, 3005, 4005) ; et
le fait de contraindre mécaniquement le dispositif orthopédique en matériau à mémoire de forme (1100, 2005, 3005, 4005) après la récupération de la portion de la déformation, **caractérisé en ce que**,
le dispositif orthopédique en matériau à mémoire de forme (1100, 2005, 3005, 4005) comprend du Nitinol, et le dispositif orthopédique en matériau à mémoire de forme (1100, 2005, 3005, 4005) comprend une première région ayant une première température de transition et une deuxième région ayant une deuxième température de transition différente.

2. Procédé selon la revendication 1, dans lequel le dispositif orthopédique en matériau à mémoire de forme (1100, 2005, 3005, 4005) est une vis de compression (1100).

3. Procédé selon la revendication 1, dans lequel le dispositif orthopédique en matériau à mémoire de forme (1100, 2005, 3005, 4005) est une agrafe (2005).

4. Procédé selon la revendication 1, dans lequel le dispositif orthopédique en matériau à mémoire de forme (1100, 2005, 3005, 4005) est un dispositif de fusion intramédullaire (3005).

5. Procédé selon la revendication 1, dans lequel le dispositif orthopédique en matériau à mémoire de forme (1100, 2005, 3005, 4005) est une plaque de compression (4005).

6. Procédé selon la revendication 1, dans lequel le dispositif orthopédique en matériau à mémoire de forme (1100, 2005, 3005, 4005) est déformé en utilisant les propriétés superélastiques jusqu'à ce que le dispositif orthopédique en matériau à mémoire de forme (1100, 2005, 3005, 4005) génère un premier niveau de contrainte qui se trouve au niveau ou au-dessus d'un plateau supérieur d'une courbe d'hystérésis mécanique du dispositif orthopédique en matériau à mémoire de forme (1100, 2005, 3005).

7. Procédé selon la revendication 6, dans lequel la portion de la déformation est récupérée jusqu'à ce que le dispositif orthopédique en matériau à mémoire de forme (1100, 2005, 3005, 4005) génère un deuxième niveau de contrainte qui est sensiblement à un plateau inférieur de la courbe d'hystérésis mécanique du dispositif orthopédique en matériau à mémoire de forme (1100, 2005, 3005, 4005).

8. Procédé selon la revendication 1, dans lequel la déformation du dispositif orthopédique en matériau à mémoire de forme (1100, 2005, 3005, 4005) en utilisant les propriétés de mémoire de forme comprend :
la déformation du dispositif orthopédique en matériau à mémoire de forme (1100, 2005, 3005, 4005) alors qu'il est à une température inférieure à une température d'amorçage de la martensite commence à former de la martensite démaclée.

9. Procédé selon la revendication 8, dans lequel la récupération de la portion de la déformation du dispositif orthopédique en matériau à mémoire de forme (1100, 2005, 3005, 4005) comprend :
l'application d'une contrainte mécanique au dispositif orthopédique en matériau à mémoire de forme (1100, 2005, 3005, 4005) d'une manière qui permet au dispositif orthopédique en matériau à mémoire de forme (1100, 2005, 3005, 4005) de récupérer partiellement la déformation lorsque le dispositif orthopédique en matériau à mémoire de forme (1100, 2005, 3005, 4005) est chauffé à une température supérieure à une température d'amorçage d'austénite.

10. Procédé selon la revendication 9, dans lequel le chauffage du dispositif orthopédique en matériau à mémoire de forme (1100, 2005, 3005, 4005) jusqu'à une température supérieure à la température d'amorçage d'austénite permet au dispositif orthopédique en matériau à mémoire de forme (1100, 2005, 3005, 4005) de récupérer une certaine contrainte par récupération libre, et dans lequel la contrainte mécanique du dispositif orthopédique en matériau à mémoire de forme (1100, 2005, 3005, 4005) limite davantage la récupération de contrainte.

11. Procédé selon la revendication 1, dans lequel la contrainte de décharge du dispositif orthopédique en matériau à mémoire de forme (1100, 2005, 3005, 4005) est commandée en utilisant à la fois les propriétés superélastiques et les propriétés de mémoire de forme du dispositif orthopédique en matériau à mémoire de forme (1100, 2005, 3005, 4005).

12. Procédé selon la revendication 1, dans lequel l'une parmi la première région et la deuxième région est une région malléable.
